# EUROPEAN PATENT SPECIFICATION

(11) **EP 4 467 663 B1**
(45) Date of publication and mention of the grant of the patent: **09.12.2026**
(21) Application number: 24205945.9
(22) Date of filing: 21.05.2021
(51) Int. Cl.: C12Q 1/6883

(54) **EPIGENETIC MODIFICATION OF SELECT GENES AS MARKERS OF HYPERSOMNOLENCE**
EPIGENETISCHE MODIFIKATION AUSGEWÄHLTER GENE ALS MARKER FÜR HYPERSOMNOLENZ
MODIFICATION ÉPIGÉNÉTIQUE DE GÈNES SÉLECTIONNÉS EN TANT QUE MARQUEURS D'HYPERSOMNOLENCE

(30) Priority: 22.05.2020 US 202063029127 P
(43) Date of publication of application: 27.11.2024
(62) Document number(s) of the earlier application(s) in accordance with Art. 76 EPC: 21739195.2 / 4 150 119
(73) Proprietor: Wisconsin Alumni Research Foundation, Madison, WI 53726 (US)
(72) Inventor: PLANTE, David Thomas, Madison, 53711 (US); ALISCH, Reid Spencer, Prairie du Sac, 53578 (US)
(74) Representative: Williams Powell

(56) References cited:
- US-A1- 2013 129 668
- BARATEAU LUCIE ET AL: "Hypersomnolence, Hypersomnia, and Mood Disorders", CURRENT PSYCHIATRY REPORTS, CURRENT SCIENCE, US, vol. 19, no. 2, 27 February 2017 (2017-02-27), pages 1 - 11, XP036176635, ISSN: 1523-3812, [retrieved on 20210907], DOI: 10.1007/S11920-017-0763-0

### SEQUENCE LISTING

<110> Wisconsin Alumni Research Foundation
<120> EPIGENETIC MODIFICATION OF SELECT GENES AS MARKERS OF HYPERSOMNOLENCE
<130> 09824263-P200207WO01
<150> US 63/029,127
   <151> 2020-05-20
<160> 37
<170> PatentIn version 3.5
<210> 1
   <211> 385
   <212> DNA
   <213> Homo sapiens
<400> 1 
cgccgccata gctgcatggc cccgggacct ccctgtcgta cctgagagga gggcctggcc 60

cgtgaactgc ccgtacacgg aggcagcatg gggaaaggca ttgaagggcg ggaccccgga 120

gccgacttgc tgcagatcca aaaaggcgga gctagataaa gaggaagggg tggagctaga 180

actggacacc tcgggggttt cctgctttat ggcgaagggt gagtgaggat ctgccggagg 240

gagggagaca acaaggagag aggggtgtga gatggcgggg agggaacacg cacaagccaa 300

gccgtgggat gtggagggtg cgggcggggc gcggggcagg ctcagctgcc ctcagagtct 360

gggctgggga gagccggggc ccacg 385
<210> 2
   <211> 2528
   <212> DNA
   <213> Homo sapiens
<400> 2 
cgccgccata gctgcatggc cccgggacct ccctgtcgta cctgagagga gggcctggcc 60

cgtgaactgc ccgtacacgg aggcagcatg gggaaaggca ttgaagggcg ggaccccgga 120

gccgacttgc tgcagatcca aaaaggcgga gctagataaa gaggaagggg tggagctaga 180

actggacacc tcgggggttt cctgctttat ggcgaagggt gagtgaggat ctgccggagg 240

gagggagaca acaaggagag aggggtgtga gatggcgggg agggaacacg cacaagccaa 300

gccgtgggat gtggagggtg cgggcggggc gcggggcagg ctcagctgcc ctcagagtct 360
 
gggctgggga gagccggggc ccacgaggcg tggcaaggcg gggagagaga agctagacct 420

ccctgctgcg cttctgcagc gaaaatggag agacccggaa ggcgtgtgtg cgcccgcctc 480

tcccacagga gggagcgcgg agacccggga gcggcctagg accggaggcg cgacccctcg 540

gcccaccttg aggcccggcc taggaccgga ggcgcgaccc ctgggcccac cttgaggccc 600

ggcctaggac tggaggcgcg acccctcggc ccaccttgcg ggagccgcct aggaccggag 660

gcgcgacccc tcggcccagc ttgaggcccg gcctaggacc ggaggcgcga cccctcggcc 720

caccttgcgg gagccgccta ggaccggagg cgcgacccct cggcccacct tgaggcccgg 780

cctaggaccg gaggcgcgac ccctgggccc accttgaggc ccggcctagg actggaggcg 840

cgacccctcg gcccacctta cgggagccgc ctagggccgg aggcgcgacc cctcggccca 900

gcttgaggcc cggcctagga ccggaggcgc gacccctcgg cccaccttga ggcccggcct 960

aggactggag gcgcgacccc tcggcccacc ttgaggcccg gcctaggacc ggaggcgcga 1020

cccctgggcc caccttgagg cccggcctag gactggaggc gcgacccctc ggcccacctt 1080

gaggcccggc ctaggaccgg aggcgcgacc cctgggccca cctggcggcc cggccggcac 1140

agcccgcctc tcctctccag gccagggccc cacaccttcc gcctgacagc cagccaagct 1200

cttcagtccc ccgccctcca cctgccaggg aggctccggg cgttgtacct gccaccacgg 1260

ggtaggtctg gtgagtcgag aggttgcgcc ccagtggcgt gttggaaggg gtcagggtgg 1320

ccttcccgtc gtccagggtg ctgttgagca agggcagcgg gtagaggccc tggggagcaa 1380

agagaagtca gcgcacagag acgatccaac aagccgacct tcctgcagac cctgagcctg 1440

tgtcttagga cttggcagcc ctcatctccc caggagaggt cctcatcgcc cccttcttcc 1500

tttacgttct caactccact cggcacgttt cgttcatgct cattgtcctc ccgcatccct 1560

ggaattcaag gccagctggt agcatgggtg cccagacgtg ggatagagag tctcagccag 1620

aggaccacac cctggttgga tggctggtcg gcttcctggt ttcaccacac tgttgggacc 1680

cacagaatgc aggacggtgc caaggactgg agcagtctcc aaccggactg ttcagggtcc 1740

ttgcatctgt taatttattc ttccccagat aggaaaccca ggcacaaagg tttaagaaac 1800

ttgtcccact tccagatggg ccaaagatag gattcagatt cgagggatca gaatgtgtct 1860

gctctcaacc actgccctaa actgggtgac ataaggacca cttcttacat ttgcaaagag 1920

cttatatcag agctttgcct ctgttgtcct agtagagtcc cccagcagcc cagtgacatg 1980

ggtgagtaat attatgtctg tgttttatat gtggagcctg gaactcaggt aaatggccca 2040
 
attttgtata gtgaaacaga ctaaacccgg gatttttcgg ctgtggaccc aggtgcttta 2100

cccaccacgt ggatttcttc ttttctctct ccacatccaa acaaatagaa gtgtgcaagt 2160

tcggcttgaa aatggtattg agagttgttt ttgttggaaa cactctttgt caagtacctg 2220

ggggtcttca tcagtaagac ctcattttag atgcctcctg gcctcccctt cccaggagca 2280

cagctatgac cttaggtact ccttccgaaa agaacttgtt taactaaagg taagtgtacc 2340

tcatcctcac catggcctcc ttccactggg gaagcagata gcgcagaaaa aagaacacac 2400

ccattcccca cataccttca cactcgtcac atacctgcta cgtgagatgt gcaaagctga 2460

attcagggaa tgctcagtag ttacataaca gtgccactaa aggcaattgt tttcagtgat 2520

ttccatcg 2528
<210> 3
   <211> 4435
   <212> DNA
   <213> Homo sapiens
<400> 3 
cgatcggctt tttctttccc tttttgtggg ttttattatt tttgttacag gtggggctgc 60

cagggatggg ttatgcctgc agggggagaa atgatcagaa gagccacaat tctaccctca 120

acccagggaa tgtgagcttt tactgcctcc attttcttaa ccagaaatct ggtttagttt 180

tgctttcagg gctcttgtca ttggaaaaag attagtgctc agaacctgca gcccagaaat 240

ggcccagtcc catccctcca ggcaggctct gctcctcaag ctgcgtgggg aaatgccatc 300

atgggcttgt gcccagcatc caccctgcca atgatgacaa cagtgataat cgaggcattc 360

ataccaacca ccatttaggg agcctttata gagacttccc cccaacagtg gcctctttgg 420

cagctctggg agacagcctt ggaaagggcg aactaagggc cgagagaagc agcaacttgc 480

gaaggtcact actggccaga ctgggcccag tctcctgcct cttcccacag tggctgcaca 540

ccctactctc taccccattg cagtaggaag agaactccag aataagggga ggctctgaac 600

agtggagtca ggatccgtgt tttgactagt ttcaaggtct agtaaagcac agtatccaag 660

gaggctcagt ccagcatggg catgggctag ggctggagaa gtagaaaaga aatttgggca 720

agtagtgaat aagactgcca aatctattgt taggagagcc aggagaggtg tcctagtggt 780

gccttagtct atgggagaga cccagacacc attctgctgg gttcccaagc tggcacctta 840

aatttagtag ctgatgaatc caaggagccc ccaagcatca tctggcatgt ttttttgtat 900
 
gtttgtttgt ttgttttttg agacagagtc tctctctgtt acccaggctg gagtgtagtg 960

acgtgatctt ggctcaccac aatgtccgcc tcccaggttc aagtgattct cttgcctcag 1020

cctcctgagt agctgggact acaggcacgt gccaccacac ctggctaagt tttgtatttt 1080

tagtagagat gggtttcacc atgttggcca ggatggtctc gatctcctga ccttgtgatc 1140

taccagcctt ggcctcccaa agtgctgcga ttataggtgt gagccaccac acccggccca 1200

tctggcatgt tttataccaa ctggagtcag gcctagagca gggaggtggc tagacagtga 1260

ctgtaacctc catttcctgt cctggccaaa ccaatactca gatgggacag tgagaggaga 1320

catcacactc tggttacaca ccattaaccc ctgtctgact acacacacac acacacacag 1380

ccgtccagat tcacaaatac gttcttatca cccccttcat ccctcagtgc ctgggtggag 1440

gcccttaagc aggaatgccc cctcagagga gagggtggga gtggaatgat gggatttcag 1500

aatcccctca cctcaccctc tcacctccag atcctagtcc agatgtaatc ctgtcctcac 1560

cactttgcgt gcacgtgcac gcacacacac atgcacacac acagtctcaa cattcacata 1620

cagacaccca ctcttgacac ccacacatga actaaaagac aaacgttggt ccataaagga 1680

acacacatgc tatctacata cccccaaccc tgcccagtga tgctttcccc agcactactg 1740

cacagatccc agacaataca gacacacagg cactccttgc acactgagtc ctaggccact 1800

tggttgagct gcccccaccc caccccagct gcacactctt ctacatccat cccataaagg 1860

aagagtccgt ctctttaacc atgcttctca tggttttcag agtacccttg ccccaaatgc 1920

tgccaacctc cccctagact caagatcttc tctttccaga tcgctggagg ctcaatgcca 1980

gtgcacagga ggagatcgcc ttccaccata ttccctgact cccagaggct ctttctaggg 2040

caggggtctg gccaggaaga ctgaagccat gacgtcttcc tgtgcaagga taggaaagat 2100

gtctgtgggt tggagggtag ttatagggaa gcagctttct ccactttttg agaaagcact 2160

tgggaacaag catccctctc aggaagtgat tctccaccat tctcctctga tcctataggc 2220

ctggtggtcc aagcctcagg ccagttgatg cctcccactg ggatcagcag acaaggccat 2280

gaccctgcag tgacctcgac cccataatca ggtcaggtcc gggcttcatg gctagaagcg 2340

aggcccaggc gagtggccaa gacgcggcag gacaggagga tgcgcctggc tgctctcggc 2400

tgctggacgc ggcctgcagt agggagagcg cagcccgggg taatcccact cagggctccg 2460

ggtgcccagt tctcccactt cccatcctgg cggcctaaag gcaaggccta gaccctggag 2520
 
aggccaaagt cgggccaaaa caacagcgaa gctgggactg gaacaggcgc tgggggcctg 2580

cgcatgaccc caggctcaga atgcgacact ccagtgacgc cggggaatga gttgagatgg 2640

agccggagac agaggttggg gttcaggacg aggcgcttgg gtatgggatc ggatctgaga 2700

cggttgcggc gctggtgctt tgggctggac cagactcgaa cccagagcgg agcgagggtc 2760

agagccaggg caagagtccg aagcccagct cagcgtccag cccgcgagag accccaccat 2820

gaatgacgtc cgagtagggt tggaactcga agcccgaacg cactggaaat gcagccggac 2880

gacggctggg atccgagtgg gtccgggcca gaaatgggga cctcagagct ccaccgaggc 2940

gctcgaggtc agaactgaag cctgagactt aggcaggacc cgagacagag ccagagacga 3000

taccgaaacc caatggaccg cgaggaccga aggcagatcc ggggcgcaaa cctgtgcagg 3060

cactggcgcg ctgcctggag ctctgggttc gcgccgctga gcgccggcag gttggaccag 3120

cgctgcgcgc cggaagtgca ctcgggacgc cggggttctg gaggagagac cacacgccgc 3180

aaaagtggcc tccgaatgcg cccggggcct gccctcggcg acactgctgg ctcggcggcc 3240

gcagcctacc tgaagtagtc catcttgcag aagatctcct tgttcttgat gtagcagctg 3300

ttctgctgcc tcagcgacgt gcgacacacg gagcactcga ggcaccgcac gtgccagatg 3360

aggttgttga cctggggacg gggcggggac ggaggtcggc tcagcgcggg cctctttcac 3420

tccgggcccc agccttcccg gtctcattta cagtcagagg cgctgaagct cagaaggtgc 3480

atgcgattcc cctatttttc tcccgtaata ctgagactca gggaaatgga aataaactct 3540

tcttattttt cagacggaac ccgggggctc aggcagaccc taagtcttgc ccaaagcttc 3600

gcagtcgggc agcagtggag ccaggatttg gaagtaagag gacctgcggt gcttccctgc 3660

agtctcctgc gctgcgtccc acgccccgac aacacgcacg caacacctac ccctgtctca 3720

ccttgagcag atatcggtcc aggatctcga ggccgcagct ggagcagatg ttcttgcctg 3780

cagacggcac ggaggaggcg gcagagggcg gtgagcagac agatggcgtg ctgggcgtac 3840

atggggaggc ctgaccctcg tccttgtcca gagctcctgc cagggcctcg gcgtcagact 3900

gagcctgcgg cgggggagag aaggagagga gcgctgatcc gggcacccag agttggggct 3960

gccttggaga caaatctggg gcccccgagc accaattgac aacgaaatct ccccaggaca 4020

atgcgccgta gactgaagga cagggatgga agggcctatt ggagaggaag ggacggcccc 4080

aacttttaac acgcgcattc tgggtgctgg gagcggttaa agccaacatt ccctgccgga 4140

aaaccgaggc tcagagaggg gcggcgcccg ccgggagtcc cccagagcgt gagcagcagt 4200
 
tcgtggccgg aaaacctggc caggtcccca gggccgggcc gctggaggac tgggcaccgc 4260

aggcagggcc aaacggactc accatggcgg gcggcgcggt cccttcaaga cagcgggtgg 4320

tcgctttgca gccggaccct ggctgggcca tcacctgggg gaggggggga gggaacgcag 4380

gcggcggcgg ctgctgaact ggctccgcac agcctgagcc cagcgcctcc ccgcg 4435
<210> 4
   <211> 500
   <212> DNA
   <213> Homo sapiens
<400> 4 
cggtgcttcc ctgcagtctc ctgcgctgcg tcccacgccc cgacaacacg cacgcaacac 60

ctacccctgt ctcaccttga gcagatatcg gtccaggatc tcgaggccgc agctggagca 120

gatgttcttg cctgcagacg gcacggagga ggcggcagag ggcggtgagc agacagatgg 180

cgtgctgggc gtacatgggg aggcctgacc ctcgtccttg tccagagctc ctgccagggc 240

ctcggcgtca gactgagcct gcggcggggg agagaaggag aggagcgctg atccgggcac 300

ccagagttgg ggctgccttg gagacaaatc tggggccccc gagcaccaat tgacaacgaa 360

atctccccag gacaatgcgc cgtagactga aggacaggga tggaagggcc tattggagag 420

gaagggacgg ccccaacttt taacacgcgc attctgggtg ctgggagcgg ttaaagccaa 480

cattccctgc cggaaaaccg 500
<210> 5
   <211> 304
   <212> DNA
   <213> Homo sapiens
<400> 5 
cgtttgacag ctgctctgga gccgaggatc acaataagag aaggcgcgtg aggctccggc 60

agcaaacacc agccaggcag gcagcggtaa tcacagtgag caaagttgaa gactataaaa 120

gagccaggct aacgtgctgc cgagcaggtg tcctggattt tattgtagtt gcaaatatgc 180

ttctggtgat aagtatcgaa attatttttt aagtgcgctg gggaggaggg aggtgcggga 240

attgagcctc ctccgtttcc agggagcctc tgaaagtggg gtggggggag atgaggcatg 300

cacg 304
<210> 6
   <211> 885
   <212> DNA
   <213> Homo sapiens
<400> 6 
aggccaagtt gctacgttac agcacccggc tcccggcctt agggttcctg cttttccatg 60

gggtcttggg gtggtgcatc cccggcaccc ctacagcatc cgccccttcc gagcatgtct 120

tccccgtcac agagaacttc atgttggcgc ctccaaaagt tgccggagga attagggaac 180

agggtggggg tggatgagac tgaggccaga gagggggctg gcggtctgcc ctgaggaccc 240

ccgtcccacc cgccgccata gctgcatggc cccgggacct ccctgtcgta cctgagagga 300

gggcctggcc cgtgaactgc ccgtacacgg aggcagcatg gggaaaggca ttgaagggcg 360

ggaccccgga gccgacttgc tgcagatcca aaaaggcgga gctagataaa gaggaagggg 420

tggagctaga actggacacc tcgggggttt cctgctttat ggcgaagggt gagtgaggat 480

ctgccggagg gagggagaca acaaggagag aggggtgtga gatggcgggg agggaacacg 540

cacaagccaa gccgtgggat gtggagggtg cgggcggggc gcggggcagg ctcagctgcc 600

ctcagagtct gggctgggga gagccggggc ccacgaggcg tggcaaggcg gggagagaga 660

agctagacct ccctgctgcg cttctgcagc gaaaatggag agacccggaa ggcgtgtgtg 720

cgcccgcctc tcccacagga gggagcgcgg agacccggga gcggcctagg accggaggcg 780

cgacccctcg gcccaccttg aggcccggcc taggaccgga ggcgcgaccc ctgggcccac 840

cttgaggccc ggcctaggac tggaggcgcg acccctcggc ccacc 885
<210> 7
   <211> 885
   <212> DNA
   <213> Artificial Sequence
<220>
   <223> Bisulfite modified sequence
<400> 7 
aggttaagtt gttacgttat agtattcggt tttcggtttt agggtttttg tttttttatg 60

gggttttggg gtggtgtatt ttcggtattt ttatagtatt cgtttttttc gagtatgttt 120

ttttcgttat agagaatttt atgttggcgt ttttaaaagt tgtcggagga attagggaat 180

agggtggggg tggatgagat tgaggttaga gagggggttg gcggtttgtt ttgaggattt 240

tcgttttatt cgtcgttata gttgtatggt ttcgggattt ttttgtcgta tttgagagga 300
 
gggtttggtt cgtgaattgt tcgtatacgg aggtagtatg gggaaaggta ttgaagggcg 360

ggatttcgga gtcgatttgt tgtagattta aaaaggcgga gttagataaa gaggaagggg 420

tggagttaga attggatatt tcgggggttt tttgttttat ggcgaagggt gagtgaggat 480

ttgtcggagg gagggagata ataaggagag aggggtgtga gatggcgggg agggaatacg 540

tataagttaa gtcgtgggat gtggagggtg cgggcggggc gcggggtagg tttagttgtt 600

tttagagttt gggttgggga gagtcggggt ttacgaggcg tggtaaggcg gggagagaga 660

agttagattt ttttgttgcg tttttgtagc gaaaatggag agattcggaa ggcgtgtgtg 720

cgttcgtttt ttttatagga gggagcgcgg agattcggga gcggtttagg atcggaggcg 780

cgatttttcg gtttattttg aggttcggtt taggatcgga ggcgcgattt ttgggtttat 840

tttgaggttc ggtttaggat tggaggcgcg atttttcggt ttatt 885
<210> 8
   <211> 20
   <212> DNA
   <213> Artificial Sequence
<220>
   <223> Primer
<400> 8
   gaggttagag agggggttgg 20
<210> 9
   <211> 20
   <212> DNA
   <213> Artificial Sequence
<220>
   <223> Primer
<400> 9
   cgctacaaaa acgcaacaaa 20
<210> 10
   <211> 1922
   <212> DNA
   <213> Homo sapiens
<400> 10 
ccttacggga gccgcctagg gccggaggcg cgacccctcg gcccagcttg aggcccggcc 60

taggaccgga ggcgcgaccc ctcggcccac cttgaggccc ggcctaggac tggaggcgcg 120
 
acccctcggc ccaccttgag gcccggccta ggaccggagg cgcgacccct gggcccacct 180

tgaggcccgg cctaggactg gaggcgcgac ccctcggccc accttgaggc ccggcctagg 240

accggaggcg cgacccctgg gcccacctgg cggcccggcc ggcacagccc gcctctcctc 300

tccaggccag ggccccacac cttccgcctg acagccagcc aagctcttca gtcccccgcc 360

ctccacctgc cagggaggct ccgggcgttg tacctgccac cacggggtag gtctggtgag 420

tcgagaggtt gcgccccagt ggcgtgttgg aaggggtcag ggtggccttc ccgtcgtcca 480

gggtgctgtt gagcaagggc agcgggtaga ggccctgggg agcaaagaga agtcagcgca 540

cagagacgat ccaacaagcc gaccttcctg cagaccctga gcctgtgtct taggacttgg 600

cagccctcat ctccccagga gaggtcctca tcgccccctt cttcctttac gttctcaact 660

ccactcggca cgtttcgttc atgctcattg tcctcccgca tccctggaat tcaaggccag 720

ctggtagcat gggtgcccag acgtgggata gagagtctca gccagaggac cacaccctgg 780

ttggatggct ggtcggcttc ctggtttcac cacactgttg ggacccacag aatgcaggac 840

ggtgccaagg actggagcag tctccaaccg gactgttcag ggtccttgca tctgttaatt 900

tattcttccc cagataggaa acccaggcac aaaggtttaa gaaacttgtc ccacttccag 960

atgggccaaa gataggattc agattcgagg gatcagaatg tgtctgctct caaccactgc 1020

cctaaactgg gtgacataag gaccacttct tacatttgca aagagcttat atcagagctt 1080

tgcctctgtt gtcctagtag agtcccccag cagcccagtg acatgggtga gtaatattat 1140

gtctgtgttt tatatgtgga gcctggaact caggtaaatg gcccaatttt gtatagtgaa 1200

acagactaaa cccgggattt ttcggctgtg gacccaggtg ctttacccac cacgtggatt 1260

tcttcttttc tctctccaca tccaaacaaa tagaagtgtg caagttcggc ttgaaaatgg 1320

tattgagagt tgtttttgtt ggaaacactc tttgtcaagt acctgggggt cttcatcagt 1380

aagacctcat tttagatgcc tcctggcctc cccttcccag gagcacagct atgaccttag 1440

gtactccttc cgaaaagaac ttgtttaact aaaggtaagt gtacctcatc ctcaccatgg 1500

cctccttcca ctggggaagc agatagcgca gaaaaaagaa cacacccatt ccccacatac 1560

cttcacactc gtcacatacc tgctacgtga gatgtgcaaa gctgaattca gggaatgctc 1620

agtagttaca taacagtgcc actaaaggca attgttttca gtgatttcca tcgagctggg 1680

ttctgcaaag atccacagca ctttccggtt gcatgctggg cacttttgga agctgcagtc 1740

aattctggag gccaccaggg caccattagc acatagcagc aattattgac taaatggtgc 1800
 
tctggttcca tgccttccaa gggggcccac ttagaggcag ggtggagttg cttagggcct 1860

tttttttttt ttttttttgt agacggagtt ttgctcttgt tgcccaagct ggagtgcaat 1920

gg 1922
<210> 11
   <211> 1922
   <212> DNA
   <213> Artificial Sequence
<220>
   <223> Bisulfite modified sequence
<400> 11 
ttttacggga gtcgtttagg gtcggaggcg cgatttttcg gtttagtttg aggttcggtt 60

taggatcgga ggcgcgattt ttcggtttat tttgaggttc ggtttaggat tggaggcgcg 120

atttttcggt ttattttgag gttcggttta ggatcggagg cgcgattttt gggtttattt 180

tgaggttcgg tttaggattg gaggcgcgat ttttcggttt attttgaggt tcggtttagg 240

atcggaggcg cgatttttgg gtttatttgg cggttcggtc ggtatagttc gttttttttt 300

tttaggttag ggttttatat ttttcgtttg atagttagtt aagtttttta gtttttcgtt 360

ttttatttgt tagggaggtt tcgggcgttg tatttgttat tacggggtag gtttggtgag 420

tcgagaggtt gcgttttagt ggcgtgttgg aaggggttag ggtggttttt tcgtcgttta 480

gggtgttgtt gagtaagggt agcgggtaga ggttttgggg agtaaagaga agttagcgta 540

tagagacgat ttaataagtc gatttttttg tagattttga gtttgtgttt taggatttgg 600

tagtttttat ttttttagga gaggttttta tcgttttttt ttttttttac gtttttaatt 660

ttattcggta cgtttcgttt atgtttattg ttttttcgta tttttggaat ttaaggttag 720

ttggtagtat gggtgtttag acgtgggata gagagtttta gttagaggat tatattttgg 780

ttggatggtt ggtcggtttt ttggttttat tatattgttg ggatttatag aatgtaggac 840

ggtgttaagg attggagtag tttttaatcg gattgtttag ggtttttgta tttgttaatt 900

tatttttttt tagataggaa atttaggtat aaaggtttaa gaaatttgtt ttatttttag 960

atgggttaaa gataggattt agattcgagg gattagaatg tgtttgtttt taattattgt 1020

tttaaattgg gtgatataag gattattttt tatatttgta aagagtttat attagagttt 1080

tgtttttgtt gttttagtag agttttttag tagtttagtg atatgggtga gtaatattat 1140
 
gtttgtgttt tatatgtgga gtttggaatt taggtaaatg gtttaatttt gtatagtgaa 1200

atagattaaa ttcgggattt ttcggttgtg gatttaggtg ttttatttat tacgtggatt 1260

tttttttttt tttttttata tttaaataaa tagaagtgtg taagttcggt ttgaaaatgg 1320

tattgagagt tgtttttgtt ggaaatattt tttgttaagt atttgggggt ttttattagt 1380

aagattttat tttagatgtt ttttggtttt ttttttttag gagtatagtt atgattttag 1440

gtattttttt cgaaaagaat ttgtttaatt aaaggtaagt gtattttatt tttattatgg 1500

ttttttttta ttggggaagt agatagcgta gaaaaaagaa tatatttatt ttttatatat 1560

ttttatattc gttatatatt tgttacgtga gatgtgtaaa gttgaattta gggaatgttt 1620

agtagttata taatagtgtt attaaaggta attgttttta gtgattttta tcgagttggg 1680

ttttgtaaag atttatagta tttttcggtt gtatgttggg tatttttgga agttgtagtt 1740

aattttggag gttattaggg tattattagt atatagtagt aattattgat taaatggtgt 1800

tttggtttta tgttttttaa gggggtttat ttagaggtag ggtggagttg tttagggttt 1860

tttttttttt ttttttttgt agacggagtt ttgtttttgt tgtttaagtt ggagtgtaat 1920

gg 1922
<210> 12
   <211> 18
   <212> DNA
   <213> Artificial Sequence
<220>
   <223> Primer
<400> 12
   tacgggagtc gtttaggg 18
<210> 13
   <211> 18
   <212> DNA
   <213> Artificial Sequence
<220>
   <223> Primer
<400> 13
   ctcaacaaca ccctaaac 18
<210> 14
   <211> 20
   <212> DNA
   <213> Artificial Sequence
<220>
   <223> Primer
<400> 14
   attttggttg gatggttggt 20
<210> 15
   <211> 20
   <212> DNA
   <213> Artificial Sequence
<220>
   <223> Primer
<400> 15
   acaaccgaaa aatcccgaat 20
<210> 16
   <211> 23
   <212> DNA
   <213> Artificial Sequence
<220>
   <223> Primer
<400> 16
   tggggaagta gatagcgtag aaa 23
<210> 17
   <211> 22
   <212> DNA
   <213> Artificial Sequence
<220>
   <223> Primer
<400> 17
   ccctaaacaa ctccacccta cc 22
<210> 18
   <211> 1000
   <212> DNA
   <213> Homo sapiens
<400> 18 
cggctcagcg cgggcctctt tcactccggg ccccagcctt cccggtctca tttacagtca 60

gaggcgctga agctcagaag gtgcatgcga ttcccctatt tttctcccgt aatactgaga 120
 
ctcagggaaa tggaaataaa ctcttcttat ttttcagacg gaacccgggg gctcaggcag 180

accctaagtc ttgcccaaag cttcgcagtc gggcagcagt ggagccagga tttggaagta 240

agaggacctg cggtgcttcc ctgcagtctc ctgcgctgcg tcccacgccc cgacaacacg 300

cacgcaacac ctacccctgt ctcaccttga gcagatatcg gtccaggatc tcgaggccgc 360

agctggagca gatgttcttg cctgcagacg gcacggagga ggcggcagag ggcggtgagc 420

agacagatgg cgtgctgggc gtacatgggg aggcctgacc ctcgtccttg tccagagctc 480

ctgccagggc ctcggcgtca gactgagcct gcggcggggg agagaaggag aggagcgctg 540

atccgggcac ccagagttgg ggctgccttg gagacaaatc tggggccccc gagcaccaat 600

tgacaacgaa atctccccag gacaatgcgc cgtagactga aggacaggga tggaagggcc 660

tattggagag gaagggacgg ccccaacttt taacacgcgc attctgggtg ctgggagcgg 720

ttaaagccaa cattccctgc cggaaaaccg aggctcagag aggggcggcg cccgccggga 780

gtcccccaga gcgtgagcag cagttcgtgg ccggaaaacc tggccaggtc cccagggccg 840

ggccgctgga ggactgggca ccgcaggcag ggccaaacgg actcaccatg gcgggcggcg 900

cggtcccttc aagacagcgg gtggtcgctt tgcagccgga ccctggctgg gccatcacct 960

gggggagggg gggagggaac gcaggcggcg gcggctgctg 1000
<210> 19
   <211> 1000
   <212> DNA
   <213> Artificial Sequence
<220>
   <223> Bisulfite modified sequence
<400> 19 
cggtttagcg cgggtttttt ttatttcggg ttttagtttt ttcggtttta tttatagtta 60

gaggcgttga agtttagaag gtgtatgcga tttttttatt tttttttcgt aatattgaga 120

tttagggaaa tggaaataaa ttttttttat tttttagacg gaattcgggg gtttaggtag 180

attttaagtt ttgtttaaag tttcgtagtc gggtagtagt ggagttagga tttggaagta 240

agaggatttg cggtgttttt ttgtagtttt ttgcgttgcg ttttacgttt cgataatacg 300

tacgtaatat ttatttttgt tttattttga gtagatatcg gtttaggatt tcgaggtcgt 360

agttggagta gatgtttttg tttgtagacg gtacggagga ggcggtagag ggcggtgagt 420
 
agatagatgg cgtgttgggc gtatatgggg aggtttgatt ttcgtttttg tttagagttt 480

ttgttagggt ttcggcgtta gattgagttt gcggcggggg agagaaggag aggagcgttg 540

attcgggtat ttagagttgg ggttgttttg gagataaatt tggggttttc gagtattaat 600

tgataacgaa atttttttag gataatgcgt cgtagattga aggataggga tggaagggtt 660

tattggagag gaagggacgg ttttaatttt taatacgcgt attttgggtg ttgggagcgg 720

ttaaagttaa tattttttgt cggaaaatcg aggtttagag aggggcggcg ttcgtcggga 780

gttttttaga gcgtgagtag tagttcgtgg tcggaaaatt tggttaggtt tttagggtcg 840

ggtcgttgga ggattgggta tcgtaggtag ggttaaacgg atttattatg gcgggcggcg 900

cggttttttt aagatagcgg gtggtcgttt tgtagtcgga ttttggttgg gttattattt 960

gggggagggg gggagggaac gtaggcggcg gcggttgttg 1000
<210> 20
   <211> 20
   <212> DNA
   <213> Artificial Sequence
<220>
   <223> Primer
<400> 20
   ggaattcggg ggtttaggta 20
<210> 21
   <211> 20
   <212> DNA
   <213> Artificial Sequence
<220>
   <223> Primer
<400> 21
   acgctcctct ccttctctcc 20
<210> 22
   <211> 20
   <212> DNA
   <213> Artificial Sequence
<220>
   <223> Primer
<400> 22
   gcgtatatgg ggaggtttga 20
<210> 23
   <211> 23
   <212> DNA
   <213> Artificial Sequence
<220>
   <223> Primer
<400> 23
   cctacgatac ccaatcctcc aac 23
<210> 24
   <211> 4934
   <212> DNA
   <213> Homo sapiens
<400> 24 
ttgggagctc ttggcctctc tttgggcaga gtgctccagt ccacatccta ccagaccctt 60

gaccccgggc ttcaggaaac agaagcctag agaacctcag catgtgaccc ctccctgggc 120

cccagctcct tagaggaaaa ttctgcctcc cacgccacca ttcacactct ctccaggaag 180

aacttcctca tagcagccac ccagagcttg ggctgcagtg cagtgcagac aatagtcctt 240

gcctggcact cgatcggctt tttctttccc tttttgtggg ttttattatt tttgttacag 300

gtggggctgc cagggatggg ttatgcctgc agggggagaa atgatcagaa gagccacaat 360

tctaccctca acccagggaa tgtgagcttt tactgcctcc attttcttaa ccagaaatct 420

ggtttagttt tgctttcagg gctcttgtca ttggaaaaag attagtgctc agaacctgca 480

gcccagaaat ggcccagtcc catccctcca ggcaggctct gctcctcaag ctgcgtgggg 540

aaatgccatc atgggcttgt gcccagcatc caccctgcca atgatgacaa cagtgataat 600

cgaggcattc ataccaacca ccatttaggg agcctttata gagacttccc cccaacagtg 660

gcctctttgg cagctctggg agacagcctt ggaaagggcg aactaagggc cgagagaagc 720

agcaacttgc gaaggtcact actggccaga ctgggcccag tctcctgcct cttcccacag 780

tggctgcaca ccctactctc taccccattg cagtaggaag agaactccag aataagggga 840

ggctctgaac agtggagtca ggatccgtgt tttgactagt ttcaaggtct agtaaagcac 900

agtatccaag gaggctcagt ccagcatggg catgggctag ggctggagaa gtagaaaaga 960

aatttgggca agtagtgaat aagactgcca aatctattgt taggagagcc aggagaggtg 1020

tcctagtggt gccttagtct atgggagaga cccagacacc attctgctgg gttcccaagc 1080
 
tggcacctta aatttagtag ctgatgaatc caaggagccc ccaagcatca tctggcatgt 1140

ttttttgtat gtttgtttgt ttgttttttg agacagagtc tctctctgtt acccaggctg 1200

gagtgtagtg acgtgatctt ggctcaccac aatgtccgcc tcccaggttc aagtgattct 1260

cttgcctcag cctcctgagt agctgggact acaggcacgt gccaccacac ctggctaagt 1320

tttgtatttt tagtagagat gggtttcacc atgttggcca ggatggtctc gatctcctga 1380

ccttgtgatc taccagcctt ggcctcccaa agtgctgcga ttataggtgt gagccaccac 1440

acccggccca tctggcatgt tttataccaa ctggagtcag gcctagagca gggaggtggc 1500

tagacagtga ctgtaacctc catttcctgt cctggccaaa ccaatactca gatgggacag 1560

tgagaggaga catcacactc tggttacaca ccattaaccc ctgtctgact acacacacac 1620

acacacacag ccgtccagat tcacaaatac gttcttatca cccccttcat ccctcagtgc 1680

ctgggtggag gcccttaagc aggaatgccc cctcagagga gagggtggga gtggaatgat 1740

gggatttcag aatcccctca cctcaccctc tcacctccag atcctagtcc agatgtaatc 1800

ctgtcctcac cactttgcgt gcacgtgcac gcacacacac atgcacacac acagtctcaa 1860

cattcacata cagacaccca ctcttgacac ccacacatga actaaaagac aaacgttggt 1920

ccataaagga acacacatgc tatctacata cccccaaccc tgcccagtga tgctttcccc 1980

agcactactg cacagatccc agacaataca gacacacagg cactccttgc acactgagtc 2040

ctaggccact tggttgagct gcccccaccc caccccagct gcacactctt ctacatccat 2100

cccataaagg aagagtccgt ctctttaacc atgcttctca tggttttcag agtacccttg 2160

ccccaaatgc tgccaacctc cccctagact caagatcttc tctttccaga tcgctggagg 2220

ctcaatgcca gtgcacagga ggagatcgcc ttccaccata ttccctgact cccagaggct 2280

ctttctaggg caggggtctg gccaggaaga ctgaagccat gacgtcttcc tgtgcaagga 2340

taggaaagat gtctgtgggt tggagggtag ttatagggaa gcagctttct ccactttttg 2400

agaaagcact tgggaacaag catccctctc aggaagtgat tctccaccat tctcctctga 2460

tcctataggc ctggtggtcc aagcctcagg ccagttgatg cctcccactg ggatcagcag 2520

acaaggccat gaccctgcag tgacctcgac cccataatca ggtcaggtcc gggcttcatg 2580

gctagaagcg aggcccaggc gagtggccaa gacgcggcag gacaggagga tgcgcctggc 2640

tgctctcggc tgctggacgc ggcctgcagt agggagagcg cagcccgggg taatcccact 2700
 
cagggctccg ggtgcccagt tctcccactt cccatcctgg cggcctaaag gcaaggccta 2760

gaccctggag aggccaaagt cgggccaaaa caacagcgaa gctgggactg gaacaggcgc 2820

tgggggcctg cgcatgaccc caggctcaga atgcgacact ccagtgacgc cggggaatga 2880

gttgagatgg agccggagac agaggttggg gttcaggacg aggcgcttgg gtatgggatc 2940

ggatctgaga cggttgcggc gctggtgctt tgggctggac cagactcgaa cccagagcgg 3000

agcgagggtc agagccaggg caagagtccg aagcccagct cagcgtccag cccgcgagag 3060

accccaccat gaatgacgtc cgagtagggt tggaactcga agcccgaacg cactggaaat 3120

gcagccggac gacggctggg atccgagtgg gtccgggcca gaaatgggga cctcagagct 3180

ccaccgaggc gctcgaggtc agaactgaag cctgagactt aggcaggacc cgagacagag 3240

ccagagacga taccgaaacc caatggaccg cgaggaccga aggcagatcc ggggcgcaaa 3300

cctgtgcagg cactggcgcg ctgcctggag ctctgggttc gcgccgctga gcgccggcag 3360

gttggaccag cgctgcgcgc cggaagtgca ctcgggacgc cggggttctg gaggagagac 3420

cacacgccgc aaaagtggcc tccgaatgcg cccggggcct gccctcggcg acactgctgg 3480

ctcggcggcc gcagcctacc tgaagtagtc catcttgcag aagatctcct tgttcttgat 3540

gtagcagctg ttctgctgcc tcagcgacgt gcgacacacg gagcactcga ggcaccgcac 3600

gtgccagatg aggttgttga cctggggacg gggcggggac ggaggtcggc tcagcgcggg 3660

cctctttcac tccgggcccc agccttcccg gtctcattta cagtcagagg cgctgaagct 3720

cagaaggtgc atgcgattcc cctatttttc tcccgtaata ctgagactca gggaaatgga 3780

aataaactct tcttattttt cagacggaac ccgggggctc aggcagaccc taagtcttgc 3840

ccaaagcttc gcagtcgggc agcagtggag ccaggatttg gaagtaagag gacctgcggt 3900

gcttccctgc agtctcctgc gctgcgtccc acgccccgac aacacgcacg caacacctac 3960

ccctgtctca ccttgagcag atatcggtcc aggatctcga ggccgcagct ggagcagatg 4020

ttcttgcctg cagacggcac ggaggaggcg gcagagggcg gtgagcagac agatggcgtg 4080

ctgggcgtac atggggaggc ctgaccctcg tccttgtcca gagctcctgc cagggcctcg 4140

gcgtcagact gagcctgcgg cgggggagag aaggagagga gcgctgatcc gggcacccag 4200

agttggggct gccttggaga caaatctggg gcccccgagc accaattgac aacgaaatct 4260

ccccaggaca atgcgccgta gactgaagga cagggatgga agggcctatt ggagaggaag 4320

ggacggcccc aacttttaac acgcgcattc tgggtgctgg gagcggttaa agccaacatt 4380
 
ccctgccgga aaaccgaggc tcagagaggg gcggcgcccg ccgggagtcc cccagagcgt 4440

gagcagcagt tcgtggccgg aaaacctggc caggtcccca gggccgggcc gctggaggac 4500

tgggcaccgc aggcagggcc aaacggactc accatggcgg gcggcgcggt cccttcaaga 4560

cagcgggtgg tcgctttgca gccggaccct ggctgggcca tcacctgggg gaggggggga 4620

gggaacgcag gcggcggcgg ctgctgaact ggctccgcac agcctgagcc cagcgcctcc 4680

ccgcgcctgt tatataaacc ggcgccgaac aatgagtcct aactttgtag tgggcattta 4740

aagcccttcc ccacaaaagc ggtgtctctc taatgaagca atttgaattt ggattggatt 4800

ttttccctct ctctccccct ctccctgcac ttaacccgtg gctcttgaag taatcgctta 4860

gttcccttgc aatccaagcc tctgaggggg gaagaaaaac acgcgcacac acacacaaac 4920

tacctgcaat taga 4934
<210> 25
   <211> 4934
   <212> DNA
   <213> Artificial Sequence
<220>
   <223> Bisulfite modified sequence
<400> 25 
ttgggagttt ttggtttttt tttgggtaga gtgttttagt ttatatttta ttagattttt 60

gatttcgggt tttaggaaat agaagtttag agaattttag tatgtgattt ttttttgggt 120

tttagttttt tagaggaaaa ttttgttttt tacgttatta tttatatttt ttttaggaag 180

aattttttta tagtagttat ttagagtttg ggttgtagtg tagtgtagat aatagttttt 240

gtttggtatt cgatcggttt tttttttttt tttttgtggg ttttattatt tttgttatag 300

gtggggttgt tagggatggg ttatgtttgt agggggagaa atgattagaa gagttataat 360

tttattttta atttagggaa tgtgagtttt tattgttttt atttttttaa ttagaaattt 420

ggtttagttt tgtttttagg gtttttgtta ttggaaaaag attagtgttt agaatttgta 480

gtttagaaat ggtttagttt tattttttta ggtaggtttt gttttttaag ttgcgtgggg 540

aaatgttatt atgggtttgt gtttagtatt tattttgtta atgatgataa tagtgataat 600

cgaggtattt atattaatta ttatttaggg agtttttata gagatttttt tttaatagtg 660

gtttttttgg tagttttggg agatagtttt ggaaagggcg aattaagggt cgagagaagt 720
 
agtaatttgc gaaggttatt attggttaga ttgggtttag ttttttgttt ttttttatag 780

tggttgtata ttttattttt tattttattg tagtaggaag agaattttag aataagggga 840

ggttttgaat agtggagtta ggattcgtgt tttgattagt tttaaggttt agtaaagtat 900

agtatttaag gaggtttagt ttagtatggg tatgggttag ggttggagaa gtagaaaaga 960

aatttgggta agtagtgaat aagattgtta aatttattgt taggagagtt aggagaggtg 1020

ttttagtggt gttttagttt atgggagaga tttagatatt attttgttgg gtttttaagt 1080

tggtatttta aatttagtag ttgatgaatt taaggagttt ttaagtatta tttggtatgt 1140

ttttttgtat gtttgtttgt ttgttttttg agatagagtt tttttttgtt atttaggttg 1200

gagtgtagtg acgtgatttt ggtttattat aatgttcgtt ttttaggttt aagtgatttt 1260

tttgttttag ttttttgagt agttgggatt ataggtacgt gttattatat ttggttaagt 1320

tttgtatttt tagtagagat gggttttatt atgttggtta ggatggtttc gattttttga 1380

ttttgtgatt tattagtttt ggttttttaa agtgttgcga ttataggtgt gagttattat 1440

attcggttta tttggtatgt tttatattaa ttggagttag gtttagagta gggaggtggt 1500

tagatagtga ttgtaatttt tattttttgt tttggttaaa ttaatattta gatgggatag 1560

tgagaggaga tattatattt tggttatata ttattaattt ttgtttgatt atatatatat 1620

atatatatag tcgtttagat ttataaatac gtttttatta ttttttttat tttttagtgt 1680

ttgggtggag gtttttaagt aggaatgttt ttttagagga gagggtggga gtggaatgat 1740

gggattttag aattttttta ttttattttt ttatttttag attttagttt agatgtaatt 1800

ttgtttttat tattttgcgt gtacgtgtac gtatatatat atgtatatat atagttttaa 1860

tatttatata tagatattta tttttgatat ttatatatga attaaaagat aaacgttggt 1920

ttataaagga atatatatgt tatttatata tttttaattt tgtttagtga tgtttttttt 1980

agtattattg tatagatttt agataatata gatatatagg tattttttgt atattgagtt 2040

ttaggttatt tggttgagtt gtttttattt tattttagtt gtatattttt ttatatttat 2100

tttataaagg aagagttcgt ttttttaatt atgtttttta tggtttttag agtatttttg 2160

ttttaaatgt tgttaatttt tttttagatt taagattttt tttttttaga tcgttggagg 2220

tttaatgtta gtgtatagga ggagatcgtt ttttattata ttttttgatt tttagaggtt 2280

ttttttaggg taggggtttg gttaggaaga ttgaagttat gacgtttttt tgtgtaagga 2340

taggaaagat gtttgtgggt tggagggtag ttatagggaa gtagtttttt ttattttttg 2400
 
agaaagtatt tgggaataag tatttttttt aggaagtgat tttttattat ttttttttga 2460

ttttataggt ttggtggttt aagttttagg ttagttgatg ttttttattg ggattagtag 2520

ataaggttat gattttgtag tgatttcgat tttataatta ggttaggttc gggttttatg 2580

gttagaagcg aggtttaggc gagtggttaa gacgcggtag gataggagga tgcgtttggt 2640

tgttttcggt tgttggacgc ggtttgtagt agggagagcg tagttcgggg taattttatt 2700

tagggtttcg ggtgtttagt ttttttattt tttattttgg cggtttaaag gtaaggttta 2760

gattttggag aggttaaagt cgggttaaaa taatagcgaa gttgggattg gaataggcgt 2820

tgggggtttg cgtatgattt taggtttaga atgcgatatt ttagtgacgt cggggaatga 2880

gttgagatgg agtcggagat agaggttggg gtttaggacg aggcgtttgg gtatgggatc 2940

ggatttgaga cggttgcggc gttggtgttt tgggttggat tagattcgaa tttagagcgg 3000

agcgagggtt agagttaggg taagagttcg aagtttagtt tagcgtttag ttcgcgagag 3060

attttattat gaatgacgtt cgagtagggt tggaattcga agttcgaacg tattggaaat 3120

gtagtcggac gacggttggg attcgagtgg gttcgggtta gaaatgggga ttttagagtt 3180

ttatcgaggc gttcgaggtt agaattgaag tttgagattt aggtaggatt cgagatagag 3240

ttagagacga tatcgaaatt taatggatcg cgaggatcga aggtagattc ggggcgtaaa 3300

tttgtgtagg tattggcgcg ttgtttggag ttttgggttc gcgtcgttga gcgtcggtag 3360

gttggattag cgttgcgcgt cggaagtgta ttcgggacgt cggggttttg gaggagagat 3420

tatacgtcgt aaaagtggtt ttcgaatgcg ttcggggttt gttttcggcg atattgttgg 3480

ttcggcggtc gtagtttatt tgaagtagtt tattttgtag aagatttttt tgtttttgat 3540

gtagtagttg ttttgttgtt ttagcgacgt gcgatatacg gagtattcga ggtatcgtac 3600

gtgttagatg aggttgttga tttggggacg gggcggggac ggaggtcggt ttagcgcggg 3660

ttttttttat ttcgggtttt agttttttcg gttttattta tagttagagg cgttgaagtt 3720

tagaaggtgt atgcgatttt tttatttttt tttcgtaata ttgagattta gggaaatgga 3780

aataaatttt ttttattttt tagacggaat tcgggggttt aggtagattt taagttttgt 3840

ttaaagtttc gtagtcgggt agtagtggag ttaggatttg gaagtaagag gatttgcggt 3900

gtttttttgt agttttttgc gttgcgtttt acgtttcgat aatacgtacg taatatttat 3960

ttttgtttta ttttgagtag atatcggttt aggatttcga ggtcgtagtt ggagtagatg 4020
 
tttttgtttg tagacggtac ggaggaggcg gtagagggcg gtgagtagat agatggcgtg 4080

ttgggcgtat atggggaggt ttgattttcg tttttgttta gagtttttgt tagggtttcg 4140

gcgttagatt gagtttgcgg cgggggagag aaggagagga gcgttgattc gggtatttag 4200

agttggggtt gttttggaga taaatttggg gttttcgagt attaattgat aacgaaattt 4260

ttttaggata atgcgtcgta gattgaagga tagggatgga agggtttatt ggagaggaag 4320

ggacggtttt aatttttaat acgcgtattt tgggtgttgg gagcggttaa agttaatatt 4380

ttttgtcgga aaatcgaggt ttagagaggg gcggcgttcg tcgggagttt tttagagcgt 4440

gagtagtagt tcgtggtcgg aaaatttggt taggttttta gggtcgggtc gttggaggat 4500

tgggtatcgt aggtagggtt aaacggattt attatggcgg gcggcgcggt ttttttaaga 4560

tagcgggtgg tcgttttgta gtcggatttt ggttgggtta ttatttgggg gaggggggga 4620

gggaacgtag gcggcggcgg ttgttgaatt ggtttcgtat agtttgagtt tagcgttttt 4680

tcgcgtttgt tatataaatc ggcgtcgaat aatgagtttt aattttgtag tgggtattta 4740

aagttttttt ttataaaagc ggtgtttttt taatgaagta atttgaattt ggattggatt 4800

tttttttttt tttttttttt ttttttgtat ttaattcgtg gtttttgaag taatcgttta 4860

gtttttttgt aatttaagtt tttgaggggg gaagaaaaat acgcgtatat atatataaat 4920

tatttgtaat taga 4934
<210> 26
   <211> 22
   <212> DNA
   <213> Artificial Sequence
<220>
   <223> Primer
<400> 26
   ttgggttgta gtgtagtgta ga 22
<210> 27
   <211> 20
   <212> DNA
   <213> Artificial Sequence
<220>
   <223> Primer
<400> 27
   aattcgccct ttccaaaact 20
<210> 28
   <211> 20
   <212> DNA
   <213> Artificial Sequence
<220>
   <223> Primer
<400> 28
   gagagcgtag ttcggggtaa 20
<210> 29
   <211> 21
   <212> DNA
   <213> Artificial Sequence
<220>
   <223> Primer
<400> 29
   tctcgcgaac taaacgctaa a 21
<210> 30
   <211> 20
   <212> DNA
   <213> Artificial Sequence
<220>
   <223> Primer
<400> 30
   aatttaatgg atcgcgagga 20
<210> 31
   <211> 20
   <212> DNA
   <213> Artificial Sequence
<220>
   <223> Primer
<400> 31
   ccccaaatca acaacctcat 20
<210> 32
   <211> 20
   <212> DNA
   <213> Artificial Sequence
<220>
   <223> Primer
<400> 32
   ggtgttggga gcggttaaag 20
<210> 33
   <211> 20
   <212> DNA
   <213> Artificial Sequence
<220>
   <223> AAAACTCATTATTCGACGCC
<400> 33
   aaaactcatt attcgacgcc 20
<210> 34
   <211> 804
   <212> DNA
   <213> Homo sapiens
<400> 34 
gagagatggg tacttcacgc cacaaccccc tcaaccaaga ccacctccgc aaccacagcg 60

ggaaaagcta ggggcaggca gcccactttt gcaattactc tacattacag tgaactgcgg 120

tcctcacgtc ttcttttctt cactcccctt aacaaaataa aataaataat ttcccttcct 180

gcccacccac ggttgcccaa gaccaagtct tagaggcagc cgggttccga tcaagtgctg 240

cgaaacgacg cgtttgacag ctgctctgga gccgaggatc acaataagag aaggcgcgtg 300

aggctccggc agcaaacacc agccaggcag gcagcggtaa tcacagtgag caaagttgaa 360

gactataaaa gagccaggct aacgtgctgc cgagcaggtg tcctggattt tattgtagtt 420

gcaaatatgc ttctggtgat aagtatcgaa attatttttt aagtgcgctg gggaggaggg 480

aggtgcggga attgagcctc ctccgtttcc agggagcctc tgaaagtggg gtggggggag 540

atgaggcatg cacgcgaggc aaagacaaac aggaaagtgt ctctatcggg accaacgtgt 600

gcgaccctac ctgaaacggc aagcagaatt cggtgttttc ttccttttgc cctgatcttg 660

acgagatagg aaacttggga gagagagaga gagagagaga gagagagaga gagagagaga 720

gagagagaga gagagagacc gagactgcag cagccaagag agcgtggagc gatgggctgg 780

tggaagccgg agaggagcgc tagg 804
<210> 35
   <211> 804
   <212> DNA
   <213> Artificial Sequence
<220>
   <223> Bisulfite modified sequence
<400> 35 
gagagatggg tattttacgt tataattttt ttaattaaga ttattttcgt aattatagcg 60

ggaaaagtta ggggtaggta gtttattttt gtaattattt tatattatag tgaattgcgg 120

tttttacgtt tttttttttt tatttttttt aataaaataa aataaataat tttttttttt 180

gtttatttac ggttgtttaa gattaagttt tagaggtagt cgggtttcga ttaagtgttg 240

cgaaacgacg cgtttgatag ttgttttgga gtcgaggatt ataataagag aaggcgcgtg 300

aggtttcggt agtaaatatt agttaggtag gtagcggtaa ttatagtgag taaagttgaa 360

gattataaaa gagttaggtt aacgtgttgt cgagtaggtg ttttggattt tattgtagtt 420

gtaaatatgt ttttggtgat aagtatcgaa attatttttt aagtgcgttg gggaggaggg 480

aggtgcggga attgagtttt tttcgttttt agggagtttt tgaaagtggg gtggggggag 540

atgaggtatg tacgcgaggt aaagataaat aggaaagtgt ttttatcggg attaacgtgt 600

gcgattttat ttgaaacggt aagtagaatt cggtgttttt ttttttttgt tttgattttg 660

acgagatagg aaatttggga gagagagaga gagagagaga gagagagaga gagagagaga 720

gagagagaga gagagagatc gagattgtag tagttaagag agcgtggagc gatgggttgg 780

tggaagtcgg agaggagcgt tagg 804
<210> 36
   <211> 21
   <212> DNA
   <213> Artificial Sequence
<220>
   <223> Primer
<400> 36
   tcgggtttcg attaagtgtt g 21
<210> 37
   <211> 20
   <212> DNA
   <213> Artificial Sequence
<220>
   <223> Primer
<400> 37
   tttcaaataa aatcgcacac 20

## Description

### STATEMENT REGARDING FEDERALLY SPONSORED RESEARCH

This invention was made with government support under T42 OH008672 awarded by the Center for Disease Control and Prevention. The government has certain rights in the invention.

### FIELD OF THE INVENTION

The invention is directed to tools and methods for diagnosing and providing a prognosis of hypersomnolence, idiopathic hypersomnia, and long sleep time using differentially methylated positions.

### BACKGROUND

Disorders of unexplained hypersomnolence are a significant cause of dysfunction and reduced quality of life (Khan and Trotti. Chest. 2015, 148(1):262-273). Much of the research into disorders such as idiopathic hypersomnia (IH) has focused on symptoms of chronic daytime sleepiness and/or impaired quality of wakefulness. However, excessive quantities of sleep that are nonrestorative contribute equally if not more to the impairing aspects of these disorders. Currently, the pathophysiology that underlies excessive sleep duration *(i.e.,* hypersomnia) in disorders such as IH remains unknown and is a vital area of investigation for sleep medicine.

A key step to begin to understand the biological causes of hypersomnia requires examination of factors that contribute to habitual sleep duration more broadly. While the specific determinants of habitual sleep time remain unknown, recent genome wide association studies (GWAS) have highlighted the role of the paired box 8 (*PAX8*) gene in human sleep duration. PAX8 likely serves several roles as a master transcription factor crucial to embryonic development and maintenance functions after birth (Fernández et al. Nat Rev Endocrinol. 2015, 11(1):29-42). Using both self-report and accelerometer data in population-based cohorts, single nucleotide polymorphisms (SNPs) in *PAX8* and its related antisense gene (*PAX8-AS1*) have repeatedly demonstrated significant effects on habitual sleep duration (Gottlieb et al. Mol Psychiatry. 2015, 20(10):1232-1239; Lane et al. Nat Genet. 2017, 49(2):274-281; Dashti et al. Nat Commun. 2019, 10(1):1100; Jones et al. PLoS Genet. 2016, 12(8):e1006125; Jones et al. Nat Commun. 2019, 10(1):1585). However, the SNPs themselves impact habitual sleep duration only marginally (e.g., only a few minutes longer sleep per day). Thus, while GWAS investigations have pointed to a role of *PAX8* in sleep duration, specific sequence variability in the *PAX8* gene is unlikely to be a major contributing factor in pathological hypersomnia.

While GWAS help identify potential genes linked to phenotypic traits, additional methods are needed to help diagnose and treat patients and examine specific identified genes that relate to the pathophysiology of human disease.

### SUMMARY OF THE INVENTION

According to an aspect of the present invention there is provided a method as specified in claim 1.

In some embodiments, the one or more human genomic positions comprise 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, or each of 113992930 of chromosome 2, 113993052 of chromosome 2, 113993142 of chromosome 2, 113993304 of chromosome 2, 113993313 of chromosome 2, 113994035 of chromosome 2, 113994578 of chromosome 2, 113995370 of chromosome 2, 113995456 of chromosome 2, 124985406 of chromosome 9, 124985756 of chromosome 9, 124987896 of chromosome 9, 124988720 of chromosome 9, 124989052 of chromosome 9, 124989241 of chromosome 9, 124989294 of chromosome 9, 124989337 of chromosome 9, 124989408 of chromosome 9, 124989550 of chromosome 9, 124989839 of chromosome 9, 46391392 of chromosome 4, 46391436 of chromosome 4, 46391448 of chromosome 4, 46391476 of chromosome 4, 46391524 of chromosome 4, 46391532 of chromosome 4, and 46391694 of chromosome 4.

In some embodiments, the one or more human genomic positions comprise one or more, two or more, three or more, four or more, five or more, six or more, seven or more, eight or more, or each of 113992930 of chromosome 2, 113993052 of chromosome 2, 113993142 of chromosome 2, 113993304 of chromosome 2, 113993313 of chromosome 2, 113994035 of chromosome 2, 113994578 of chromosome 2, 113995370 of chromosome 2, and 113995456 of chromosome 2.

In some embodiments, the one or more human genomic positions comprise one or more, two or more, three or more, four or more, five or more, or each of 113993142 of chromosome 2, 113993304 of chromosome 2, 113993313 of chromosome 2, 113994578 of chromosome 2, 113995370 of chromosome 2, and 113995456 of chromosome 2.

In some embodiments, the one or more human genomic positions comprise one or more, two or more, or each of 113994578 of chromosome 2, 113995370 of chromosome 2, and 113995456 of chromosome 2.

In some embodiments, the one or more human genomic positions comprise 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, or each of 124985406 of chromosome 9, 124985756 of chromosome 9, 124987896 of chromosome 9, 124988720 of chromosome 9, 124989052 of chromosome 9, 124989241 of chromosome 9, 124989294 of chromosome 9, 124989337 of chromosome 9, 124989408 of chromosome 9, 124989550 of chromosome 9, 124989839 of chromosome 9, 46391392 of chromosome 4, 46391436 of chromosome 4, 46391448 of chromosome 4, 46391476 of chromosome 4, 46391524 of chromosome 4, 46391532 of chromosome 4, and 46391694 of chromosome 4.

In some embodiments, the one or more human genomic positions comprise one or more, two or more, three or more, four or more, five or more, six or more, seven or more, eight or more, nine or more, ten or more, or each of 124985406 of chromosome 9, 124985756 of chromosome 9, 124987896 of chromosome 9, 124988720 of chromosome 9, 124989052 of chromosome 9, 124989241 of chromosome 9, 124989294 of chromosome 9, 124989337 of chromosome 9, 124989408 of chromosome 9, 124989550 of chromosome 9, and 124989839 of chromosome 9.

In some embodiments, the one or more human genomic positions comprise one or more, two or more, three or more, four or more, five or more, six or more, or each of 46391392 of chromosome 4, 46391436 of chromosome 4, 46391448 of chromosome 4, 46391476 of chromosome 4, 46391524 of chromosome 4, 46391532 of chromosome 4, and 46391694 of chromosome 4.

In some embodiments, the primers consist of fewer than 60 total primers, fewer than 54 total primers, fewer than 48 total primers, fewer than 42 total primers, fewer than 36 total primers, fewer than 30 total primers, fewer than 24 total primers, fewer than 20 total primers, fewer than 18 total primers, fewer than 16 total primers, fewer than 14 total primers, fewer than 12 total primers, fewer than 10 total primers, fewer than 8 total primers, fewer than 6 total primers, fewer than 4 total primers, or fewer than 2 total primers.

In some embodiments, the primers comprise one or more pairs of primers. In some embodiments, each pair of primers amplifies a DNA fragment of 100-400 bp, 150-350 bp, or 200-300 bp in length.

In some embodiments, the one or more pairs of primers consists of fewer than 30 pairs of primers, fewer than 25 pairs of primers, fewer than 20 pairs of primers, fewer than 18 pairs of primers, fewer than 16 pairs of primers, fewer than 14 pairs of primers, fewer than 12 pairs of primers, fewer than 10 pairs of primers, fewer than 9 pairs of primers, fewer than 8 pairs of primers, fewer than 7 pairs of primers, fewer than 6 pairs of primers, fewer than 5 pairs of primers, fewer than 4 pairs of primers, fewer than 3 pairs of primers, fewer than 2 pairs of primers, or 1 pair of primers.

In some embodiments, the biofluid is selected from the group consisting of saliva, blood, cerebrospinal fluid, sweat, tears, semen, and urine. In some embodiments, the biofluid is blood or saliva.

In some embodiments, the subject is a human.

In some embodiments, the biofluid sample is from a healthy subject.

In some embodiments, the biofluid sample is from a subject with a mean sleep latency as measured by a multiple sleep latency test of less than 10 minutes.

In some embodiments, the biofluid sample is from a subject with a pupillary unrest index as measured by infrared pupillometry of at least 9.8.

In some embodiments, the biofluid sample is from a subject demonstrating lapses in a psychomotor vigilance test of at least 4.8.

In some embodiments, the biofluid sample is from a subject previously diagnosed with diabetes, asthma, or hypertension.

In some embodiments, the quantifying provides a degree of methylation at the one or more human genomic positions in the amplified DNA. In some embodiments, the degree of methylation at the one or more human genomic positions indicates total sleep time in the subject. In some embodiments, the degree of methylation at the one or more human genomic positions indicates hypersomnolence in the subject. The degree of methylation for any one the one or more human genomic position in any one of the aforementioned embodiments can be as shown in Tables 1 and 2.

In some methods, such as methods in which the degree of methylation at the one or more human genomic positions indicates hypersomnolence in the subject, the method further comprises administering to the subject a wake promoting medication. The wake promoting medication may be an amphetamine or modafinil. In some methods, such as methods in which the degree of methylation at the one or more human genomic positions indicates hypersomnolence in the subject, the method further comprises administering to the subject an oxybate or derivative thereof. In some methods, such as methods in which the degree of methylation at the one or more human genomic positions indicates hypersomnolence in the subject, the method further comprises having the subject undergo sleep extension therapy.

Also disclosed but not claimed is a method of amplification, such as a method of amplifying a differentially methylated position (DMP). In some embodiments, the method comprises providing a reaction mixture comprising bisulfite modified target DNA from a biofluid sample from a subject and primers specific to one or more human genomic positions, heating the reaction mixture to a first predetermined temperature for a first predetermined time, cooling the reaction mixture to a second predetermined temperature for a second predetermined time under conditions to allow the primers to hybridize with complementary sequences on the bisulfite modified target DNA, and repeating the heating and the cooling, wherein an amplified target DNA sample is formed.

The one or more human genomic positions can comprise any of the one or more human genomic positions as described above in any combination.

In some embodiments, the primers consist of fewer than 60 total primers, fewer than 54 total primers, fewer than 48 total primers, fewer than 42 total primers, fewer than 36 total primers, fewer than 30 total primers, fewer than 24 total primers, fewer than 20 total primers, fewer than 18 total primers, fewer than 16 total primers, fewer than 14 total primers, fewer than 12 total primers, fewer than 10 total primers, fewer than 8 total primers, fewer than 6 total primers, fewer than 4 total primers, or fewer than 2 total primers.

In some embodiments, the primers comprise one or more pairs of primers. In some embodiments, each pair of primers amplifies a DNA fragment of 100-400 bp, 150-350 bp, or 200-300 bp in length.

In some embodiments, the one or more pairs of primers consists of fewer than 30 pairs of primers, fewer than 25 pairs of primers, fewer than 20 pairs of primers, fewer than 18 pairs of primers, fewer than 16 pairs of primers, fewer than 14 pairs of primers, fewer than 12 pairs of primers, fewer than 10 pairs of primers, fewer than 9 pairs of primers, fewer than 8 pairs of primers, fewer than 7 pairs of primers, fewer than 6 pairs of primers, fewer than 5 pairs of primers, fewer than 4 pairs of primers, fewer than 3 pairs of primers, fewer than 2 pairs of primers, or 1 pair of primers.

In some embodiments, the biofluid is selected from the group consisting of saliva, blood, cerebrospinal fluid, sweat, tears, semen, and urine. In some embodiments, the biofluid is blood or saliva, in some embodiments, the subject is a human.

In some embodiments, the reaction mixture additionally comprises a polymerase and free nucleotides comprising adenine, thymine, cytosine, and guanine. In some embodiments, the reaction mixture additionally comprises a reaction buffer and MgCl₂. In some embodiments, at least one of the primers is biotinylated.

Also disclosed but not claimed is a biomarker panel. The biomarker panel may comprise a probe, primers, or a combination thereof specific to one or more human genomic positions. The one or more human genomic positions can comprise any of the one or more human genomic positions as described above in any combination.

In some embodiments, the primers consist of fewer than 60 total primers, fewer than 54 total primers, fewer than 48 total primers, fewer than 42 total primers, fewer than 36 total primers, fewer than 30 total primers, fewer than 24 total primers, fewer than 20 total primers, fewer than 18 total primers, fewer than 16 total primers, fewer than 14 total primers, fewer than 12 total primers, fewer than 10 total primers, fewer than 8 total primers, fewer than 6 total primers, fewer than 4 total primers, or fewer than 2 total primers.

In some embodiments, the primers comprise one or more pairs of primers. In some embodiments, each pair of primers amplifies a DNA fragment of 100-400 bp, 150-350 bp, or 200-300 bp in length.

In some embodiments, the one or more pairs of primers consists of fewer than 30 pairs of primers, fewer than 25 pairs of primers, fewer than 20 pairs of primers, fewer than 18 pairs of primers, fewer than 16 pairs of primers, fewer than 14 pairs of primers, fewer than 12 pairs of primers, fewer than 10 pairs of primers, fewer than 9 pairs of primers, fewer than 8 pairs of primers, fewer than 7 pairs of primers, fewer than 6 pairs of primers, fewer than 5 pairs of primers, fewer than 4 pairs of primers, fewer than 3 pairs of primers, fewer than 2 pairs of primers, or 1 pair of primers.

The biomarker panel may comprise a probe specific to each of the one or more human genomic positions.

The biomarker panel may comprise probes and the probes consist of fewer than 28 probes.

The probes or the primers may be arrayed on a substrate.

The substrate may be selected from the group consisting of a chip, a bead, a plate, a microfluidic device, or a multiwall plate.

The biomarker panel may comprise: a probe specific to each of the one or more human genomic positions; primers specific to each of the one or more human genomic positions; and a carrier. The carrier may be a substrate. The substrate may be selected from the group consisting of a chip, a bead, a plate, a microfluidic device, or a multiwall plate. The probes may be arrayed on the substrate. The carrier may be a buffered solution.

### BRIEF DESCRIPTION OF THE DRAWINGS

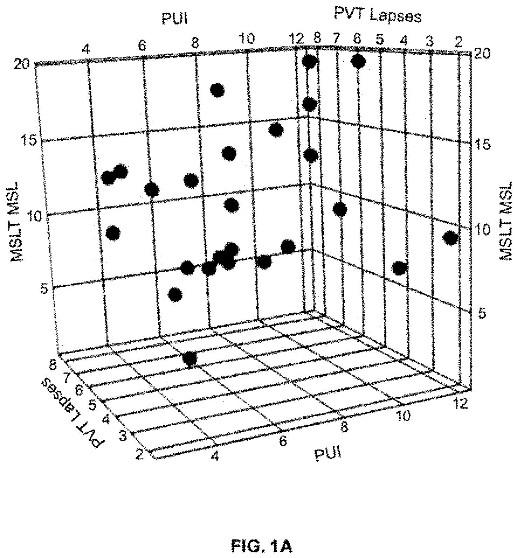

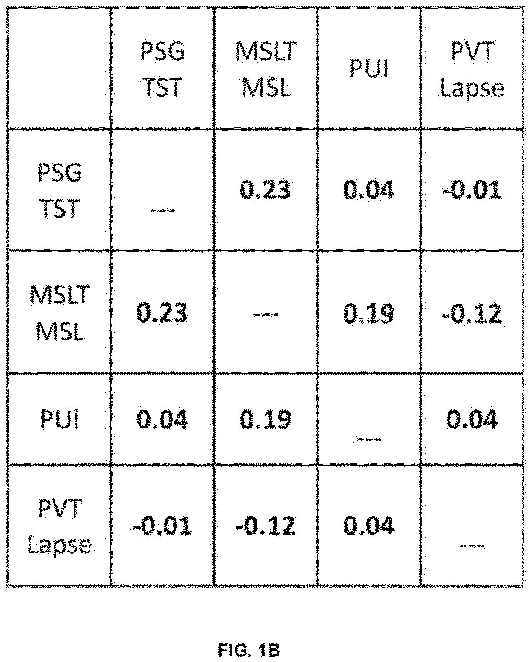

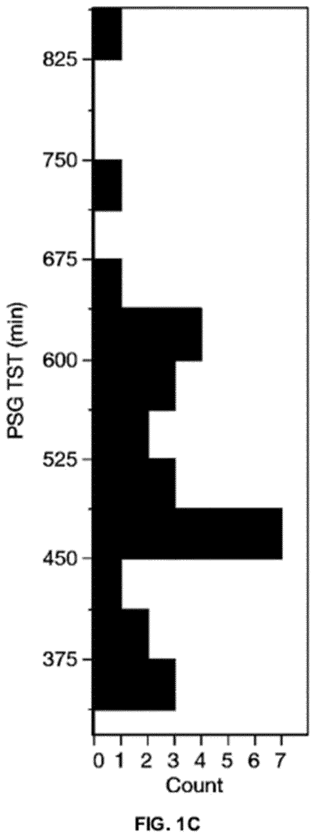

FIGS. 1A-1C show quantification of hypersomnolence variables. A) 3D-scatterplot of, MSLT mean sleep latency (MSL), pupillary unrest index (PUI), and PVT lapses in full data sample (N=28). B) Correlation (r) matrix for all hypersomnolence variables (p>0.2 for all associations). C) Histogram of ad libitum PSG TST (mean 8.7±1.9 hours; range 5.8 to 13.9 hours).

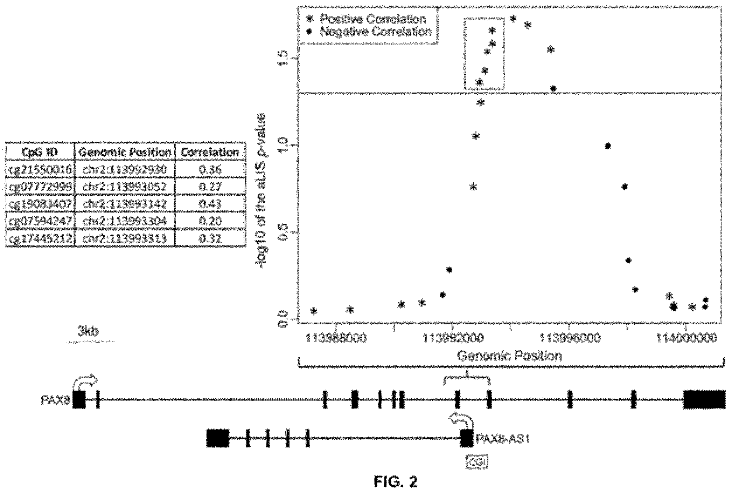

FIG. 2 shows relative positions of PSG-associated differentially methylated positions at the *PAX8* and *PAX-AS1* loci. Schematic of *PAX8* and its antisense gene *PAX8-AS1.* The relative positions of probes measuring methylation levels of CpG sites annotated to *PAX8* and *PAX8-AS1* with their genomic 5'-3'positions are provided (inset panel; x-axis) vs. the -log10 of the adjusted local index of significance (aLIS) P-value (y-axis). All probes were tested for positive correlations with PSG and negative PSG sleep duration. The level of aLIS P-values <0.05 (black line) is indicated. Dotted box denotes cluster of 5 CpG sites near *PAX8-AS1* promoter. The inset table provides the Illumina provided CpG ID number (CpG ID), the CpG chromosome (chr) number and genomic position, and the correlation R matrix between DNA methylation levels and total sleep duration for these 5 CpG sites.

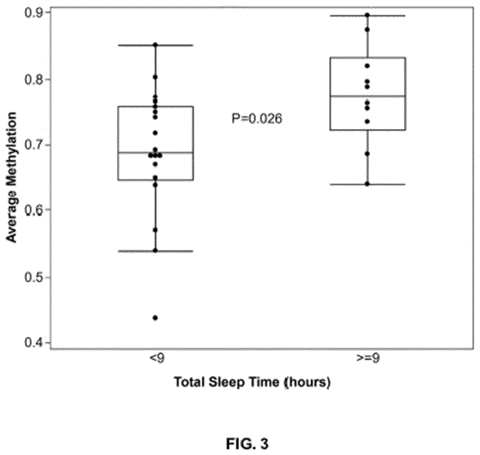

FIG. 3 shows average percent methylation of 5 DMPs in PAX8-AS1 promoter stratified by total sleep time above and below 9 hours. Average methylation was 8.6% greater in those with excessive sleep duration at this threshold (77.4% vs. 68.8%; P-value = 0.026).

### DETAILED DESCRIPTION OF THE INVENTION

Subjects with unexplained hypersomnolence have differentially methylated genes that are implicated in idiopathic hypersomnia and hypersomnolence associated conditions.

The present disclosure describes biofluid based assays for the diagnosis, prognosis, or therapeutic treatment recommendation of hypersomnolence, idiopathic hypersomnia, and acute or chronic long sleep time in a subject. The present disclosure describes differentially methylated positions (DMPs) associated with the paired box 8 (PAX8) gene and the paired box 8 anti-sense gene (PAX8-AS1) that are characteristic of hypersomnolence, idiopathic hypersomnia, and acute or chronic long sleep time in a subject. Also described are DMPs associated with LIM/homeobox protein 6 (LHX6) and gamma-aminobutyric acid receptor subunit alpha-2 (GABRA2). These characteristic biomarkers may be used to assay methylation in DNA isolated from a biofluid sample from a subject. These characteristic biomarkers may be used in the development of a screening panel, a resequencing panel, or a diagnostic kit for the processing of DNA isolated from a biofluid sample from a subject.

As used herein, "hypersomnolence" refers to excessive daytime sleepiness often accompanied by prolonged sleep duration. Biological and physiological causes of hypersomnolence are not universally known, many subjects may receive an ambiguous diagnosis despite comprehensive evaluation. Hypersomnolence is associated with a number of clinical phenotypes including, but not limited to reduced mean sleep latency (MSL), increased total sleep time (TST), increased pupillary unrest index (PUI), and increased lapses on a psychomotor vigilance test (PVT). Methods for measuring each of the hypersomnolence-associated phenotypes are known in the art.

In some embodiments, the hypersomnolence-associated phenotype is reduced MSL. MSL may be measured using a multiple sleep latency test in which quantifies sleep propensity during repeated nap opportunities and the presence of sleep onset rapid eye movement (REM) periods. In some embodiments, MSL measurements associated with hypersomnolence are less than 5 minutes for pathogenic sleepiness, and less than 8-10 minutes for moderate sleepiness.

In some embodiments, the hypersomnolence-associated phenotype is increased TST. TST may be measured using ad libitum polysomnography. In some embodiments, TST associated with hypersomnolence is greater than 9 hours. In more severe forms it is greater than 10 or 11 hours.

In some embodiments, the hypersomnolence-associated phenotype is increased PUI. PUI may be measured using infrared pupillometry. In some embodiments, PUI associated with hypersomnolence is at least 9.8 (unitless).

In some embodiments, the hypersomnolence-associated phenotype is lapses in the PVT. PVT is a well-validated measure used in sleep research to quantify the ability to sustain attention and response in a timely manner to salient signals. A PVT lapse is generally characterized as a failure to respond to the given stimuli within a predetermined time (typically 500 msec). In some embodiments, PVT lapses associated with hypersomnolence is at least 4.8 lapses.

As used herein, "idiopathic hypersomnia" refers to excessive daytime sleepiness even after a full or prolonged night's sleep. A subject with idiopathic hypersomnia may sleep normally or for long periods during the night but is still excessively sleepy during the day, which may cause dangerous situations if sleepiness is brought on during driving, working, or other activities. Napping generally does not relieve the sleepiness of an individual with idiopathic hypersomnia.

As used herein, "long sleep time" refers to acute or chronic periods of sleep by an individual that are longer than about 9 hours for an adult human, about 10 hours for a teenaged human, or about 12 hours for a young child.

Described herein are differentially methylated positions associated with the PAX8 and PAX8-AS1 genes that are characteristic of hypersomnolence, idiopathic hypersomnia, and long sleep time in a subject. Also described herein are differentially methylated positions associated with LHX6 that are characteristic of mean sleep latency and GABRA2 that are characteristic of pupillary unrest index.

As used herein, "differentially methylated positions" or "DMP" refers to CpG dinucleotide positions with a significant increase (hypermethylation), a significant decrease (hypomethylation) in methylation (*e.g.,* composite of 5-methylcytosine + 5-hydroxymethylation [5mC + 5hmC]) relative to hypersomnolence clinical phenotypes, or a significant change in methylation that correlates to a hypersomnolence clinical phenotype. In some embodiments, the DMP corresponds to a position with a change in methylation that correlates to a hypersomnolence clinical phenotype as outlined in Table 1 and Table 2.

**Table 1: Differentially methylated positions within PAX8 and PAX8-AS1 associated with total sleep time.**

| **CpG ID** | **Chromosome** | **Genomic position** | **DNA strand¹** | **Relation to a CpG Island²** | **UCSC RefGene Name** | **aLIS *P*-value³** | **Correlation⁴** | **DMP Status** | **DNAm cutoff** |
|---|---|---|---|---|---|---|---|---|---|
| cg21550016 | chr2 | 113992930 | + | N_Shore | PAX8;LOC440839;LOC654433 | 0.04313874 | 0.3592121 | Hyper | >0.7 |
| cg07772999 | chr2 | 113993052 | + | N_Shore | PAX8;LOC440839;LOC654433 | 0.03748023 | 0.2653631 | Hyper | >0.7 |
| cg19083407 | chr2 | 113993142 | - | N_Shore | PAX8;LOC440839;LOC654433 | 0.02880542 | 0.4265851 | Hyper | >0.7 |
| cg07594247 | chr2 | 113993304 | - | Island | PAX8;LOC440839;LOC654433 | 0.02598576 | 0.1988143 | Hyper | >0.7 |
| cg17445212 | chr2 | 113993313 | - | Island | PAX8; LOC440839;LOC654433 | 0.02179952 | 0.3193101 | Hyper | >0.7 |
| cg21610815 | chr2 | 113994035 | + | Island | PAX8;LOC654433;LOC440839 | 0.01861881 | 0.1549738 | Hyper | >0.8 |
| cg00422909 | chr2 | 113994578 | - | S_Shore | PAX8-AS1;PAX8 | 0.02050616 | 0.3243021 | Hyper | >0.6 |
| cg13020245 | chr2 | 113995370 | + | S_Shore | PAX8-AS1;PAX8 | 0.02841775 | 0.01047076 | Hyper | >0.8 |
| cg04345118 | chr2 | 113995456 | + | S_Shore | PAX8;LOC654433;LOC440839 | 0.04720507 | -0.0790697 | Hypo | <0.7 |

| | | | | | | | | | |
|---|---|---|---|---|---|---|---|---|---|
| (+) sense and (-) antisense DNA strands ²CpG island relationships are described in detail above ³The R package NHMMfdr was used to detect the adjusted local index of significance (aLIS), an extension of adjusted P-values, for each probe by first converting all P-values to z-scores, followed by employing a Hidden Markov Model to determine the "aLIS P-value." ⁴Correlation R matrix between DNA methylation levels and hypersomnolence variables | | | | | | | | | |

**Table 2: Differentially methylated positions within other DMP-associated genes.**

| **CpG ID** | **Chromosome** | **Genomic position** | **DNA strand¹** | **Relation to a CpG Island²** | **UCSC RefGene Name** | **aLIS *P*-value³** | **Correlation⁴** | **DMP Status** | **Associated Phenotype** | **DNAm cutoff** |
|---|---|---|---|---|---|---|---|---|---|---|
| cg10264903 | chr9 | 124985406 | - | N_Shelf | LHX6 | 0.04040517 | 0.2371739 | Hyper | MSLT | <0.15 |
| cg02825412 | chr9 | 124985756 | - | N_Shore | LHX6 | 0.02633677 | 0.0431312 | Hyper | MSLT | <0.68 |
| cg09723488 | chr9 | 124987896 | + | Island | LHX6 | 0.02187027 | -0.2624312 | Hypo | MSLT | >0.7 |
| cg04282082 | chr9 | 124988720 | - | Island | LHX6 | 0.01565797 | -0.2763055 | Hypo | MSLT | >0.65 |
| cg11479503 | chr9 | 124989052 | + | Island | LHX6 | 0.0181288 | -0.186529 | Hypo | MSLT | >0.7 |
| cg21213617 | chr9 | 124989241 | + | Island | LHX6 | 0.02273288 | -0.1609523 | Hypo | MSLT | >0.7 |
| cg21469772 | chr9 | 124989294 | - | Island | LHX6 | 0.02100529 | -0.2001834 | Hypo | MSLT | >0.7 |
| cg13571460 | chr9 | 124989337 | + | Island | LHX6 | 0.02199509 | -0.1841574 | Hypo | MSLT | >0.65 |
| cg05136264 | chr9 | 124989408 | - | Island | LHX6 | 0.02387765 | -0.1936458 | Hypo | MSLT | >0.7 |
| cg05037505 | chr9 | 124989550 | - | Island | LHX6 | 0.02907199 | -0.2365778 | Hypo | MSLT | >0.6 |
| cg15124400 | chr9 | 124989839 | + | Island | LHX6 | 0.03946631 | -0.2126105 | Hypo | MSLT | >0.7 |
| cg25145765 | chr4 | 46391392 | - | N_Shore | GABRA2 | 0.03552697 | -0.1922142 | Hypo | PUI | <0.03 |
| cg02620694 | chr4 | 46391436 | + | N_Shore | GABRA2 | 0.01720495 | -0.3900152 | Hypo | PUI | <0.03 |
| cg14778074 | chr4 | 46391448 | + | N_Shore | GABRA2 | 0.01414029 | -0.2889577 | Hypo | PUI | <0.09 |
| cg02633148 | chr4 | 46391476 | + | N_Shore | GABRA2 | 0.01611223 | 0.1642397 | Hyper | PUI | >0.06 |
| cg08206959 | chr4 | 46391524 | + | N_Shore | GABRA2 | 0.01240927 | -0.2945528 | Hypo | PUI | <0.05 |
| cg14411873 | chr4 | 46391532 | + | N_Shore | GABRA2 | 0.01317009 | -0.4174173 | Hypo | PUI | <0.06 |
| cg12205729 | chr4 | 46391694 | + | N_Shore | GABRA2 | 0.02400582 | -0.3520862 | Hypo | PUI | <0.02 |

| | | | | | | | | | | |
|---|---|---|---|---|---|---|---|---|---|---|
| ¹(+) sense and (-) antisense DNA strands ²CpG island relationships are described in detail above ³The R package NHMMfdr was used to detect the adjusted local index of significance (aLIS), an extension of adjusted P-values, for each probe by first converting all P-values to z-scores, followed by employing a Hidden Markov Model to determine the "aLIS P-value." ⁴Correlation R matrix between DNA methylation levels and hypersomnolence variables | | | | | | | | | | |

As used herein, "significant change" refers to a change (i.e., increase or a decrease) with a statistical signification of aLIS P-value < 0.05 when compared to hypersomnolence clinical phenotype.

As used herein, "significant increase" refers to an increase with a statistical signification of aLIS P-value < 0.05 when compared to hypersomnolence clinical phenotype.

As used herein, "significant decrease" refers to a decrease with a statistical significance of aLIS P-value < 0.05 when compared to hypersomnolence clinical phenotype.

As used herein, "differentially methylated position-associated genes" or "DMP-associated genes" refers to the genes in which the DMPs are located or the genes with which the DMPs are most closely associated. In some embodiments, the DMP may be in the coding region of the DMP-associated gene. In some embodiments, the DMP may be in the promoter region of the DMP-associated gene. In some embodiments, the DMP-associated genes are PAX8, PAX8-AS1, LHX6, and GABRA2.

As used herein, "CpG island" refers to short CpG-rich areas defined as >0.5 kb stretches of DNA with a G+C content greater than or equal to 55% and an observed:expected frequency of at least 0.6. In general, CpG are located in the promoter region and 5' to the transcription start site, although a CpG island may occur anywhere in a gene. Many terms of art are associated with characterization of CpG sites and differential methylation relative to a particular gene. A CpG shore refers to a region with lower CpG density than a CpG island that lies within the 2 kb up- and downstream of a CpG island. A CpG shelf refer to the 2 kb outside of a CpG shore. A CpG canyon refers to regions of low methylation more than 3.5 kb distant from CpG islands and CpG shores that frequently contain transcription factors. A CpG ocean refers to regions with low methylation and not characterized as either a CpG island, CpG shore, CpG shelf, or CpG canyon. A gene body refers to the entire gene from transcription start site to the end of the transcript. A gene desert refers to regions with very few, if any, genes in a 500 kb region. See, for example, Asmar et al. (DNA methylation and hydroxymethylation in cancer, Epigenetic Cancer Therapy, 2015, p. 9-30).

DMP biomarker candidates associated with PAX8 and PAX8-AS1 show significant (aLIS P-value < 0.05) association between degree of methylation in target regions and total sleep time measured with ad libitum polysomnography when DNA samples from subjects with hypersomnolence are analyzed. DMP biomarkers associated with PAX8 and PAX8-AS1 are recited in Table 1.

In some embodiments, the CpG sites of interest within an about 400 base pair region (SEQ ID NO:1) located in the coding region of the PAX8 gene and near the promoter of the PAX8-AS1 gene. All DMPs in this about 400 base pair region are hypermethylated (*i.e.,* increasing methylation is associated with longer sleep time). In some embodiments, the CpG sites of interest are within the region defined by SEQ ID NO:2.
SEQ ID NO:2 (CpG DMPs indicated in **BOLD**, the ~400 bp region of SEQ ID NO:1 is in *italics*):

CGCCGCCATAGCTGCATGGCCCCGGGACCTCCCTGTCGTACCTGAGAGGAGGGCCTGGCCCGTG
AACTGCCCGTACACGGAGGCAGCATGGGGAAAGGCATTGAAGGGCGGGACCCCGGAGCCGACTT
GCTGCAGATCCAAAAAGGCGGAGCTAGATAAAGAGGAAGGGGTGGAGCTAGAACTGGACACCTC
GGGGGTTTCCTGCTTTATGGCGAAGGGTGAGTGAGGATCTGCCGGAGGGAGGGAGACAACAAGG
AGAGAGGGGTGTGAGATGGCGGGGAGGGAACACGCACAAGCCAAGCCGTGGGATGTGGAGGGTG
CGGGCGGGGCGCGGGGCAGGCTCAGCTGCCCTCAGAGTCTGGGCTGGGGAGAGCCGGGGCCCAC
GAGGCGTGGCAAGGCGGGGAGAGAGAAGCTAGACCTCCCTGCTGCGCTTCTGCAGCGAAAATGG
AGAGACCCGGAAGGCGTGTGTGCGCCCGCCTCTCCCACAGGAGGGAGCGCGGAGACCCGGGAGC
GGCCTAGGACCGGAGGCGCGACCCCTCGGCCCACCTTGAGGCCCGGCCTAGGACCGGAGGCGCG
ACCCCTGGGCCCACCTTGAGGCCCGGCCTAGGACTGGAGGCGCGACCCCTCGGCCCACCTTGCG
GGAGCCGCCTAGGACCGGAGGCGCGACCCCTCGGCCCAGCTTGAGGCCCGGCCTAGGACCGGAG
GCGCGACCCCTCGGCCCACCTTGCGGGAGCCGCCTAGGACCGGAGGCGCGACCCCTCGGCCCAC
CTTGAGGCCCGGCCTAGGACCGGAGGCGCGACCCCTGGGCCCACCTTGAGGCCCGGCCTAGGAC
TGGAGGCGCGACCCCTCGGCCCACCTTACGGGAGCCGCCTAGGGCCGGAGGCGCGACCCCTCGG
CCCAGCTTGAGGCCCGGCCTAGGACCGGAGGCGCGACCCCTCGGCCCACCTTGAGGCCCGGCCT
AGGACTGGAGGCGCGACCCCTCGGCCCACCTTGAGGCCCGGCCTAGGACCGGAGGCGCGACCCC
TGGGCCCACCTTGAGGCCCGGCCTAGGACTGGAGGCGCGACCCCTCGGCCCACCTTGAGGCCCG
GCCTAGGACCGGAGGCGCGACCCCTGGGCCCACCTGGCGGCCCGGCCGGCACAGCCCGCCTCTC
CTCTCCAGGCCAGGGCCCCACACCTTCCGCCTGACAGCCAGCCAAGCTCTTCAGTCCCCCGCCC
TCCACCTGCCAGGGAGGCTCCGGGCGTTGTACCTGCCACCACGGGGTAGGTCTGGTGAGTCGAG
AGGTTGCGCCCCAGTGGCGTGTTGGAAGGGGTCAGGGTGGCCTTCCCGTCGTCCAGGGTGCTGT
TGAGCAAGGGCAGCGGGTAGAGGCCCTGGGGAGCAAAGAGAAGTCAGCGCACAGAGACGATCCA
ACAAGCCGACCTTCCTGCAGACCCTGAGCCTGTGTCTTAGGACTTGGCAGCCCTCATCTCCCCA
GGAGAGGTCCTCATCGCCCCCTTCTTCCTTTACGTTCTCAACTCCACTCGGCACGTTTCGTTCA
TGCTCATTGTCCTCCCGCATCCCTGGAATTCAAGGCCAGCTGGTAGCATGGGTGCCCAGACGTG
GGATAGAGAGTCTCAGCCAGAGGACCACACCCTGGTTGGATGGCTGGTCGGCTTCCTGGTTTCA
CCACACTGTTGGGACCCACAGAATGCAGGACGGTGCCAAGGACTGGAGCAGTCTCCAACCGGAC
TGTTCAGGGTCCTTGCATCTGTTAATTTATTCTTCCCCAGATAGGAAACCCAGGCACAAAGGTT
TAAGAAACTTGTCCCACTTCCAGATGGGCCAAAGATAGGATTCAGATTCGAGGGATCAGAATGT
GTCTGCTCTCAACCACTGCCCTAAACTGGGTGACATAAGGACCACTTCTTACATTTGCAAAGAG

CTTATATCAGAGCTTTGCCTCTGTTGTCCTAGTAGAGTCCCCCAGCAGCCCAGTGACATGGGTG
AGTAATATTATGTCTGTGTTTTATATGTGGAGCCTGGAACTCAGGTAAATGGCCCAATTTTGTA
TAGTGAAACAGACTAAACCCGGGATTTTTCGGCTGTGGACCCAGGTGCTTTACCCACCACGTGG
ATTTCTTCTTTTCTCTCTCCACATCCAAACAAATAGAAGTGTGCAAGTTCGGCTTGAAAATGGT
ATTGAGAGTTGTTTTTGTTGGAAACACTCTTTGTCAAGTACCTGGGGGTCTTCATCAGTAAGAC
CTCATTTTAGATGCCTCCTGGCCTCCCCTTCCCAGGAGCACAGCTATGACCTTAGGTACTCCTT
CCGAAAAGAACTTGTTTAACTAAAGGTAAGTGTACCTCATCCTCACCATGGCCTCCTTCCACTG
GGGAAGCAGATAGCGCAGAAAAAAGAACACACCCATTCCCCACATACCTTCACACTCGTCACAT
ACCTGCTACGTGAGATGTGCAAAGCTGAATTCAGGGAATGCTCAGTAGTTACATAACAGTGCCA
CTAAAGGCAATTGTTTTCAGTGATTTCCATCG

DMP biomarker candidates associated with LHX6 show significant (aLIS P-value < 0.05) association between degree of methylation in target regions and mean sleep latency measured by multiple sleep latency test when DNA samples from subjects with hypersomnolence are analyzed. DMP biomarkers associated with LHX6 are recited in Table 2.
SEQ ID NO:3 (CpG DMPs indicated in **BOLD**, the top candidate region of SEQ ID NO:4 is in *italics*)

CGATCGGCTTTTTCTTTCCCTTTTTGTGGGTTTATTATTTTTGTTACAGGTGGGGCTGCCAGG
GATGGGTTATGCCTGCAGGGGGAGAAATGATCAGAAGAGCCACAATTCTACCCTCAACCCAGGG
AATGTGAGCTTTTACTGCCTCCATTTTCTTAACCAGAAATCTGGTTTAGTTTTGCTTTCAGGGC
TCTTGTCATTGGAAAAAGATTAGTGCTCAGAACCTGCAGCCCAGAAATGGCCCAGTCCCATCCC
TCCAGGCAGGCTCTGCTCCTCAAGCTGCGTGGGGAAATGCCATCATGGGCTTGTGCCCAGCATC
CACCCTGCCAATGATGACAACAGTGATAATCGAGGCATTCATACCAACCACCATTTAGGGAGCC
TTTATAGAGACTTCCCCCCAACAGTGGCCTCTTTGGCAGCTCTGGGAGACAGCCTTGGAAAGGG
CGAACTAAGGGCCGAGAGAAGCAGCAACTTGCGAAGGTCACTACTGGCCAGACTGGGCCCAGTC
TCCTGCCTCTTCCCACAGTGGCTGCACACCCTACTCTCTACCCCATTGCAGTAGGAAGAGAACT
CCAGAATAAGGGGAGGCTCTGAACAGTGGAGTCAGGATCCGTGTTTTGACTAGTTTCAAGGTCT
AGTAAAGCACAGTATCCAAGGAGGCTCAGTCCAGCATGGGCATGGGCTAGGGCTGGAGAAGTAG
AAAAGAAATTTGGGCAAGTAGTGAATAAGACTGCCAAATCTATTGTTAGGAGAGCCAGGAGAGG
TGTCCTAGTGGTGCCTTAGTCTATGGGAGAGACCCAGACACCATTCTGCTGGGTTCCCAAGCTG
GCACCTTAAATTTAGTAGCTGATGAATCCAAGGAGCCCCCAAGCATCATCTGGCATGTTTTTTT
GTATGTTTGTTTGTTTGTTTTTTGAGACAGAGTCTCTCTCTGTTACCCAGGCTGGAGTGTAGTG
ACGTGATCTTGGCTCACCACAATGTCCGCCTCCCAGGTTCAAGTGATTCTCTTGCCTCAGCCTC
CTGAGTAGCTGGGACTACAGGCACGTGCCACCACACCTGGCTAAGTTTGTATTTTTAGTAGAG

ATGGGTTTCACCATGTTGGCCAGGATGGTCTCGATCTCCTGACCTTGTGATCTACCAGCCTTGG
CCTCCCAAAGTGCTGCGATTATAGGTGTGAGCCACCACACCCGGCCCATCTGGCATGTTTTATA
CCAACTGGAGTCAGGCCTAGAGCAGGGAGGTGGCTAGACAGTGACTGTAACCTCCATTTCCTGT
CCTGGCCAAACCAATACTCAGATGGGACAGTGAGAGGAGACATCACACTCTGGTTACACACCAT
TAACCCCTGTCTGACTACACACACACACACACACAGCCGTCCAGATTCACAAATACGTTCTTAT
CACCCCCTTCATCCCTCAGTGCCTGGGTGGAGGCCCTTAAGCAGGAATGCCCCCTCAGAGGAGA
GGGTGGGAGTGGAATGATGGGATTTCAGAATCCCCTCACCTCACCCTCTCACCTCCAGATCCTA
GTCCAGATGTAATCCTGTCCTCACCACTTTGCGTGCACGTGCACGCACACACACATGCACACAC
ACAGTCTCAACATTCACATACAGACACCCACTCTTGACACCCACACATGAACTAAAAGACAAAC
GTTGGTCCATAAAGGAACACACATGCTATCTACATACCCCCAACCCTGCCCAGTGATGCTTTCC
CCAGCACTACTGCACAGATCCCAGACAATACAGACACACAGGCACTCCTTGCACACTGAGTCCT
AGGCCACTTGGTTGAGCTGCCCCCACCCCACCCCAGCTGCACACTCTTCTACATCCATCCCATA
AAGGAAGAGTCCGTCTCTTTAACCATGCTTCTCATGGTTTTCAGAGTACCCTTGCCCCAAATGC
TGCCAACCTCCCCCTAGACTCAAGATCTTCTCTTTCCAGATCGCTGGAGGCTCAATGCCAGTGC
ACAGGAGGAGATCGCCTTCCACCATATTCCCTGACTCCCAGAGGCTCTTTCTAGGGCAGGGGTC
TGGCCAGGAAGACTGAAGCCATGACGTCTTCCTGTGCAAGGATAGGAAAGATGTCTGTGGGTTG
GAGGGTAGTTATAGGGAAGCAGCTTTCTCCACTTTTGAGAAAGCACTTGGGAACAAGCATCCC
TCTCAGGAAGTGATTCTCCACCATTCTCCTCTGATCCTATAGGCCTGGTGGTCCAAGCCTCAGG
CCAGTTGATGCCTCCCACTGGGATCAGCAGACAAGGCCATGACCCTGCAGTGACCTCGACCCCA
TAATCAGGTCAGGTCCGGGCTTCATGGCTAGAAGCGAGGCCCAGGCGAGTGGCCAAGACGCGGC
AGGACAGGAGGATGCGCCTGGCTGCTCTCGGCTGCTGGACGCGGCCTGCAGTAGGGAGAGCGCA
GCCCGGGGTAATCCCACTCAGGGCTCCGGGTGCCCAGTTCTCCCACTTCCCATCCTGGCGGCCT
AAAGGCAAGGCCTAGACCCTGGAGAGGCCAAAGTCGGGCCAAAACAACAGCGAAGCTGGGACTG
GAACAGGCGCTGGGGGCCTGCGCATGACCCCAGGCTCAGAATGCGACACTCCAGTGACGCCGGG
GAATGAGTTGAGATGGAGCCGGAGACAGAGGTTGGGGTTCAGGACGAGGCGCTTGGGTATGGGA
TCGGATCTGAGACGGTTGCGGCGCTGGTGCTTTGGGCTGGACCAGACTCGAACCCAGAGCGGAG
CGAGGGTCAGAGCCAGGGCAAGAGTCCGAAGCCCAGCTCAGCGTCCAGCCCGCGAGAGACCCCA
CCATGAATGACGTCCGAGTAGGGTTGGAACTCGAAGCCCGAACGCACTGGAAATGCAGCCGGAC
GACGGCTGGGATCCGAGTGGGTCCGGGCCAGAAATGGGGACCTCAGAGCTCCACCGAGGCGCTC
GAGGTCAGAACTGAAGCCTGAGACTTAGGCAGGACCCGAGACAGAGCCAGAGACGATACCGAAA
CCCAATGGACCGCGAGGACCGAAGGCAGATCCGGGGCGCAAACCTGTGCAGGCACTGGCGCGCT
GCCTGGAGCTCTGGGTTCGCGCCGCTGAGCGCCGGCAGGTTGGACCAGCGCTGCGCGCCGGAAG
TGCACTCGGGACGCCGGGGTTCTGGAGGAGAGACCACACGCCGCAAAAGTGGCCTCCGAATGCG
CCCGGGGCCTGCCCTCGGCGACACTGCTGGCTCGGCGGCCGCAGCCTACCTGAAGTAGTCCATC
TTGCAGAAGATCTCCTTGTTCTTGATGTAGCAGCTGTTCTGCTGCCTCAGCGACGTGCGACACA

CGGAGCACTCGAGGCACCGCACGTGCCAGATGAGGTTGTTGACCTGGGGACGGGGCGGGGACGG
AGGTCGGCTCAGCGCGGCCTCTTTCACTCCGGCCCCAGCCTTCCCGGTCTCATTTACAGTCA
GAGGCGCTGAAGCTCAGAAGGTGCATGCGATTCCCCTATTTTTCTCCCGTAATACTGAGACTCA
GGGAAATGGAAATAAACTCTTCTTATTTTTCAGACGGAACCCGGGGGCTCAGGCAGACCCTAAG
TCTTGCCCAAAGCTTCGCAGTCGGGCAGCAGTGGAGCCAGGATTTGGAAGTAAGAGGACCTGCG
GTGCTTCCCTGCAGTCTCCTGCGCTGCGTCCCACGCCCCGACAACACGCACGCAACACCTACCC
CTGTCTCACCTTGAGCAGATATCGGTCCAGGATCTCGAGGCCGCAGCTGGAGCAGATGTTCTTG
CCTGCAGACGGCACGGAGGAGGCGGCAGAGGGCGGTGAGCAGACAGATGGCGTGCTGGGCGTAC
ATGGGGAGGCCTGACCCTCGTCCTTGTCCAGAGCTCCTGCCAGGGCCTCGGCGTCAGACTGAGC
CTGCGGCGGGGGAGAGAAGGAGAGGAGCGCTGATCCGGGCACCCAGAGTTGGGGCTGCCTTGGA
GACAAATCTGGGGCCCCCGAGCACCAATTGACAACGAAATCTCCCCAGGACAATGCGCCGTAGA
CTGAAGGACAGGGATGGAAGGGCCTATTGGAGAGGAAGGACGGCCCCAACTTTTAACACGCGC
ATTCTGGGTGCTGGGAGCGGTTAAAGCCAACATTCCCTGCCGGAAAACCGAGGCTCAGAGAGGG
GCGGCGCCCGCCGGGAGTCCCCCAGAGCGTGAGCAGCAGTTCGTGGCCGGAAAACCTGGCCAGG
TCCCCAGGGCCGGGCCGCTGGAGGACTGGGCACCGCAGGCAGGGCCAAACGGACTCACCATGGC
GGGCGGCGCGGTCCCTTCAAGACAGCGGGTGGTCGCTTTGCAGCCGGACCCTGGCTGGGCCATC
ACCTGGGGGAGGGGGGGAGGGAACGCAGGCGGCGGCGGCTGCTGAACTGGCTCCGCACAGCCTG
AGCCCAGCGCCTCCCCGCG

DMP biomarker candidates associated with GABRA2 show significant (aLIS P-value < 0.05) association between degree of methylation in target regions and pupillary unrest index measured by infrared pupillometry when DNA samples from subjects with hypersomnolence are analyzed. DMP biomarkers associated with GABRA2 are recited in Table 2.
SEQ ID NO:5 (CpG DMPs are indicated in **BOLD**)

CGTTTGACAGCTGCTCTGGAGCCGAGGATCACAATAAGAGAAGGCGCGTGAGGCTCCGGCAGCA
AACACCAGCCAGGCAGGCAGCGGTAATCACAGTGAGCAAAGTTGAAGACTATAAAGAGCCAGG
CTAACGTGCTGCCGAGCAGGTGTCCTGGATTTTATTGTAGTTGCAAATATGCTTCTGGTGATAA
GTATCGAAATTATTTTTTAAGTGCGCTGGGGAGGAGGGAGGTGCGGGAATTGAGCCTCCTCCGT
TTCCAGGGAGCCTCTGAAAGTGGGGTGGGGGGAGATGAGGCATGCACG

Any combination of DMPs outlined in Tables 1 and 2 may be used to diagnose or give a prognosis of hypersomnolence, idiopathic hypersomnia, or long sleep time in a subject. Any combination of DMPs outlined in Tables 1 and 2 may be used in an assay to quantify methylation. Any combination of DMPs outlined in Tables 1 and 2 may be used in an assay to amplify the DMPs or DMP-associated genes for sequencing to quantify methylation.

In some embodiments, the DMPs of interest are within any one or more of SEQ ID NO:1, SEQ ID NO:2, SEQ ID NO:3, SEQ ID NO:4, SEQ ID NO:5, SEQ ID NO:6, SEQ ID NO:7, SEQ ID NO:10, SEQ ID NO:11, SEQ ID NO:18, SEQ ID NO:19, SEQ ID NO:24, SEQ ID NO:25, SEQ ID NO:34, and SEQ ID NO:35. In some embodiments, DMPs within any one or more of SEQ ID NO:1, SEQ ID NO:2, SEQ ID NO:3, SEQ ID NO:4, SEQ ID NO:5, SEQ ID NO:6, SEQ ID NO:7, SEQ ID NO:10, SEQ ID NO:11, SEQ ID NO:18, SEQ ID NO:19, SEQ ID NO:24, SEQ ID NO:25, SEQ ID NO:34, and SEQ ID NO:35 are assayed to diagnose or give a prognosis of hypersomnolence, idiopathic hypersomnia, or long sleep time in a subject.

In some embodiments, at least 1, at least 2, at least 3, at least 4, at least 5, at least 6, at least 7, at least 8, or all 9 of the DMPs of PAX8 and PAX8-AS1 outlined in Table 1 are assayed to diagnose or give a prognosis of hypersomnolence, idiopathic hypersomnia, or long sleep time in a subject. In some embodiments, at least 1 or both of the DMP-associated genes PAX8 and PAX8-AS1 are assayed to diagnose or give a prognosis of hypersomnolence, idiopathic hypersomnia, or long sleep time.

In some embodiments, at least 1, at least 2, at least 3, at least 4, at least 5, at least 6, at least 7, at least 8, at least 9, at least 10, or all 11 of the DMPs of LHX6 outlined in Table 2 are assayed. In some embodiments, at least 1, at least 2, at least 3, at least 4, at least 5, at least 6, or all 7 of the DMPs of GABRA2 outlined in Table 2 are assayed.

In some embodiments, the biomarkers described herein are used in the production of a biomarker panel for use in assaying DNA methylation. The biomarker panel includes probes or primers specific to the sequences of the DMPs or DMP-associated genes disclosed herein. In some embodiments, the biomarker panel includes probes or primers specific to the sequences of the DMP-associated genes PAX8 and PAX8-AS1. In some embodiments, the biomarker panel includes probes or primers specific to the sequences of the DMP-associated genes LHX6 and GABRA2. In some embodiments, the biomarker panel includes probes and/or primers specific to the DMPs listed in Tables 1 and 2. In some embodiments, the biomarker panel includes probes and/or primers specific to any one or more of SEQ ID NO:1, SEQ ID NO:2, SEQ ID NO:3, SEQ ID NO:4, SEQ ID NO:5, SEQ ID NO:6, SEQ ID NO:7, SEQ ID NO:10, SEQ ID NO:11, SEQ ID NO:18, SEQ ID NO:19, SEQ ID NO:24, SEQ ID NO:25, SEQ ID NO:34, and SEQ ID NO:35.

Primers specific to the DMPs or DMP-associated genes disclosed herein are between about 10 base pairs (bp) and about 40 bp and are complementary to sequences upstream and downstream of the DMP or DMP-associated gene of interest. Generally, a pair of forward and reverse primers that are designed to be complementary to the sequences flanking the DMP or DMP-associated gene are included. The size of the fragment to be amplified by the primer pair can range from less than 50 bp to greater than 10,000 bp. Primers can be designed that are complementary to a sequence less than 50 bp upstream of the DMP or more than 1,000 bp upstream depending on the sequence technology selected and the application of the biomarker panel. Therefore, it is possible to design many permutations of primer sets that are capable of amplifying a given DMP or DMP-associated gene of interest. For example, a given sample containing genomic DNA with a 500 bp DMP, a primer set can be designed to amplify i) the exact target region; or ii) a region encompassing the DMP including upstream and downstream regions. In some embodiments, the size of the fragment to be amplified by the primer pair has a length less than 1000 bp, less than 950 bp, less than 900 bp, less than 850 bp, less than 800 bp, less than 750 bp, less than 700 bp, less than 650 bp, less than 600 bp, less than 550 bp, less than 500 bp, less than 450 bp, less than 400 bp, 350 bp, less than 300 bp, less than 250 bp, less than 200 bp, less than 150 bp, less than 100 bp, less than 75 bp, or less than 500 bp. In some embodiments, the size of the fragment to be amplified by the primer pair has a length of at least 100 bp, at least 125 bp, at least 150 bp, at least 175 bp, or at least 200 bp and less than 400 bp, less than 375 bp, less than 350 bp, less than 325 bp, or less than 300 bp.

Probes specific to the DMPs or DMP-associated genes disclosed herein are between about 10 bp and about 40 bp and are commentary to sequences including or adjacent to the DMP or DMP-associated gene of interest. In some embodiments, the probe is complementary to the DMP of interest.

The disclosure includes a number of preferred primers and probes for amplification, selection, and identification of specific DMPs or DMP-associated genes. However, a skilled artisan will appreciate that the DMPs and DMP-associated genes disclosed can be amplified, selected, and identified by primers and probes other than those specific disclosed, which have been presented for purposes of illustration. We envision that the biomarker panel is compatible with a number of amplification and sequencing schemes and the scope of the claims should not be limited to the description of the embodiments contained herein.

Probes or primers for use in the biomarker panels described herein may be fused to a tag or label. Suitable tags and labels are known in the art, including but not limited to fluorescent labels (e.g., GFP, RFP, etc.), biotin, and combinations thereof. In some embodiments, the probe or primer is biotinylated and the biotinylated probe or primer bound sequence can be purified or captured with a streptavidin bound substrate.

In some embodiments, the primers or probes are covalently or non-covalently linked to a substrate. Suitable substrates for the biomarker panel include a bead, a plate, a microfluidic devise, a cuvette, a chip, a multiwell plate (e.g. 6-, 12-, 24-, 48-, 96-, 384-, or 1536-well plates).

Primers specific to the DMPs or DMP-associated genes disclosed herein can be obtained using a number of methods known in the art. In one example, the abundance of 5mC and/or 5hmC in regions of interest can be interrogated (see below for an exemplary method). Once regions of interest are identified, genomic sequences corresponding to each locus can be obtained from a reference genome and imported to a methyl primer design software package, such as the Thermo Fisher Scientific Methyl Primer Express v1.0 software, to generate high quality PCR primers pairs for methylation mapping. A second pair of primers can be designed that contains the initial primer sequence flanked by a sequencing adapter sequence (e.g., Illumina or others). Primers can be ordered through any preferred provider.

An exemplary method for interrogating the abundance of 5mC and/or 5hmC in regions of interest can be performed with the Methyl Primer Express^{™} Software v1.0 (Thermo Fisher Scientific). Methyl Primer Express^{™} can be downloaded from the Thermo Fisher Scientific Applied Biosystems website (www.thermofisher.com/us/en/home.html). The target sequence can be input into the box labeled "Insert Nucleotide Sequence." (To increase the likelihood of finding primers that will create an amplicon of ~200 base pairs (bp) or larger, input a minimum sequence of ~500 base pairs. The longer the input sequence, the better chance of finding suitable primers to target the region of interest.) "Design Primers" can then be clicked. "Select Target Sequence" can then be chosen to select the specific region of interest within the input DNA sequence. (As above, the longer the target sequence selected, the more likely it is to find suitable primers.) Following selection of primers, "Next" can be pressed, and a new window will open. If finding CpG islands within the target sequence is of interest, "Find CpG Islands" can be clicked. (Check the current default parameters that define a CpG island. If the input sequence is only 500 base pairs, consider shortening the defined minimum length of the island, as the length of a CpG island will later serve to help in choosing where to design the primers. It is possible that these analyses may not yield any CpG islands. If this is the case, simply move on to the next step.) In the bottom right corner of the new window, bisulfite sequencing primer design can be selected by clicking "Design BSP Primers." (BSP = Bisulfite Sequencing Primers. MSP = Methylation-Specific Primers.) Parameters used to design the BSPs can be reviewed by clicking "Next." (It is preferred to design primers that will generate an amplicon length of 200 to 300 base pairs. Amplicons <200 base pairs will result in sequencing the adaptors, meaning it will not maximize the data from the final sequencing run. Primers are preferably between 20 to 30 base pairs in length. Annealing temperature of the PCR should have a small range (e.g., 58° to 60°C).) It is preferred to initially weight performance towards the Low Speed/High Accuracy setting (between 8 to 10). If no appropriate primers are found, the scale can be moved towards the Hi Speed/Low Accuracy; however, this is not preferred. The designed primers can be purchased through any of a number of standard primer providers. Exemplary parameters include 25 nmole DNA oligos, standard desalting, and RNase free water (unless shipped desiccated). Two to three primer pairs per target region can be designed and purchased for simultaneous testing.

Exemplary non-modified and bisulfite-modified regions of interest for PAX8/PAX8-AS1, LHX6, and GABRA2 comprising the DMPs disclosed herein and exemplary primers for amplifying the DMPs are provided below. All positions below refer to the UCSC hg19 human reference genome assembly.

### PAX8/PAX8-AS1

**PAX8/PAX8-AS1 Region of Interest 1: chr2:113,992,930-113,993,314 5'pad=250 3'pad=250 Non-Modified PAX8/PAX8-AS1Region of Interest 1 Sequence:** 
AGGCCAAGTTGCTACGTTACAGCACCCGGCTCCCGGCCTTAGGGTTCCTGCTTTTCCAT
GGGGTCTTGGGGTGGTGCATCCCCGGCACCCCTACAGCATCCGCCCCTTCCGAGCATGT
CTTCCCCGTCACAGAGAACTTCATGTTGGCGCCTCCAAAAGTTGCCGGAGGAATTAGGG
AACAGGGTGGGGGTGGATGAGACTGAGGCCAGAGAGGGGGCTGGCGGTCTGCCCTGAGG
ACCCCCGTCCCACCCGCCGCCATAGCTGCATGGCCCCGGACCTCCCTGTCGTACCTGA
GAGGAGGGCCTGGCCCGTGAACTGCCCGTACACGGAGGCAGCATGGGGAAAGGCATTGA
AGGGCGGGACCCCGGAGCCGACTTGCTGCAGATCCAAAAAGGCGGAGCTAGATAAAGAG
GAAGGGGTGGAGCTAGAACTGGACACCTCGGGGGTTTCCTGCTTTATGGCGAAGGGTGA
GTGAGGATCTGCCGGAGGGAGGGAGACAACAAGGAGAGAGGGGTGTGAGATGGCGGGGA
GGGAACACGCACAAGCCAAGCCGTGGGATGTGGAGGGTGCGGGCGGGGCGCGGGGCAGG
CTCAGCTGCCCTCAGAGTCTGGGCTGGGGAGAGCCGGGGCCCACGAGGCGTGGCAAGGC
GGGGAGAGAGAAGCTAGACCTCCCTGCTGCGCTTCTGCAGCGAAAATGGAGAGACCCGG
AAGGCGTGTGTGCGCCCGCCTCTCCCACAGGAGGGAGCGCGGAGACCCGGAGCGGCCT
AGGACCGGAGGCGCGACCCCTCGGCCCACCTTGAGGCCCGGCCTAGGACCGGAGGCGCG
ACCCCTGGGCCCACCTTGAGGCCCGGCCTAGGACTGGAGGCGCGACCCCTCGGCCCACC
(SEQ ID NO:6)
 **Bisulfite-Modified PAX8/PAX8-AS1 Region of Interest 1 Sequence:**

AGGTTAAGTTGTTACGTTATAGTATTCGGTTTTCGGTTTAGGGTTTTGTTTTTTTAT
GGGGTTTTGGGGTGGTGTATTTTCGGTATTTTTATAGTATTCGTTTTTTCGAGTATGT
TTTTTTCGTTATAGAGAATTTTATGTTGGCGTTTTTAAAAGTTGTCGGAGGAATTAGGG
AATAGGGTGGGGGTGGATGAGATTGAGGTTAGAGAGGGGGTTGGCGGTTTGTTTTGAGG
ATTTTCGTTTTATTCGTCGTTATAGTTGTATGGTTTCGGGATTTTTTTGTCGTATTTGA

GAGGAGGGTTTGGTTCGTGAATTGTTCGTATACGGAGGTAGTATGGGAAAGGTATTGA
AGGGCGGGATTTCGGAGTCGATTTGTTGTAGATTTAAAAAGGCGGAGTTAGATAAAGAG
GAAGGGGTGGAGTTAGAATTGGATATTTCGGGGGTTTTTTGTTTTATGGCGAAGGGTGA
GTGAGGATTTGTCGGAGGGAGGGAGATAATAAGGAGAGAGGGGTGTGAGATGGCGGGGA
GGGAATACGTATAAGTTAAGTCGTGGATGTGGAGGGTGCGGGCGGGGCGCGGGGTAGG
TTTAGTTGTTTTTAGAGTTTGGGTTGGGAGAGTCGGGGTTTACGAGGCGTGGTAAGGC
GGGGAGAGAGAAGTTAGATTTTTTTGTTGCGTTTTTGTAGCGAAAATGGAGAGATTCGG
AAGGCGTGTGTGCGTTCGTTTTTTTTATAGGAGGGAGCGCGGAGATTCGGAGCGGTTT
AGGATCGGAGGCGCGATTTTTCGGTTTATTTTGAGGTTCGGTTTAGGATCGGAGGCGCG
ATTTTTGGGTTTATTTTGAGGTTCGGTTTAGGATTGGAGGCGCGATTTTTCGGTTTATT
(SEQ ID NO:7)

### Exemplary Primer Sequences for PAX8/PAX8-AS1 Region of Interest 1 Amplification:

Forward primer: GAGGTTAGAGAGGGGGTTGG (SEQ ID NO:8)
Reverse primer: CGCTACAAAAACGCAACAAA (SEQ ID NO:9)

**PAX8/PAX8-AS1 Region of Interest 2: chr2:113993785-113995706 5'pad=250 3'pad=250 Non-Modified PAX8/PAX8-AS1 Region of Interest 2 Sequence:** 
CCTTACGGGAGCCGCCTAGGGCCGGAGGCGCGACCCCTCGGCCCAGCTTGAGGCCCGGC
CTAGGACCGGAGGCGCGACCCCTCGGCCCACCTTGAGGCCCGGCCTAGGACTGGAGGCG
CGACCCCTCGGCCCACCTTGAGGCCCGGCCTAGGACCGGAGGCGCGACCCCTGGGCCCA
CCTTGAGGCCCGGCCTAGGACTGGAGGCGCGACCCCTCGGCCCACCTTGAGGCCCGGCC
TAGGACCGGAGGCGCGACCCCTGGGCCCACCTGGCGGCCCGGCCGGCACAGCCCGCCTC
TCCTCTCCAGGCCAGGGCCCCACACCTTCCGCCTGACAGCCAGCCAAGCTCTTCAGTCC
CCCGCCCTCCACCTGCCAGGGAGGCTCCGGGCGTTGTACCTGCCACCACGGGGTAGGTC
TGGTGAGTCGAGAGGTTGCGCCCCAGTGGCGTGTTGGAAGGGGTCAGGGTGGCCTTCCC
GTCGTCCAGGGTGCTGTTGAGCAAGGGCAGCGGGTAGAGGCCCTGGGGAGCAAAGAGAA
GTCAGCGCACAGAGACGATCCAACAAGCCGACCTTCCTGCAGACCCTGAGCCTGTGTCT
TAGGACTTGGCAGCCCTCATCTCCCCAGGAGAGGTCCTCATCGCCCCCTTCTTCCTTTA
CGTTCTCAACTCCACTCGGCACGTTTCGTTCATGCTCATTGTCCTCCCGCATCCCTGGA
ATTCAAGGCCAGCTGGTAGCATGGGTGCCCAGACGTGGATAGAGAGTCTCAGCCAGAG
GACCACACCCTGGTTGGATGGCTGGTCGGCTTCCTGGTTTCACCACACTGTTGGACCC
ACAGAATGCAGGACGGTGCCAAGGACTGGAGCAGTCTCCAACCGGACTGTTCAGGGTCC
TTGCATCTGTTAATTTATTCTTCCCCAGATAGGAAACCCAGGCACAAAGGTTTAAGAAA
 
CTTGTCCCACTTCCAGATGGGCCAAAGATAGGATTCAGATTCGAGGGATCAGAATGTGT
CTGCTCTCAACCACTGCCCTAAACTGGGTGACATAAGGACCACTTCTTACATTTGCAAA
GAGCTTATATCAGAGCTTTGCCTCTGTTGTCCTAGTAGAGTCCCCCAGCAGCCCAGTGA
CATGGGTGAGTAATATTATGTCTGTGTTTTATATGTGGAGCCTGGAACTCAGGTAAATG
GCCCAATTTTGTATAGTGAAACAGACTAAACCCGGGATTTTTCGGCTGTGGACCCAGGT
GCTTTACCCACCACGTGGATTTCTTCTTTTCTCTCCACATCCAAACAAATAGAAGTG
TGCAAGTTCGGCTTGAAAATGGTATTGAGAGTTGTTTTTGTTGGAAACACTCTTTGTCA
AGTACCTGGGGGTCTTCATCAGTAAGACCTCATTTTAGATGCCTCCTGGCCTCCCCTTC
CCAGGAGCACAGCTATGACCTTAGGTACTCCTTCCGAAAAGAACTTGTTTAACTAAAGG
TAAGTGTACCTCATCCTCACCATGGCCTCCTTCCACTGGGGAAGCAGATAGCGCAGAAA
AAAGAACACACCCATTCCCCACATACCTTCACACTCGTCACATACCTGCTACGTGAGAT
GTGCAAAGCTGAATTCAGGGAATGCTCAGTAGTTACATAACAGTGCCACTAAAGGCAAT
TGTTTTCAGTGATTTCCATCGAGCTGGGTTCTGCAAAGATCCACAGCACTTTCCGGTTG
CATGCTGGGCACTTTTGGAAGCTGCAGTCAATTCTGGAGGCCACCAGGGCACCATTAGC
ACATAGCAGCAATTATTGACTAAATGGTGCTCTGGTTCCATGCCTTCCAAGGGGGCCCA
CTTAGAGGCAGGGTGGAGTTGCTTAGGGCCTTTTTTTTTTTTTTTTTTGTAGACGGAG
TTTTGCTCTTGTTGCCCAAGCTGGAGTGCAATGG (SEQ ID NO:10)
 **Bisulfite-Modified PAX8/PAX8-AS1 Region of Interest 2 Sequence:**

TTTTACGGGAGTCGTTTAGGGTCGGAGGCGCGATTTTTCGGTTTAGTTTGAGGTTCGGT
TTAGGATCGGAGGCGCGATTTTTCGGTTTATTTTGAGGTTCGGTTTAGGATTGGAGGCG
CGATTTTTCGGTTTATTTTGAGGTTCGGTTTAGGATCGGAGGCGCGATTTTTGGGTTTA
TTTTGAGGTTCGGTTTAGGATTGGAGGCGCGATTTTTCGGTTTATTTTGAGGTTCGGTT
TAGGATCGGAGGCGCGATTTTTGGGTTTATTTGGCGGTTCGGTCGGTATAGTTCGTTTT
TTTTTTTTAGGTTAGGGTTTTATATTTTTCGTTTGATAGTTAGTTAAGTTTTTTAGTTT
TTCGTTTTTTTATTTGTTAGGGAGGTTTCGGGCGTTGTATTTGTTATTACGGGTAGGTT
TGGTGAGTCGAGAGGTTGCGTTTTAGTGGCGTGTTGGAAGGGGTTAGGGTGGTTTTTTC
GTCGTTTAGGGTGTTGTTGAGTAAGGGTAGCGGGTAGAGGTTTTGGGGAGTAAAGAGAA
GTTAGCGTATAGAGACGATTTAATAAGTCGATTTTTTTGTAGATTTTGAGTTTGTGTTT
TAGGATTTGGTAGTTTTTATTTTTTTAGGAGAGGTTTTTATCGTTTTTTTTTTTTTTTA
CGTTTTTAATTTTATTCGGTACGTTTCGTTTATGTTTATTGTTTTTTCGTATTTTTGGA
ATTTAAGGTTAGTTGGTAGTATGGGTGTTTAGACGTGGATAGAGAGTTTTAGTTAGAG
GATTATATTTTGGTTGGATGGTTGGTCGGTTTTTTGGTTTTATTATATTGTTGGATTT
ATAGAATGTAGGACGGTGTTAAGGATTGGAGTAGTTTTTAATCGGATTGTTTAGGGTTT
TTGTATTTGTTAATTTATTTTTTTTTAGATAGGAAATTTAGGTATAAAGGTTTAAGAAA

TTTGTTTTATTTTTAGATGGGTTAAAGATAGGATTTAGATTCGAGGATTAGAATGTGT
TTGTTTTTAATTATTGTTTTAAATTGGGTGATATAAGGATTATTTTTTATATTTGTAAA
GAGTTTATATTAGAGTTTTGTTTTTGTTGTTTTAGTAGAGTTTTTAGTAGTTTAGTGA
TATGGGTGAGTAATATTATGTTTGTGTTTTATATGTGGAGTTTGGAATTTAGGTAAATG
GTTTAATTTTGTATAGTGAAATAGATTAAATTCGGGATTTTTCGGTTGTGGATTTAGGT
GTTTTATTTATTACGTGGATTTTTTTTTTTTTTTTTTTATATTTAAATAAATAGAAGTG
TGTAAGTTCGGTTTGAAAATGGTATTGAGAGTTGTTTTTGTTGGAAATATTTTTTGTTA
AGTATTTGGGGGTTTTTATTAGTAAGATTTTATTTTAGATGTTTTTTGGTTTTTTTTTT
TTAGGAGTATAGTTATGATTTTAGGTATTTTTTTCGAAAAGAATTTGTTTAATTAAAGG
TAAGTGTATTTTATTTTTATTATGGTTTTTTTTTATTGGGGAAGTAGATAGCGTAGAAA
AAAGAATATATTTATTTTTTATATATTTTTATATTCGTTATATATTTGTTACGTGAGAT
GTGTAAAGTTGAATTTAGGGAATGTTTAGTAGTTATATAATAGTGTTATTAAAGGTAAT
TGTTTTTAGTGATTTTTATCGAGTTGGGTTTTGTAAAGATTTATAGTATTTTTCGGTTG
TATGTTGGGTATTTTTTGGAAGTTGTAGTTAATTTTGGAGGTTATTAGGGTATTATTAGT
ATATAGTAGTAATTATTGATTAAATGGTGTTTTGGTTTTATGTTTTTTAAGGGGGTTTA
TTTAGAGGTAGGGTGGAGTTGTTTAGGGTTTTTTTTTTTTTTTTTTTTTGTAGACGGAG
TTTTGTTTTTGTTGTTTAAGTTGGAGTGTAATGG (SEQ ID NO:11)

### Exemplary Primer Sequences for Amplification of PAX8/PAX8-AS1 Region of Interest 1 (Chr2: 113994035-113995456):

Forward primer1: TACGGGAGTCGTTTAGGG (SEQ ID NO: 12)
Reverse primer1: CTCAACAACACCCTAAAC (SEQ ID NO: 13)
Forward primer2: **ATTTTGGTTGGATGGTTGGT** (SEQ ID NO:14)
Reverse primer2: **ACAACCGAAAAATCCCGAAT** (SEQ ID NO:15)
Forward primer3: TGGGGAAGTAGATAGCGTAGAAA (SEQ ID NO:16)
Reverse primer3: CCCTAAACAACTCCACCCTACC (SEQ ID NO: 17)

### LHX6

### LHX6 Region of Interest 1: chr9:124,989,052-124,989,551 5'pad=250 3'pad=250 Non-Modified LHX6 Region of Interest 1 Sequence:

CGGCTCAGCGCGGCCTCTTTCACTCCGGCCCCAGCCTTCCCGGTCTCATTTACAGTC
AGAGGCGCTGAAGCTCAGAAGGTGCATGCGATTCCCCTATTTTTCTCCCGTAATACTGA
GACTCAGGGAAATGGAAATAAACTCTTCTTATTTTTCAGACGGAACCCGGGGGCTCAGG
CAGACCCTAAGTCTTGCCCAAAGCTTCGCAGTCGGGCAGCAGTGGAGCCAGGATTTGGA
AGTAAGAGGACCTGCGGTGCTTCCCTGCAGTCTCCTGCGCTGCGTCCCACGCCCCGACA

ACACGCACGCAACACCTACCCCTGTCTCACCTTGAGCAGATATCGGTCCAGGATCTCGA
GGCCGCAGCTGGAGCAGATGTTCTTGCCTGCAGACGGCACGGAGGAGGCGGCAGAGGGC
GGTGAGCAGACAGATGGCGTGCTGGGCGTACATGGGGAGGCCTGACCCTCGTCCTTGTC
CAGAGCTCCTGCCAGGGCCTCGGCGTCAGACTGAGCCTGCGGCGGGGAGAGAAGGAGA
GGAGCGCTGATCCGGGCACCCAGAGTTGGGGCTGCCTTGGAGACAAATCTGGGGCCCCC
GAGCACCAATTGACAACGAAATCTCCCCAGGACAATGCGCCGTAGACTGAAGGACAGGG
ATGGAAGGGCCTATTGGAGAGGAAGGACGGCCCCAACTTTTAACACGCGCATTCTGGG
TGCTGGGAGCGGTTAAAGCCAACATTCCCTGCCGGAAAACCGAGGCTCAGAGAGGGGCG
GCGCCCGCCGGGAGTCCCCCAGAGCGTGAGCAGCAGTTCGTGGCCGGAAAACCTGGCCA
GGTCCCCAGGGCCGGGCCGCTGGAGGACTGGGCACCGCAGGCAGGGCCAAACGGACTCA
CCATGGCGGGCGGCGCGGTCCCTTCAAGACAGCGGGTGGTCGCTTTGCAGCCGGACCCT
GGCTGGGCCATCACCTGGGGGAGGGGGGAGGGAACGCAGGCGGCGGCGGCTGCTG
(SEQ ID NO:18)

**Bisulfite-Modified LHX6 Region of Interest 1 Sequence:**

CGGTTTAGCGCGGGTTTTTTTTATTTCGGGTTTTAGTTTTTTCGGTTTTATTTATAGTT
AGAGGCGTTGAAGTTTAGAAGGTGTATGCGATTTTTTTATTTTTTTTCGTAATATTGA
GATTTAGGGAAATGGAAATAAATTTTTTTTATTTTTTAGACGGAATTCGGGGGTTTAGG
TAGATTTTAAGTTTTGTTTAAAGTTTCGTAGTCGGGTAGTAGTGGAGTTAGGATTTGGA
AGTAAGAGGATTTGCGGTGTTTTTTTGTAGTTTTTTGCGTTGCGTTTTACGTTTCGATA
ATACGTACGTAATATTTATTTTTGTTTTATTTTGAGTAGATATCGGTTTAGGATTTCGA
GGTCGTAGTTGGAGTAGATGTTTTTGTTTGTAGACGGTACGGAGGAGGCGGTAGAGGGC
GGTGAGTAGATAGATGGCGTGTTGGGCGTATATGGGGAGGTTTGATTTTCGTTTTTGTT
TAGAGTTTTTGTTAGGGTTTCGGCGTTAGATTGAGTTTGCGGCGGGGGAGAGAAGGAGA
GGAGCGTTGATTCGGGTATTTAGAGTTGGGGTTGTTTTGGAGATAAATTTGGGGTTTTC
GAGTATTAATTGATAACGAAATTTTTTTAGGATAATGCGTCGTAGATTGAAGGATAGGG
ATGGAAGGGTTTATTGGAGAGGAAGGGACGGTTTTAATTTTTAATACGCGTATTTTGGG
TGTTGGGAGCGGTTAAAGTTAATATTTTTTGTCGGAAAATCGAGGTTTAGAGAGGGGCG
GCGTTCGTCGGGAGTTTTTTAGAGCGTGAGTAGTAGTTCGTGGTCGGAAAATTTGGTTA
GGTTTTTAGGGTCGGGTCGTTGGAGGATTGGGTATCGTAGGTAGGGTTAAACGGATTTA
TTATGGCGGGCGGCGCGGTTTTTTTAAGATAGCGGGTGGTCGTTTTGTAGTCGGATTTT
GGTTGGGTTATTATTTGGGGGAGGGGGGAGGGAACGTAGGCGGCGGCGGTTGTTG
(SEQ ID NO:19)

### Exemplary Primer Sequences for Amplification of LHX6 Region of Interest 1:

Forward primer A: GGAATTCGGGGGTTTAGGTA (SEQ ID NO:20)
Reverse primer A: ACGCTCCTCTCCTTCTCTCC (SEQ ID NO:21)
Forward primer B: **GCGTATATGGGGAGGTTTGA** (SEQ ID NO:22)
Reverse primer B: **CCTACGATACCCAATCCTCCAAC** (SEQ ID NO:23)

### LHX6 Region of Interest 2: chr9:124985156-124990089 5'pad=250 3'pad=250 Non-Modified LHX6 Region of Interest 2 Sequence:

TTGGGAGCTCTTGGCCTCTCTTTGGGCAGAGTGCTCCAGTCCACATCCTACCAGACCCT
TGACCCCGGGCTTCAGGAAACAGAAGCCTAGAGAACCTCAGCATGTGACCCCTCCCTGG
GCCCCAGCTCCTTAGAGGAAAATTCTGCCTCCCACGCCACCATTCACACTCTCTCCAGG
AAGAACTTCCTCATAGCAGCCACCCAGAGCTTGGGCTGCAGTGCAGTGCAGACAATAGT
CCTTGCCTGGCACTCGATCGGCTTTTCTTTCCCTTTTTGTGGGTTTATTATTTTTGT
TACAGGTGGGGCTGCCAGGGATGGGTTATGCCTGCAGGGGGAGAAATGATCAGAAGAGC
CACAATTCTACCCTCAACCCAGGGAATGTGAGCTTTTACTGCCTCCATTTTCTTAACCA
GAAATCTGGTTTAGTTTTGCTTTCAGGGCTCTTGTCATTGGAAAAAGATTAGTGCTCAG
AACCTGCAGCCCAGAAATGGCCCAGTCCCATCCCTCCAGGCAGGCTCTGCTCCTCAAGC
TGCGTGGGGAAATGCCATCATGGGCTTGTGCCCAGCATCCACCCTGCCAATGATGACAA
CAGTGATAATCGAGGCATTCATACCAACCACCATTTAGGGAGCCTTTATAGAGACTTCC
CCCCAACAGTGGCCTCTTTGGCAGCTCTGGGAGACAGCCTTGGAAAGGGCGAACTAAGG
GCCGAGAGAAGCAGCAACTTGCGAAGGTCACTACTGGCCAGACTGGGCCCAGTCTCCTG
CCTCTTCCCACAGTGGCTGCACACCCTACTCTCTACCCCATTGCAGTAGGAAGAGAACT
CCAGAATAAGGGGAGGCTCTGAACAGTGGAGTCAGGATCCGTGTTTTGACTAGTTTCAA
GGTCTAGTAAAGCACAGTATCCAAGGAGGCTCAGTCCAGCATGGGCATGGGCTAGGGCT
GGAGAAGTAGAAAAGAAATTTGGGCAAGTAGTGAATAAGACTGCCAAATCTATTGTTAG
GAGAGCCAGGAGAGGTGTCCTAGTGGTGCCTTAGTCTATGGGAGAGACCCAGACACCAT
TCTGCTGGGTTCCCAAGCTGGCACCTTAAATTTAGTAGCTGATGAATCCAAGGAGCCCC
CAAGCATCATCTGGCATGTTTTTTTGTATGTTTGTTTGTTTTTTGAGACAGAGTC
TCTCTCTGTTACCCAGGCTGGAGTGTAGTGACGTGATCTTGGCTCACCACAATGTCCGC
CTCCCAGGTTCAAGTGATTCTCTTGCCTCAGCCTCCTGAGTAGCTGGGACTACAGGCAC
GTGCCACCACACCTGGCTAAGTTTTGTATTTTTAGTAGAGATGGGTTTCACCATGTTGG
CCAGGATGGTCTCGATCTCCTGACCTTGTGATCTACCAGCCTTGGCCTCCCAAAGTGCT
GCGATTATAGGTGTGAGCCACCACACCCGGCCCATCTGGCATGTTTTATACCAACTGGA
GTCAGGCCTAGAGCAGGGAGGTGGCTAGACAGTGACTGTAACCTCCATTTCCTGTCCTG
GCCAAACCAATACTCAGATGGGACAGTGAGAGGAGACATCACACTCTGGTTACACACCA

TTAACCCCTGTCTGACTACACACACACACACACAGCCGTCCAGATTCACAAATACGT
TCTTATCACCCCCTTCATCCCTCAGTGCCTGGGTGGAGGCCCTTAAGCAGGAATGCCCC
CTCAGAGGAGAGGGTGGGAGTGGAATGATGGGATTTCAGAATCCCCTCACCTCACCCTC
TCACCTCCAGATCCTAGTCCAGATGTAATCCTGTCCTCACCACTTTGCGTGCACGTGCA
CGCACACACACATGCACACACACAGTCTCAACATTCACATACAGACACCCACTCTTGAC
ACCCACACATGAACTAAAAGACAAACGTTGGTCCATAAAGGAACACACATGCTATCTAC
ATACCCCCAACCCTGCCCAGTGATGCTTTCCCCAGCACTACTGCACAGATCCCAGACAA
TACAGACACACAGGCACTCCTTGCACACTGAGTCCTAGGCCACTTGGTTGAGCTGCCCC
CACCCCACCCCAGCTGCACACTCTTCTACATCCATCCCATAAAGGAAGAGTCCGTCTCT
TTAACCATGCTTCTCATGGTTTTCAGAGTACCCTTGCCCCAAATGCTGCCAACCTCCCC
CTAGACTCAAGATCTTCTCTTTCCAGATCGCTGGAGGCTCAATGCCAGTGCACAGGAGG
AGATCGCCTTCCACCATATTCCCTGACTCCCAGAGGCTCTTTCTAGGGCAGGGGTCTGG
CCAGGAAGACTGAAGCCATGACGTCTTCCTGTGCAAGGATAGGAAAGATGTCTGTGGGT
TGGAGGGTAGTTATAGGGAAGCAGCTTTCTCCACTTTTTGAGAAAGCACTTGGGAACAA
GCATCCCTCTCAGGAAGTGATTCTCCACCATTCTCCTCTGATCCTATAGGCCTGGTGGT
CCAAGCCTCAGGCCAGTTGATGCCTCCCACTGGGATCAGCAGACAAGGCCATGACCCTG
CAGTGACCTCGACCCCATAATCAGGTCAGGTCCGGGCTTCATGGCTAGAAGCGAGGCCC
AGGCGAGTGGCCAAGACGCGGCAGGACAGGAGGATGCGCCTGGCTGCTCTCGGCTGCTG
GACGCGGCCTGCAGTAGGGAGAGCGCAGCCCGGGGTAATCCCACTCAGGGCTCCGGGTG
CCCAGTTCTCCCACTTCCCATCCTGGCGGCCTAAAGGCAAGGCCTAGACCCTGGAGAGG
CCAAAGTCGGGCCAAAACAACAGCGAAGCTGGGACTGGAACAGGCGCTGGGGGCCTGCG
CATGACCCCAGGCTCAGAATGCGACACTCCAGTGACGCCGGGGAATGAGTTGAGATGGA
GCCGGAGACAGAGGTTGGGGTTCAGGACGAGGCGCTTGGGTATGGATCGGATCTGAGA
CGGTTGCGGCGCTGGTGCTTTGGGCTGGACCAGACTCGAACCCAGAGCGGAGCGAGGGT
CAGAGCCAGGGCAAGAGTCCGAAGCCCAGCTCAGCGTCCAGCCCGCGAGAGACCCCACC
ATGAATGACGTCCGAGTAGGGTTGGAACTCGAAGCCCGAACGCACTGGAAATGCAGCCG
GACGACGGCTGGGATCCGAGTGGGTCCGGGCCAGAAATGGGGACCTCAGAGCTCCACCG
AGGCGCTCGAGGTCAGAACTGAAGCCTGAGACTTAGGCAGGACCCGAGACAGAGCCAGA
GACGATACCGAAACCCAATGGACCGCGAGGACCGAAGGCAGATCCGGGGCGCAAACCTG
TGCAGGCACTGGCGCGCTGCCTGGAGCTCTGGGTTCGCGCCGCTGAGCGCCGGCAGGTT
GGACCAGCGCTGCGCGCCGGAAGTGCACTCGGACGCCGGGGTTCTGGAGGAGAGACCA
CACGCCGCAAAAGTGGCCTCCGAATGCGCCCGGGGCCTGCCCTCGGCGACACTGCTGGC
TCGGCGGCCGCAGCCTACCTGAAGTAGTCCATCTTGCAGAAGATCTCCTTGTTCTTGAT
GTAGCAGCTGTTCTGCTGCCTCAGCGACGTGCGACACACGGAGCACTCGAGGCACCGCA
CGTGCCAGATGAGGTTGTTGACCTGGGGACGGGGCGGGGACGGAGGTCGGCTCAGCGCG

GGCCTCTTTCACTCCGGGCCCCAGCCTTCCCGGTCTCATTTACAGTCAGAGGCGCTGAA
GCTCAGAAGGTGCATGCGATTCCCCTATTTTTCTCCCGTAATACTGAGACTCAGGGAAA
TGGAAATAAACTCTTCTTATTTTTCAGACGGAACCCGGGGGCTCAGGCAGACCCTAAGT
CTTGCCCAAAGCTTCGCAGTCGGGCAGCAGTGGAGCCAGGATTTGGAAGTAAGAGGACC
TGCGGTGCTTCCCTGCAGTCTCCTGCGCTGCGTCCCACGCCCCGACAACACGCACGCAA
CACCTACCCCTGTCTCACCTTGAGCAGATATCGGTCCAGGATCTCGAGGCCGCAGCTGG
AGCAGATGTTCTTGCCTGCAGACGGCACGGAGGAGGCGGCAGAGGGCGGTGAGCAGACA
GATGGCGTGCTGGGCGTACATGGGGAGGCCTGACCCTCGTCCTTGTCCAGAGCTCCTGC
CAGGGCCTCGGCGTCAGACTGAGCCTGCGGCGGGGAGAGAAGGAGAGGAGCGCTGATC
CGGGCACCCAGAGTTGGGGCTGCCTTGGAGACAAATCTGGGGCCCCCGAGCACCAATTG
ACAACGAAATCTCCCCAGGACAATGCGCCGTAGACTGAAGGACAGGGATGGAAGGGCCT
ATTGGAGAGGAAGGGACGGCCCCAACTTTTAACACGCGCATTCTGGGTGCTGGGAGCGG
TTAAAGCCAACATTCCCTGCCGGAAAACCGAGGCTCAGAGAGGGCGGCGCCCGCCGGG
AGTCCCCCAGAGCGTGAGCAGCAGTTCGTGGCCGGAAAACCTGGCCAGGTCCCCAGGGC
CGGGCCGCTGGAGGACTGGGCACCGCAGGCAGGGCCAAACGGACTCACCATGGCGGGCG
GCGCGGTCCCTTCAAGACAGCGGGTGGTCGCTTTGCAGCCGGACCCTGGCTGGGCCATC
ACCTGGGGGAGGGGGGGAGGGAACGCAGGCGGCGGCGGCTGCTGAACTGGCTCCGCACA
GCCTGAGCCCAGCGCCTCCCCGCGCCTGTTATATAAACCGGCGCCGAACAATGAGTCCT
AACTTTGTAGTGGGCATTTAAAGCCCTTCCCCACAAAAGCGGTGTCTCTCTAATGAAGC
AATTTGAATTTGGATTGGATTTTTTCCCTCTCTCTCCCCCTCTCCCTGCACTTAACCCG
TGGCTCTTGAAGTAATCGCTTAGTTCCCTTGCAATCCAAGCCTCTGAGGGGGGAAGAAA
AACACGCGCACACACACACAAACTACCTGCAATTAGA (SEQ ID NO:24)

**Bisulfite-Modified LHX6 Region of Interest 2 Sequence:**

TTGGGAGTTTTTGGTTTTTTTTTGGGTAGAGTGTTTTAGTTTATATTTTATTAGATTTT
TGATTTCGGGTTTTAGGAAATAGAAGTTTAGAGAATTTTAGTATGTGATTTTTTTTTGG
GTTTTAGTTTTTTAGAGGAAAATTTTGTTTTTTACGTTATTATTTATATTTTTTTTTAGG
AAGAATTTTTTTATAGTAGTTATTTAGAGTTTGGGTTGTAGTGTAGTGTAGATAATAGT
TTTTGTTTGGTATTCGATCGGTTTTTTTTTTTTTTTTTTGTGGGTTTTATTATTTTTGT
TATAGGTGGGGTTGTTAGGGATGGGTTATGTTTGTAGGGGGAGAAATGATTAGAAGAGT
TATAATTTTATTTTTAATTTAGGGAATGTGAGTTTTTATTGTTTTTATTTTTTTAATTA
GAAATTTGGTTTAGTTTTGTTTTTAGGGTTTTTGTTATTGGAAAAAGATTAGTGTTTAG
AATTTGTAGTTTAGAAATGGTTTAGTTTTATTTTTTTAGGTAGGTTTTGTTTTTTAAGT
TGCGTGGGGAAATGTTATTATGGGTTTGTGTTTAGTATTTATTTTGTTAATGATGATAA
TAGTGATAATCGAGGTATTTATATTAATTATTATTTAGGGAGTTTTTATAGAGATTTTT

TTTTAATAGTGGTTTTTTTGGTAGTTTTGGGAGATAGTTTTGGAAAGGCGAATTAAGG
GTCGAGAGAAGTAGTAATTTGCGAAGGTTATTATTGGTTAGATTGGGTTTAGTTTTTTG
TTTTTTTTTATAGTGGTTGTATATTTTATTTTTTATTTTATTGTAGTAGGAAGAGAATT
TTAGAATAAGGGGAGGTTTTGAATAGTGGAGTTAGGATTCGTGTTTTGATTAGTTTTAA
GGTTTAGTAAAGTATAGTATTTAAGGAGGTTTAGTTTAGTATGGGTATGGGTTAGGGTT
GGAGAAGTAGAAAAGAAATTTGGGTAAGTAGTGAATAAGATTGTTAAATTTATTGTTAG
GAGAGTTAGGAGAGGTGTTTTAGTGGTGTTTTAGTTTATGGGAGAGATTTAGATATTAT
TTTGTTGGGTTTTTAAGTTGGTATTTTAAATTTAGTAGTTGATGAATTTAAGGAGTTTT
TAAGTATTATTTGGTATGTTTTTTTGTATGTTTGTTTGTTTGTTTTTTGAGATAGAGTT
TTTTTTTGTTATTTAGGTTGGAGTGTAGTGACGTGATTTTGGTTTATTATAATGTTCGT
TTTTTAGGTTTAAGTGATTTTTTTGTTTTAGTTTTTTGAGTAGTTGGGATTATAGGTAC
GTGTTATTATATTTGGTTAAGTTTTGTATTTTTAGTAGAGATGGGTTTTATTATGTTGG
TTAGGATGGTTTCGATTTTTTGATTTTGTGATTTATTAGTTTTGGTTTTTTAAAGTGTT
GCGATTATAGGTGTGAGTTATTATATTCGGTTTATTTGGTATGTTTTATATTAATTGGA
GTTAGGTTTAGAGTAGGGAGGTGGTTAGATAGTGATTGTAATTTTTATTTTTTGTTTTG
GTTAAATTAATATTTAGATGGGATAGTGAGAGGAGATATTATATTTGGTTATATATTA
TTAATTTTTGTTTGATTATATATATATATATATATAGTCGTTTAGATTTATAAATACGT
TTTTATTATTTTTTTTATTTTTTAGTGTTTGGGTGGAGGTTTTTAAGTAGGAATGTTTT
TTTAGAGGAGAGGGTGGGAGTGGAATGATGGGATTTTAGAATTTTTTTATTTTATTTTT
TTATTTTTAGATTTTAGTTTAGATGTAATTTTGTTTTTATTATTTTGCGTGTACGTGTA
CGTATATATATATGTATATATATAGTTTTAATATTTATATATAGATATTTATTTTTGAT
ATTTATATATGAATTAAAAGATAAACGTTGGTTTATAAAGGAATATATATGTTATTTAT
ATATTTTTAATTTTGTTTAGTGATGTTTTTTTTAGTATTATTGTATAGATTTTAGATAA
TATAGATATATAGGTATTTTTTGTATATTGAGTTTTAGGTTATTTGGTTGAGTTGTTTT
TATTTTATTTTAGTTGTATATTTTTTTTATATTTATTTTATAAAGGAAGAGTTCGTTTTT
TTAATTATGTTTTTTTATGGTTTTTAGAGTATTTTTGTTTTAAATGTTGTTAATTTTTTT
TTAGATTTAAGATTTTTTTTTTTTAGATCGTTGGAGGTTTAATGTTAGTGTATAGGAGG
AGATCGTTTTTTATTATATTTTTTGATTTTTAGAGGTTTTTTTTAGGGTAGGGGTTTGG
TTAGGAAGATTGAAGTTATGACGTTTTTTTGTGTAAGGATAGGAAAGATGTTTGTGGGT
TGGAGGGTAGTTATAGGGAAGTAGTTTTTTTTATTTTTTGAGAAAGTATTTGGGAATAA
GTATTTTTTTTTAGGAAGTGATTTTTTATTATTTTTTTTTTGATTTTATAGGTTTGGTGGT
TTAAGTTTTAGGTTAGTTGATGTTTTTTATTGGGATTAGTAGATAAGGTTATGATTTTG
TAGTGATTTCGATTTTATAATTAGGTTAGGTTCGGGTTTTATGGTTAGAAGCGAGGTTT
AGGCGAGTGGTTAAGACGCGGTAGGATAGGAGGATGCGTTTGGTTGTTTTCGGTTGTTG
GACGCGGTTTGTAGTAGGGAGAGCGTAGTTCGGGGTAATTTTATTTAGGGTTTCGGGTG

TTTAGTTTTTTTATTTTTTATTTTGGCGGTTTAAAGGTAAGGTTTAGATTTTGGAGAGG
TTAAAGTCGGGTTAAAATAATAGCGAAGTTGGGATTGGAATAGGCGTTGGGGGTTTGCG
TATGATTTTAGGTTTAGAATGCGATATTTTAGTGACGTCGGGGAATGAGTTGAGATGGA
GTCGGAGATAGAGGTTGGGGTTTAGGACGAGGCGTTTGGGTATGGGATCGGATTTGAGA
CGGTTGCGGCGTTGGTGTTTTGGGTTGGATTAGATTCGAATTTAGAGCGGAGCGAGGGT
TAGAGTTAGGGTAAGAGTTCGAAGTTTAGTTTAGCGTTTAGTTCGCGAGAGATTTTATT
ATGAATGACGTTCGAGTAGGGTTGGAATTCGAAGTTCGAACGTATTGGAAATGTAGTCG
GACGACGGTTGGGATTCGAGTGGGTTCGGGTTAGAAATGGGGATTTTAGAGTTTTATCG
AGGCGTTCGAGGTTAGAATTGAAGTTTGAGATTTAGGTAGGATTCGAGATAGAGTTAGA
GACGATATCGAAATTTAATGGATCGCGAGGATCGAAGGTAGATTCGGGGCGTAAATTTG
TGTAGGTATTGGCGCGTTGTTTGGAGTTTTGGGTTCGCGTCGTTGAGCGTCGGTAGGTT
GGATTAGCGTTGCGCGTCGGAAGTGTATTCGGGACGTCGGGGTTTTGGAGGAGAGATTA
TACGTCGTAAAAGTGGTTTTCGAATGCGTTCGGGGTTTGTTTTCGGCGATATTGTTGGT
TCGGCGGTCGTAGTTTATTTGAAGTAGTTTATTTTGTAGAAGATTTTTTTGTTTTTGAT
GTAGTAGTTGTTTTGTTGTTTTAGCGACGTGCGATATACGGAGTATTCGAGGTATCGTA
CGTGTTAGATGAGGTTGTTGATTTGGGGACGGGGCGGGGACGGAGGTCGGTTTAGCGCG
GGTTTTTTTTATTTCGGGTTTTAGTTTTTTCGGTTTTATTTATAGTTAGAGGCGTTGAA
GTTTAGAAGGTGTATGCGATTTTTTTATTTTTTTTTCGTAATATTGAGATTTAGGGAAA
TGGAAATAAATTTTTTTTATTTTTTAGACGGAATTCGGGGGTTTAGGTAGATTTTAAGT
TTTGTTTAAAGTTTCGTAGTCGGGTAGTAGTGGAGTTAGGATTTGGAAGTAAGAGGATT
TGCGGTGTTTTTTTGTAGTTTTTTGCGTTGCGTTTTACGTTTCGATAATACGTACGTAA
TATTTATTTTTGTTTTATTTTGAGTAGATATCGGTTTAGGATTTCGAGGTCGTAGTTGG
AGTAGATGTTTTTGTTTGTAGACGGTACGGAGGAGGCGGTAGAGGGCGGTGAGTAGATA
GATGGCGTGTTGGGCGTATATGGGGAGGTTTGATTTTCGTTTTTGTTTAGAGTTTTTGT
TAGGGTTTCGGCGTTAGATTGAGTTTGCGGCGGGGGAGAGAAGGAGAGGAGCGTTGATT
CGGGTATTTAGAGTTGGGGTTGTTTTGGAGATAAATTTGGGGTTTTCGAGTATTAATTG
ATAACGAAATTTTTTTAGGATAATGCGTCGTAGATTGAAGGATAGGGATGGAAGGGTTT
ATTGGAGAGGAAGGGACGGTTTTAATTTTTAATACGCGTATTTTGGGTGTTGGGAGCGG
TTAAAGTTAATATTTTTTGTCGGAAAATCGAGGTTTAGAGAGGGGCGGCGTTCGTCGGG
AGTTTTTTAGAGCGTGAGTAGTAGTTCGTGGTCGGAAAATTTGGTTAGGTTTTTAGGGT
CGGGTCGTTGGAGGATTGGGTATCGTAGGTAGGGTTAAACGGATTTATTATGGCGGGCG
GCGCGGTTTTTTTAAGATAGCGGGTGGTCGTTTTGTAGTCGGATTTTGGTTGGGTTATT
ATTTGGGGGAGGGGGGGAGGGAACGTAGGCGGCGGCGGTTGTTGAATTGGTTTCGTATA
GTTTGAGTTTAGCGTTTTTTCGCGTTTGTTATATAAATCGGCGTCGAATAATGAGTTTT
AATTTTGTAGTGGGTATTTAAAGTTTTTTTTTTATAAAAGCGGTGTTTTTTTTAATGAAGT

AATTTGAATTTGGATTGGATTTTTTTTTTTTTTTTTTTTTTTTTTTGTATTTAATTCG

TGGTTTTTGAAGTAATCGTTTAGTTTTTTTGTAATTTAAGTTTTTGAGGGGGAAGAAA

AATACGCGTATATATATATAAATTATTTGTAATTAGA (SEQ ID NO:25)

### Exemplary Primer Sequences for Amplification of LHX6 Region of Interest 2 (Chr9: 124985406-124989839):

Forward primer1: TTGGGTTGTAGTGTAGTGTAGA (SEQ ID NO:26)
Reverse primer1: AATTCGCCCTTTCCAAAACT (SEQ ID NO:27)
Forward primer2: **GAGAGCGTAGTTCGGGGTAA** (SEQ ID NO:28)
Reverse primer2: **TCTCGCGAACTAAACGCTAAA** (SEQ ID NO:29)
Forward primer3: **AATTTAATGGATCGCGAGGA** (SEQ ID NO:30)
Reverse primer3: **CCCCAAATCAACAACCTCAT** (SEQ ID NO:31)
Forward primer4: **GGTGTTGGGAGCGGTTAAAG** (SEQ ID NO:32)
Reverse primer4: **AAAACTCATTATTCGACGCC** (SEQ ID NO:33)

### GABRA2

### GABRA2 Region of Interest: chr4:46,391,392-46,391,695 5'pad=250 3'pad=250 Non-Modified GABRA2 Region of Interest Sequence:

GAGAGATGGGTACTTCACGCCACAACCCCCTCAACCAAGACCACCTCCGCAACCACAGC
GGGAAAAGCTAGGGGCAGGCAGCCCACTTTTGCAATTACTCTACATTACAGTGAACTGC
GGTCCTCACGTCTTCTTTTCTTCACTCCCCTTAACAAAATAAAATAAATAATTTCCCTT
CCTGCCCACCCACGGTTGCCCAAGACCAAGTCTTAGAGGCAGCCGGGTTCCGATCAAGT
GCTGCGAAACGACGCGTTTGACAGCTGCTCTGGAGCCGAGGATCACAATAAGAGAAGGC
GCGTGAGGCTCCGGCAGCAAACACCAGCCAGGCAGGCAGCGGTAATCACAGTGAGCAAA
GTTGAAGACTATAAAAGAGCCAGGCTAACGTGCTGCCGAGCAGGTGTCCTGGATTTTAT
TGTAGTTGCAAATATGCTTCTGGTGATAAGTATCGAAATTATTTTTAAGTGCGCTGGG
GAGGAGGGAGGTGCGGAATTGAGCCTCCTCCGTTTCCAGGGAGCCTCTGAAAGTGGGG
TGGGGGGAGATGAGGCATGCACGCGAGGCAAAGACAAACAGGAAAGTGTCTCTATCGGG
ACCAACGTGTGCGACCCTACCTGAAACGGCAAGCAGAATTCGGTGTTTTCTTCCTTTTG
CCCTGATCTTGACGAGATAGGAAACTTGGGAGAGAGAGAGAGAGAGAGAGAGAGAGAGA
GAGAGAGAGAGAGAGAGAGAGAGAGAGAGACCGAGACTGCAGCAGCCAAGAGAGCGTGG
AGCGATGGGCTGGTGGAAGCCGGAGAGGAGCGCTAGG (SEQ ID NO:34)

**Bisulfite-Modified GABRA2 Region of Interest Sequence:**

GAGAGATGGGTATTTTACGTTATAATTTTTTTAATTAAGATTATTTTCGTAATTATAGC
GGGAAAAGTTAGGGGTAGGTAGTTTATTTTTGTAATTATTTTATATTATAGTGAATTGC
GGTTTTTACGTTTTTTTTTTTTTATTTTTTTTAATAAAATAAAATAAATAATTTTTTTT
TTTGTTTATTTACGGTTGTTTAAGATTAAGTTTTAGAGGTAGTCGGGTTTCGATTAAGT
GTTGCGAAACGACGCGTTTGATAGTTGTTTTGGAGTCGAGGATTATAATAAGAGAAGGC
GCGTGAGGTTTCGGTAGTAAATATTAGTTAGGTAGGTAGCGGTAATTATAGTGAGTAAA
GTTGAAGATTATAAAAGAGTTAGGTTAACGTGTTGTCGAGTAGGTGTTTTGGATTTTAT
TGTAGTTGTAAATATGTTTTTGGTGATAAGTATCGAAATTATTTTTTAAGTGCGTTGGG
GAGGAGGGAGGTGCGGGAATTGAGTTTTTTTCGTTTTTAGGGAGTTTTTGAAAGTGGGG
TGGGGGGAGATGAGGTATGTACGCGAGGTAAAGATAAATAGGAAAGTGTTTTTATCGGG
ATTAACGTGTGCGATTTTATTTGAAACGGTAAGTAGAATTCGGTGTTTTTTTTTTTTTG
TTTTGATTTTGACGAGATAGGAAATTTGGGAGAGAGAGAGAGAGAGAGAGAGAGAGAGA
GAGAGAGAGAGAGAGAGAGAGAGAGAGAGAGATCGAGATTGTAGTAGTTAAGAGAGCGTGG
AGCGATGGGTTGGTGGAAGTCGGAGAGGAGCGTTAGG (SEQ ID NO:35)

### Exemplary Primer Sequences for Amplification of GABRA2 Region of Interest:

Forward primer: TCGGGTTTCGATTAAGTGTTG (SEQ ID NO:36)
Reverse primer: TTTCAAATAAAATCGCACAC (SEQ ID NO:37)

In some embodiments, the biomarker panel is a microarray.

In some embodiments, the primers or probes are biotinylated and bind to streptavidin coated substrates for selection of the DMPs or DMP-associated genes targeted by the probe or primers. In some embodiments, the streptavidin-coated substrates are beads.

Described herein are methods to assay the methylation status of DMPs or DMP-associated genes described herein to diagnose or give a prognosis for hypersomnolence, idiopathic hypersomnia, or long sleep time in an individual. Methylation levels of at least one DMP or DMP-associated gene recited in Tables 1 and 2, or any combination of DMPs or DMP-associated genes recited therein, is measured in target DNA from a biofluid sample from a subject.

Methylation may be quantified by any suitable means known in the art. Suitable methods for assaying quantification are disclosed, for example, by Kurdyukov and Bullock ("DNA methylation analysis: Choosing the right method," Biology, 2016, 5(3)). Suitable methods for quantifying or assaying methylation may include, but are not limited to methylation specific polymerase chain reaction (PCR), high resolution melting, cold-PCR, pyrosequencing, PCR and sequencing, bead array, and digestion-based assay followed by PCR or quantitative PCR (qPCR).

In some embodiments, the target DNA is bisulfite modified. Bisulfite treatment mediates the deamination of cytosine to uracil, whereby the modified uracil residue will be read as a thymine as determined by PCR-amplification and sequencing. Methylated cytosine (5mC) resides are protected from this conversion and will remain as cytosine.

To examine the methylation status of the DMP or DMP-associated gene, target genomic DNA may be isolated from a biofluid sample from a subject. In some embodiments, the target DNA is isolated from a biofluid sample from a human.

As used herein, "biofluid" refers to a biological fluid produced by a subject. Suitable biofluids include, but are not limited to blood, saliva, sweat, urine, semen, tears, and cerebrospinal fluid. In some embodiments, the biofluid is selected from the group consisting of blood, saliva, sweat, urine, semen, tears, and cerebrospinal fluid. In some embodiments, the biofluid is blood. In some embodiments, the biofluid is saliva.

Following isolation of target DNA, the target DNA will be contacted with probes specific to the DMPs outlined in Tables 1 and 2 to isolate and enrich these genomic regions from the target DNA sample. In some embodiments, sequences of the DMP is used as bait to isolate the genomic regions of interest for amplification and sequencing.

After isolation and enrichment of the genomic regions within the target DNA that include the DMP, methylated adapters are ligated to the enriched regions. The sample with the ligated methylated adapters may then be subject to sodium bisulfite modification.

In some embodiments, the target DNA sample is bisulfite modified without first enriching the sample of the genomic regions of the DMPs. In some embodiments, the target DNA is first bisulfite modified then enriched for the genomic regions of the DMPs.

In general, target DNA or bisulfite modified target DNA is subject to amplification. The amplification may be polymerase chain reaction (PCR) amplification. PCR amplification will include single or multiple pair(s) of primers and probes at specific DMPs outlined in Tables 1 and 2. The target DNA amplification and methylation quantification will be evaluated in one or multiple tubes.

In some embodiments, methylation is quantified by amplification and sequencing of target DNA. Bisulfite modified target DNA may be subject to PCR to amplify target regions comprising DMPs outlined in Tables 1 and 2. The PCR reaction mixture typically includes at least one pair of primers designed to target a DMP detailed in Tables 1 and 2, PCR buffer, dNTPs (e.g., adenine, thymine, cytosine and guanine), MgCl₂, and polymerase. PCR amplification generally includes the steps of heating the reaction mixture to separate the strands of the target DNA, annealing the primers to the target DNA by cooling the reaction mixture, allowing the polymerase to extend the primers by addition of NTPs, and repeating the process at least 2, at least 5, at least 10, at least 15, at least 20, at least 25, or at least 30 times to produce a PCR amplification product. If the target DNA in the reaction mixture is single stranded, the initial heating step may be omitted, however this heating step will need to be included when the second and subsequent times the reaction is completed to separate the extended primer strands from the opposite strand and DNA (e.g., the target DNA or another previously extended primer strand). In some embodiments, the target DNA is bisulfite modified prior to amplification.

In some embodiments, the bisulfite modified target DNA is used in a methylation-specific-quantitative PCR (MS-QPCR) reaction such as MethylLight (WO 2000/070090A1) or HeavyMethyl (WO 2002/072880A2). For example, a reaction mixture for use in a MethylLight methylation specific PCR reaction would contain primers and probes specific to the DMPs recited in Tables 1 and 2, PCR buffer, dNTPs (e.g., adenine, thymine, cytosine and guanine), MgCl₂, and polymerase. A typical kit for methylation specific PCR may include primers and probes specific to the DMPs recited in Tables 1 and 2, wild type reference gene primers such as β-actin, PCR buffer, dNTPs, MgCl₂, polymerase, positive and negative methylation controls, and a dilution reference. The MS-QPCR may be carried out in one or multiple reaction tubes.

In some embodiments, either the forward or reverse primer of the primer pair used in the PCR amplification reaction is biotinylated. When a biotinylated primer is used in a PCR amplification reaction, PCR products may be purified, captured, and/or sorted with a streptavidin coated substrate. In some embodiments, the substrate is a streptavidin coated bead. In some embodiments, the beads are streptavidin sepharose beads. In some embodiments, the beads are magnetic.

In some embodiments, the PCR amplification product is contacted with one or more probes specific for and complementary to a DMP detailed in Tables 1 and 2. The probe may be biotinylated. The PCR amplification product and probe mixture can then be purified, captured and/or sorted with a streptavidin coated substrate. In some embodiments, the substrate is a streptavidin coated bead. In some embodiments, the beads are streptavidin sepharose beads. In some embodiments, the streptavidin beads are magnetic.

In some embodiments, methylation is quantified using pyrosequencing. Bisulfite modified target DNA may be subject to PCR to amplify target regions comprising DMPs outlined in Tables 1 and 2 as described above. PCR amplification products are purified, denatured to single-stranded DNA, and annealed to a sequencing primer for methylation quantification by pyrosequencing of the DMP or DMP-associated gene as detailed in Tables 1 and 2. In some embodiments, methylation may be quantified with PyroMark^{™}MD Pyrosequencing System (Qiagen) using PyroPyroMark^{®} Gold Q96 Reagents (Qiagen, Cat# 972804) (QIAGEN PyroMark Gold Q96 Reagents Handbook 08/2009, 36-38).

In some embodiments, bisulfite treated DNA is subject to an Invader^{®} assay to detect changes in methylation. The Invader^{®} assay entails the use of Invader^{®} chemistry (Hologic Inc.; invaderchemistry.com; Day, S., and Mast, A. Invader assay, 2004; Chapter in Encyclopedia of Diagnostic Genomics and Proteomics. Marcel Dekker, Inc., U.S. Patent Nos. 7,011,944; 6,913,881; 6,875,572 and 6,872,816). In the Invader^{®} assay, one would use a structure-specific flap endonuclease (FEN) to cleave a three-dimensional complex formed by hybridization of C/T specific overlapping oligonucleotides to target DNA containing a CG site. Initial PCR amplification of the bisulfite treated target DNA may be necessary if the quantity of the bisulfite treated target DNA is less than 20 ng.

In some embodiments, the methylation status is measured by methylation-specific PCR, quantitative methylation-specific PCR, methylation-sensitive DNA restriction enzyme analysis, or bisulfite genomic sequencing PCR, or quantitative bisulfite pyrosequencing. See, *e.g.,* U.S. Patent Nos. 10,370,726 and 10,934,592.

Methylation status of the DMPs and DMP-associated genes described herein may be useful in identification and management of both acute and chronic sleep problems in a subject. Assays described herein are useful for identifying insufficient sleep time in persons complaining of daytime sleepiness improving patient care in sleep medicine. The assays described can also be used as to quantify change in sleep duration in research and clinical care (e.g., identifying increased sleep time caused by a medication; clarifying whether a behavioral intervention increases sleep time in people who are chronically sleep restricted, etc.) or in general assessments of sleep time in a subject.

For applications in general wellness assessments of subject (e.g., a healthy subject), the methylation assays to evaluate sleep in a subject as described herein can be used to optimize mental performance, to optimize physical performance, to optimize recovery after mental exertion, to optimize recovery after physical exertion, to optimize academic performance, to help control weight, to help reduce weight, to help reduce obesity, to help control acute pain, to help control chronic pain, and to prevent the onset of medical, psychiatric, and neurological disease.

Methods described herein for assaying methylation to evaluate sleep in a subject as described herein may be used to increase or optimize fecundity or fertility in a subject. Likewise, the methods described herein for assaying methylation to evaluate sleep in a subject as described herein may be used to aid in a subject's general wellness during pregnancy. The methods described herein for assaying methylation to evaluate sleep in a subject may be used to aid in post-partum recovery and mental and physical wellness post-partum.

In some embodiments, the methods described herein for assaying methylation to evaluate sleep in a subject may be used to increase or optimize wound healing. The methods may also help in physical or mental trauma recovery in an individual or in post-surgical recovery.

In some embodiments, the methods described herein for assaying methylation may be used to evaluate sleep duration and sleepiness in a subject. In some embodiments, the methods described herein for assaying methylation may be used to evaluate sleep duration and sleepiness in a subject diagnosed with a sleep disorder (e.g., insomnia, restless legs syndrome, circadian rhythm sleep-wake disorders, sleep apnea, and parasomnias).

In some embodiments, methods described herein for assaying methylation may be used to evaluate sleep duration in a subject with a medical or neurological disorder (e.g., cancer, coronary heart disease, atrial fibrillation, congestive heart failure, epilepsy/seizure disorders, thyroid dysfunction, Parkinson's disease, fibromyalgia, Myotonic dystrophy, Kline Levin Syndrome, rheumatologic disease such as systemic lupus erythematosus, renal disease, hepatic disease such as non-alcoholic fatty liver disease, diabetes, hypertension, and asthma). In some embodiments, methods described herein for assaying methylation may be used to evaluate sleep duration in a subject with a psychiatric disorder (e.g., mood, thought, attentional, psychotic, personality, anxiety, and PTSD)

Assaying methylation status of the DMPs and DMP-associated genes described herewith will be beneficial as a measure of habitual sleep duration to help subjects identify if they are sleeping too little or too much to maintain personal wellness. In some embodiments, assays for determining the methylation status of DMPs or DMP-associated genes described herein may be incorporated into a person fitness tracker and the target DNA may be from sweat.

Assaying methylation status of the DMPs and DMP-associated genes described herein may also be beneficial for use in employee health screening (e.g., to determine if an employee is at risk for chronic disease), for use in actuarial sciences and risk estimation (e.g., to determine if an individual has sleep insufficiencies or excessive sleep times that would be associated with increased mortality), to monitor sleep and its impact on chronic disease treatment (e.g., identifying and characterizing sleep duration can improve management of chronic illnesses such as diabetes, asthma, and hypertension), identification of individuals who may be at risk for psychological disorders or mental illness (e.g. too much or too little sleep are associated with and may precede major depressive disorder, manic episodes, etc. allowing for targeted intervention strategies to prevent negative mental illness outcomes), and to determine the factors associated with attention and cognitive disorders (e.g. characterization of sleep loss as a contributing factor to attention-deficit hyperactivity disorder and Alzheimer's disease).

Methylation status of the DMPs and DMP-associated genes described herein may also be beneficial in occupational health and safety applications. For example, identification of people who are sleeping too little to safely perform their required occupational duties (e.g., airplane pilots, bus/train drivers, long haul truckers, etc.), forensic applications (e.g., determining if reduced sleep duration or excessive sleep preceding an accident was a contributing factor in the accident, etc.), and in law-enforcement (e.g., analysis of compliance or non-compliance with fatigued-driver laws where it is a crime to drive if a person has been awake for greater than 24 hours, etc.).

In some embodiments, the methods described herein additionally include a step of treating or providing treatment recommendations to a subject diagnosed with hypersomnolence, idiopathic hypersomnia, or long sleep time. Suitable treatments include administering to a subject a wake promoting medication or stimulant (e.g., amphetamines, modafinil, etc.), administering to a subject an oxybate or derivative thereof (e.g., sodium oxybate), or having the subject undergo sleep extension therapy (e.g., sleeping 1-2 hours longer to see if this improves symptoms). In some embodiments, suitable treatments may include changing one or more sleep practices to improve sleep quality (e.g., amount of light, ambient noise, temperature, etc.), changing the time of day a subject sleeps (e.g., going to bed earlier or staying up later), having a subject nap more or less during the day, or administrating to a subject one or more over the counter sleep promoting aids such as melatonin.

The genomic positions described herein refer to genomic positions as provided in the UCSC hg19 human reference genome (Fujita PA, Rhead B, Zweig AS, Hinrichs AS, Karolchik D, Cline MS, Goldman M, Barber GP, Clawson H, Coelho A, Diekhans M, Dreszer TR, Giardine BM, Harte RA, Hillman-Jackson J, Hsu F, Kirkup V, Kuhn RM, Learned K, Li CH, Meyer LR, Pohl A, Raney BJ, Rosenbloom KR, Smith KE, Haussler D, Kent WJ. The UCSC Genome Browser database: update 2011. Nucleic Acids Res. 2011 Jan;39(Database issue):D876-82. doi: 10.1093/nar/gkq963.) and positions in other genomes aligning thereto. Suitable alignment methods are known in the art. Alignments are typically performed by computer programs that apply various algorithms, however it is also possible to perform an alignment by hand. Alignment programs typically iterate through potential alignments of sequences and score the alignments using substitution tables, employing a variety of strategies to reach a potential optimal alignment score. Commonly-used alignment algorithms include, but are not limited to, CLUSTALW, (see, Thompson J. D., Higgins D. G., Gibson T. J., CLUSTAL W: improving the sensitivity of progressive multiple sequence alignment through sequence weighting, position-specific gap penalties and weight matrix choice, Nucleic Acids Research 22: 4673-4680, 1994); CLUSTALV, (see, Larkin M. A., et al., CLUSTALW2, ClustalW and ClustalX version 2, Bioinformatics 23(21): 2947-2948, 2007); Jotun-Hein, Muscle et al., MUSCLE: a multiple sequence alignment method with reduced time and space complexity, BMC Bioinformatics 5: 113, 2004); Mafft, Kalign, ProbCons, and T-Coffee (see Notredame et al., T-Coffee: A novel method for multiple sequence alignments, Journal of Molecular Biology 302: 205-217, 2000). Exemplary programs that implement one or more of the above algorithms include, but are not limited to MegAlign from DNAStar (DNAStar, Inc. 3801 Regent St. Madison, Wis. 53705), MUSCLE, T-Coffee, CLUSTALX, CLUSTALV, JalView, Phylip, and Discovery Studio from Accelrys (Accelrys, Inc., 10188 Telesis Ct, Suite 100, San Diego, Calif. 92121). In a non-limiting example, MegAlign is used to implement the CLUSTALW alignment algorithm with the following parameters: Gap Penalty 10, Gap Length Penalty 0.20, Delay Divergent Seqs (30%) DNA Transition Weight 0.50, Protein Weight matrix Gonnet Series, DNA Weight Matrix IUB.

The genomic positions of any CpG sites described herein refer to the position of the C in the CpG site in the original genome.

Unless defined otherwise, all technical and scientific terms used herein have the same meaning as commonly understood by one of ordinary skill in the art to which the invention pertains.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein, the terms "approximately" or "about" in reference to a number are generally taken to include numbers that fall within a range of 5% in either direction (greater than or less than) the number unless otherwise stated or otherwise evident from the context (except where such number would exceed 100% of a possible value). Where ranges are stated, the endpoints are included within the range unless otherwise stated or otherwise evident from the context.

### EXAMPLES

### Background

Hypersomnolence, broadly defined as excessive daytime sleepiness often accompanied by prolonged sleep duration, is one of the most common symptom constellations encountered in persons with sleep disorders. While the pathophysiology of hypersomnolence can be identified in some cases, for example orexin deficiency in type 1 narcolepsy,¹ the cause of hypersomnolence cannot universally be determined for many patients despite comprehensive evaluation, resulting in ambiguous diagnoses and a lack of targeted therapies to improve symptomatology. Thus, there is a clear need to better understand the biological basis of unexplained hypersomnolence to advance clinical care through the development of advanced diagnostics and personalized therapeutics for these patients.

One of the great challenges that must be overcome in this area of research is the fact that hypersomnolence is a multifaceted symptom, for which no singular objective measure is able to fully capture the subjective complaint.²⁻³ It is widely appreciated that the multiple sleep latency test (MSLT), while highly useful in confirming type 1 narcolepsy, has limited test-retest reliability in disorders of unexplained hypersomnolence.⁴⁻⁶ While the MSLT remains a key tool in the practice of sleep medicine, it quantifies sleep propensity during repeated nap opportunities and the presence of sleep onset REM periods, but does not capture other relevant aspects of hypersomnolence. Supplementary measures frequently capture other key facets of hypersomnolence, including the maintenance of wakefulness test, which quantifies the ability to remain awake under soporific conditions, infrared pupillometry, which quantifies drowsiness under resting conditions, the psychomotor vigilance task, which quantifies neurobehavioral alertness, and extended duration polysomnographic recordings, which measure excessive sleep duration. While these aspects of hypersomnolence correlate with subjective clinical complaints, they only marginally explain the variance of one another.⁷⁻⁹ Thus, the measurable phenotype in patients with unexplained hypersomnolence can be quite complex, with variable combinations of abnormalities occurring among individual patients.⁷⁻⁹

Since there is heterogeneity among objective measurable deficits in persons with hypersomnolence, it is highly likely that there is also significant heterogeneity in the underlying biology associated with clinical complaints. Sleep medicine has traditionally focused on research that compares various disorders of excessive sleepiness defined by the International Classification of Sleep Disorders (ICSD) both against one another and healthy persons. The diagnostic criteria for disorders such as idiopathic hypersomnia have shifted over time, often with limited clinical or biological data to support alterations in nosology. To disentangle the undoubtedly complex causes of unexplained central nervous system hypersomnolence requires that research efforts focus on the biology of specific and measurable phenotypes that may cut across traditional diagnostic boundaries, rather than nosological distinctions defined largely by expert opinion.¹⁰

The importance of using refined and objective phenotypes is particularly salient for the study of the genetics that may be related to hypersomnolence complaints. Effort has been made to utilize genome-wide association studies (GWAS) to identify candidate single nucleotide polymorphisms (SNPs) that are associated with various sleep phenotypes.¹¹ Unfortunately, the SNPs that are statistically related to sleep measures in these large-scale and hypothesis-free investigations often have modest effect sizes on the overall phenotype, suggesting that genetic variation alone is unlikely to account for extremes of daytime sleepiness and/or sleep duration observed in clinical practice.¹²⁻¹⁶ Additionally, the sleep phenotype used in GWAS studies is almost universally based on self-report, which may be both inaccurate and have limited connection to relevant underlying genetic variability. Thus, while GWAS can help identify potential genes linked to broad phenotypic traits, alternative approaches are needed to connect findings from large-scale population-based GWAS investigations to smaller populations of heterogeneous and disordered patients.

In this context, epigenetics, the study of molecular modifications not affecting the genetic code itself, may be highly applicable to the study of unexplained hypersomnolence. Employing epigenetic methodologies is a crucial step in clarifying how candidate genes are related to specific observable and measurable phenotypes in sleep disorders. In particular, combining epigenetics with a detailed and objective phenotyping approach is likely to advance our understanding of how specific gene regulatory elements are related to the pathophysiology of human disease. Therefore, the present examples examined alterations in DNA methylation, a key component of epigenetic regulation, in a well-characterized sample of patients with unexplained hypersomnolence, to determine if epigenetic modification of candidate genes were related to specific aspects of the hypersomnolence phenotype.

### Methods

### Participants

All participants were clinical patients referred by their treating clinician for sleep testing to evaluate complaints of hypersomnolence at Wisconsin Sleep, the sleep clinic and laboratory affiliated with the University of Wisconsin-Madison. For inclusion, participants had to be 1) free of psychotropic medications at the time of and preceding sleep procedures, 2) not have any identifiable cause of hypersomnolence [e.g., sleep apnea defined as apnea hypopnea index (AHI)>5/hr), evidence of sleep deprivation prior to sleep testing (using either sleep logs and/or actigraphy), complaints of cataplexy suggesting type 1 narcolepsy, etc.], and 3) have no sleep onset REM periods on either overnight polysomnography (PSG) or MSLT. Participants provided informed consent for all study procedures, including both sleep phenotyping measures not part of routine clinical care, as well as collection of biospecimens for subsequent genetic analysis.

### Hypersomnolence Phenotyping Procedures

The hypersomnolence phenotype was determined using four parameters: 1) mean sleep latency on MSLT, 2) total sleep time on ad libitum polysomnography, 3) pupillary unrest index measured by infrared pupillometry, and 4) lapses on the psychomotor vigilance task. All measures were collected within a single sleep laboratory visit.

Participants arrived at the sleep laboratory for testing at approximately 19:00-20:00. After polysomnographic set-up, participants determined their bedtime and were only disturbed after sleep onset if technical issues arose with the recording that would inhibit sleep scoring/staging. The end of the PSG recording was determined by the patient informing the technician that they were ready to get up for the day, rather than a universally applied standard wake time. An MSLT was subsequently performed, with sleep latency defined as the time from lights out to the first 30-second epoch scored as any stage of sleep. The nap was terminated after 20 minutes (if no sleep was achieved) or 15 minutes after the first epoch of scored sleep.¹⁷ Both PSG and MSLT were collected using Alice Sleepware (Phillips Respironics, Murrysville, Pennsylvania, United States) and scored following standard criteria.¹⁸

Following MSLT naps 1 and 3, the pupillographic sleepiness test (PST) and the psychomotor vigilance task were collected with values at each timepoint averaged. The PST is an assessment of drowsiness under constant darkness that records oscillations of the pupil diameter via a computer-based infrared video technique.²⁵ These undulations result from progressive reduction of noradrenergic central activation from the locus coeruleus, resulting in disinhibition of the parasympathetic Edinger-Westphal nucleus.¹⁹ The fluctuations in pupil diameter are utilized to calculate the pupillary unrest index (PUI), defined by absolute values of cumulative changes in pupil size based on the mean values of consecutive data sequences,²⁰ with higher values suggestive of increased drowsiness. Reproducibility, reliability, and normative values of the PUI have been established for adults.^{19,21-23} The PSTEco system (AMTech, Germany) was used for ascertainment of PUI in the present examples and was applied following established protocols.¹⁹

The 10-minute psychomotor vigilance task (PVT) was collected after PST to minimize the potential impact of time-on-task effects from this measure of neurobehavioral alertness on PUI. The PVT is a well-validated measure used in sleep research that quantifies the ability to sustain attention and respond in a timely manner to salient signals.²⁴ The PVT requires responses to a stimulus (digital counter) by pressing a button as soon as the stimulus appears, which stops the stimulus counter and displays the reaction time in milliseconds for a 1-second period. The number of lapses (failure to respond within 500 msec of stimulus; Tukey transformed) was considered the primary PVT measure of interest in the present examples.

### DNA Extraction and Methylation Detection

Saliva samples were collected from participants approximately two hours after awakening using Oragene kits for DNA methylation analysis (DNA Genotek, Canada). DNA was extracted following the manufacturers protocol. Genomic DNA samples were resolved on a 1% agarose gel, to verify the DNA was of high molecular weight, and quantified using Qubit (Qiagen, USA). Genome-wide DNA methylation levels were determined using the HumanMethylationEPIC array and raw intensity data files were imported into the R package *minfi* to assess sample quality, calculate the detection P-value of each tested probe, filter probes, and determine beta values.²⁵ Probes were background- and control-corrected, followed by subset-quantile within array normalization to correct for probe-type bias. ²⁵⁻²⁷ Probes were removed from further analysis if: one sample or more exhibited a detection P-value > 0.01; the probe contained a known single nucleotide polymorphism (SNP); the probe was derived from a sex chromosome; the probe measured methylation at a cytosine followed by a nucleotide other than guanine; or the probe was a cross-reactive probe. With these filtration criteria, 97,964 probes were discarded and 768,127 probes were available for further analysis.

### Statistical analyses

Methylation levels (*i.e.,* beta-values) were calculated in *minfi* as the ratio of methylated to total signal (*i.e.*, beta-value = methylated signal/[methylated signal + unmethylated signal + 100]), where beta-values range from 0 (unmethylated) to 1 (methylated). Beta values were further converted to M-values (*i.e.,* logit-transformed beta-values) for differential analysis, as M-values are more appropriate for statistical testing. Individual models for each independent objective variable of interest [*i.e.,* average PUI on infrared pupillometry, total sleep time (TST) on PSG, mean sleep latency (MSL) on MSLT, and average PVT lapses] were generated, while also accounting for the dependent variables of age, sex, body mass index (BMI), and HumanMethylationEPIC Beadchip identification number. Score on the Epworth Sleepiness Scale (ESS) was similarly evaluated as a subjective measure of daytime sleepiness for comparison purposes.²⁸ Since saliva tissues may be confounded due to latent factors such as heterogeneous cell populations, the R package *sva* was employed to identify any surrogate variables not accounted for in the model.²⁹ In each model, one surrogate variable was identified and adjusted for in the model using *sva.* Linear regression for each tested CpG using a multivariate model was employed using the R package *limma.*³⁰

To assess systematic bias of the linear regression model, the genomic inflation factor was calculated for the obtained P-values for each model, yielding a genomic inflation factor of: PUI (*λ* = 0.99), TST (*λ* = 1.05), MSL (*λ* = 1.05), PVT (*λ* = 1.37), and ESS (*λ* = 1.19). In the latter two cases (PVT and ESS) the slightly elevated *λ* score suggests modest inflation of the generated P-values, indicative of potential bias in the model. As such, these two models were reanalyzed without adjusting for surrogate variables and the genomic inflation factor was reassessed. Without surrogate variable adjustment, the genomic inflation factors were: PVT (*λ* = 0.956) and ESS (*λ* = 0.76), indicating a more unbiased approach without adjusting for surrogate variables in the cases of the independent variables PVT and ESS. In addition, the R package NHMMfdr used here reports a BIC score based on the P-values generated from model fitting for both the adjusted and unadjusted models. Model fitting in NHMMfdr resulted in a lower BIC score using the unadjusted models for PVT and ESS. Together, these data supported that differential methylation modeling should not adjust for the identified surrogate variable for the PVT and ESS variables. Notably, the deflated lambdas for these two cases would result in type II error (i.e., false negative), and may be caused by population stratification in our sample, potentially stemming from the higher proportion of female to male participants.

The methylation levels of immediately flanking probes tested by array-based platforms often exhibit dependence upon one another, and because corrections for multiple testing such as the Benjamini-Hochberg false discovery rate may be inefficient under a varying dependence structure, the R package *NHMMfdr* was used to detect the adjusted local index of significance (aLIS), an extension of adjusted P-values, for each probe by first converting all P-values to z-scores, followed by employing a Hidden Markov Model to determine the "aLIS *P*-value."³¹ An aLIS *P*-value threshold of <0.05 was used to identify significant differentially methylated loci.

### Results

### Participant Demographics and Phenotype

A summary of the 28 patients included in these analyses is provided in Table 3. The sample was predominantly (75%) female. While the mean age was 31.7±11.9 years, the overall range was relatively broad (20 to 66 years). The mean Epworth Sleepiness Scale score was 12.8±3.6 for the sample. Overall, there also was a wide range of values for each hypersomnolence phenotypic trait of interest (e.g., TST, MSL, PUI, and PVT) across the sample, with minimal correlation between values for any objective measure (FIGS. 1A-1C).

**Table 3: Demographics and Sleep Phenotypic Data for the Sample (N = 28).**

| **Characteristic** | **Value** |
|---|---|
| Female Sex, n (%) | 21 (75) |
| Age in years, mean (SD) | 31.7 (11.9) |
| BMI in kg/m², mean (SD) | 26.2 (5.3) |
| MSLT MSL in minutes (SD) | 12.8 (4.2) |
| PSG TST in minutes (SD) | 521.7 (112.4) |
| PUI (SD) | 5.9 (2.5) |
| Lapses, mean (SD) | 2.5 (1.4) |

### PAX8/PAX8-AS1 DNA methylation levels are associated with sleep duration

We first took a hypothesis-driven approach by examining the DNA methylation levels of eleven genes previously associated with the hypersomnolence phenotype (Table 4). Genomic DNAs from saliva were examined using the HumanMethylationEPIC beadchip array, which provides a quantitative measure of DNA methylation levels at 866,091 CpG/CpH dinucleotides across the genome at single-nucleotide resolution, including enhancers and all coding regions. Initial differential methylation analyses centered only on the 832 CpG sites annotated to these eleven genes and utilized independent regression models of the 5 hypersomnolence scores as the explanatory variable (Methods). These analyses revealed that only 9 CpG sites exhibited significant associations between DNA methylation levels and any of the 5 hypersomnolence measures (Table 1). All 9 differentially methylated CpG sites were annotated to the paired box 8 (*PAX8*) gene and its related antisense gene (*PAX8-AS1*) and these methylation levels were correlated to total sleep time measured using ad libitum PSG (aLIS *P*-value < 0.05). Among these 9 differentially methylated positions (DMPs) was a notable cluster of 5 CpG sites within a small (~400 base pair) region located in the body of the *PAX8* gene and promoter of *PAX8-AS1* (FIG. 2). All five DMPs within this cluster had a positive correlation (mean 0.31 ± 0.09) with sleep duration *(i.e.,* increasing methylation was associated with longer sleep time). To quantify the magnitude of the effect of methylation of the 5 DMPs, the sample was also stratified into those with TST < and ≥9 hours.^{32,33} Participants with hypersomnia had mean methylation of these 5 DMPs that was 8.6% greater than those without excessive sleep duration (77.4% vs. 68.6%; P-value = 0.026; FIG. 3).

**Table 4: A priori list of genes contributing to hypersomnolence.**

| **Gene** | **Name** | **aLIS *P*-value** | **Ref** |
|---|---|---|---|
| *PDE4D* | Phosphodiesterase 4D | *N.S.* | *57,58* |
| *NPR2* | Natriuretic peptide receptor 2 | *N.S.* | *59* |
| *SP140* | Speckled protein 140 | *N.S.* | *59* |
| ***PAX8*** | **Thyroid-specific transcription factor paired box gene 8** | **0.021** | *12,13* |
| *IL1RN* | Interleukin-1 receptor antagonist | *N.S.* | *12,60* |
| *ADORA2A* | Adenosine A(2) receptor | *N.S.* | *61,62* |
| *AR* | Androgen receptor | *N.S.* | *13* |
| *OPHN1* | Oligophrenin-1 | *N.S.* | *13* |
| *PER3* | Period 3 | *N.S.* | *62,63* |
| *CACNA1C* | Calcium voltage-gated channel subunit Alpha1 C | *N.S.* | *64-66* |
| *ZFYVE28* | Zinc Finger FYVE-Type Containing 28 | *N.S.* | *67* |

### Genome-wide DNA methylation levels are associated with other aspects of the hypersomnolence phenotype

We additionally conducted genome-wide exploratory comparisons of the 5 independent continuous variables of hypersomnolence scores as the explanatory variable (Methods), which each yielded a unique set of DMPs. Notably, DMPs were found in two genes. First, *LHX6* contained 11 DMPs associated with mean sleep latency on the MSLT (Table 2). Among these 11 DMPs was a notable cluster of 6 CpG sites within a small (~500 base pair) region that were located in the body of the *LHX6* gene. All six DMPs within this cluster had a negative correlation with MSLT MSL (*i.e.,* increasing methylation was associated with reduced sleep latency). Second, *GABRA2* contained 7 DMPs associated with PUI using infrared pupillometry (Table 2). These 7 DMPs were within a small (~300 base pair) region located in the promoter of the *GABRA2* gene. The majority (6/7) of these DMPs had a negative correlation with sleep duration (*i.e.,* decreasing methylation was associated with increasing PUI). Significant findings were not found using either ESS or PVT as the independent variable in either the hypothesis-driven or genome-wide analyses.

### Discussion

The present examples demonstrate that *PAX8*/*PAX8-AS1* DNA methylation levels are associated with sleep duration quantified using in-laboratory ad libitum polysomnography in a well-characterized, unmedicated group of clinical patients with unexplained hypersomnolence. Specifically, 9 differentially methylated sites within *PAX8*/*PAX8-AS1* were associated with sleep duration, of which 5 formed a distinct cluster of CpG sites within a small (~400 base pair) region in the body of the *PAX8* gene and promoter of *PAX8-AS1.* Since antisense genes frequently play key roles in reducing expression of overlapping sense genes and form self-influencing circuits with regulatory advantages over transcription factor proteins³⁴, the location and clustering of these significantly differentially methylated CpG sites increase the likelihood they may directly impact both *PAX8* and *PAX8-AS1* expression. In fact, more than 70% of the transcripts in humans have antisense transcripts that are generated by independent promoters.³⁵ Antisense transcript can function by the act of its own transcription in *cis* (controlling genes locally on the DNA strand involved in its origination), which regulates sense gene expression by interfering/blocking sense transcriptional machinery.³⁶

While the specific determinants of habitual sleep time remain unknown, recent genome wide association studies (GWAS) have highlighted the role of *PAX8*/*PAX8-AS1* in human sleep duration. *PAX8* likely serves several roles as a master transcription factor crucial to embryonic development and maintenance functions after birth.³⁸ Using both self-report and accelerometer data in population-based cohorts, SNPs in *PAX8*/*PAX8-AS1* have repeatedly demonstrated significant effects on sleep duration.¹²⁻¹⁶ However, the SNPs themselves impact sleep duration only marginally (*i.e.,* only a few minutes longer sleep per day). Thus, while hypothesis-free GWAS investigations have pointed to a role of *PAX8*/*PAX8-AS1* in habitual sleep duration, specific sequence variability in *PAX8*/*PAX8-AS1* is unlikely to be a major contributing factor in pathological hypersomnia. In the present examples, we have further extended these well-replicated associations between *PAX8*/*PAX8-AS1* and sleep duration by finding a significant association between *PAX8*/*PAX8-AS1* DNA methylation levels and nocturnal sleep duration in symptomatic patients. The magnitude of the effect between sleep duration and *PAX8*/*PAX8-AS1* methylation levels (*e.g.,* ~10%) observed in the present examples is on par with methylation level changes that are the epigenetic hallmark of complex disease phenotypes.³⁹ Unique to our approach was the application of deep phenotyping¹⁰, which carefully and objectively quantified several different facets of hypersomnolence, including ad libitum polysomnography to measure total sleep time.

There are several lines of evidence that further support the role of *PAX8*/*PAX8-AS1,* and particularly its epigenetic modification, on sleep duration and hypersomnia. First, one of the few compounds that has demonstrated a measurable impact on excessive sleep duration in idiopathic hypersomnia is levothyroxine, evaluated in a small open-label study in euthyroid patients.⁴⁰ While *PAX8* is expressed in many tissues, it is predominantly expressed in fetal and adult thyroid tissue, and mutations in *PAX8* have been associated with thyroid dysgenesis and thyroid cancers. Second, fluctuations in cerebral *PAX8* methylation levels mirror patterns of total sleep quantity across the lifespan, with a high rate of change early in the first few years of life, followed by a general plateau beginning in the second decade.⁴¹ Thus, it is probable given the large number of GWAS studies implicating *PAX8*/*PAX8-AS1* in sleep duration, relevant supportive data in the literature, and our findings linking *PAX8*/*PAX8-AS1* methylation to sleep duration, that DNA methylation changes in *PAX8*/*PAX8-AS1* may be a biomarker for, or mechanistically related to hypersomnia.

In the secondary genome-wide analysis, DMPs also were found in two genes that have been associated with hypersomnolence in other investigations, *LHX6* and *GABRA2.* LHX6-positive GABA-releasing neurons in the zona incerta promote NREM sleep, likely by inhibiting hypocretin positive neurons, consistent with a plausible connection between these findings and sleep propensity quantified by the MSLT.⁴⁷ A SNP in *GABRA2* was recently associated with self-reported daytime sleepiness in a large-scale GWAS study, published after the study methods were executed.⁴⁸ Together, these data suggest that genes other than *PAX8* may exhibit disruptions in DNA methylation levels correlated to other aspects of hypersomnolence. Thus, future studies that both replicate the primary finding of an association between PAX8/PAX8-AS1 methylation and sleep duration, as well as associations between LHX6 and MSLT sleep latency and GABRA2 and infrared pupillometry, are indicated.

In summary, the present examples demonstrate a significant association between *PAX8*/*PAX8-AS1* DNA methylation levels and objectively quantified sleep duration in persons with unexplained hypersomnolence. These results represent a key step forward in understanding the underlying biology that may be related to both sleep duration and pathological hypersomnia, bridging key findings in prior GWAS studies to disordered populations in sleep medicine. This work will serve as a nidus for further investigation that clarifies the role of *PAX8*/*PAX8-AS1* regulation and expression in both sleep need and duration for healthy persons and patients with pathological hypersomnia.

### References

1. Dauvilliers Y, Arnulf I, Mignot E. Narcolepsy with cataplexy. Lancet. 2007; 369 (9560): 499-511.
2. Bliwise DL. Is the measurement of sleepiness the Holy Grail of sleep medicine? Am J Respir Crit Care Med. 2001; 163 (7): 1517-1519.
3. Pizza F. Sleepiness Assessment. In: Sleepiness and Human Impact Assessment. Milano, Italy.: Springer; 2014: 313-324.
4. Lopez R, Doukkali A, Barateau L, et al. Test-Retest Reliability of the Multiple Sleep Latency Test in Central Disorders of Hypersomnolence. Sleep. 2017; 40 (12).
5. Ruoff C, Pizza F, Trotti LM, et al. The MSLT is Repeatable in Narcolepsy Type 1 But Not Narcolepsy Type 2: A Retrospective Patient Study. J Clin Sleep Med. 2017.
6. Trotti LM, Staab BA, Rye DB. Test-retest reliability of the multiple sleep latency test in narcolepsy without cataplexy and idiopathic hypersomnia. J Clin Sleep Med. 2013; 9 (8): 789-795.
7. Sangal RB, Thomas L, Mitler MM. Maintenance of wakefulness test and multiple sleep latency test. Measurement of different abilities in patients with sleep disorders. Chest. 1992; 101 (4): 898-902.
8. Johns MW. Sensitivity and specificity of the multiple sleep latency test (MSLT), the maintenance of wakefulness test and the epworth sleepiness scale: failure of the MSLT as a gold standard. J Sleep Res. 2000; 9 (1): 5-11.
9. Plante DT, Cook JD, Prairie ML. Multimodal Assessment Increases Objective Identification of Hypersomnolence in Patients Referred for Multiple Sleep Latency Testing. J Clin Sleep Med. 2020.
10. Robinson PN. Deep phenotyping for precision medicine. Hum Mutat. 2012; 33 (5): 777-780.
11. Raizen DM, Wu MN. Genome-wide association studies of sleep disorders. Chest. 2011; 139 (2): 446-452.
12. Gottlieb DJ, Hek K, Chen TH, et al. Novel loci associated with usual sleep duration: the CHARGE Consortium Genome-Wide Association Study. Mol Psychiatry. 2015; 20 (10): 1232-1239.
13. Lane JM, Liang J, Vlasac I, et al. Genome-wide association analyses of sleep disturbance traits identify new loci and highlight shared genetics with neuropsychiatric and metabolic traits. Nat Genet. 2017; 49 (2): 274-281.
14. Dashti HS, Jones SE, Wood AR, et al. Genome-wide association study identifies genetic loci for self-reported habitual sleep duration supported by accelerometer-derived estimates. Nat Commun. 2019; 10 (1): 1100.
15. Jones SE, Tyrrell J, Wood AR, et al. Genome-Wide Association Analyses in 128,266 Individuals Identifies New Morningness and Sleep Duration Loci. PLoS Genet. 2016; 12 (8): e1006125.
16. Jones SE, van Hees VT, Mazzotti DR, et al. Genetic studies of accelerometer-based sleep measures yield new insights into human sleep behaviour. Nat Commun. 2019; 10 (1): 1585.
17. Littner MR, Kushida C, Wise M, et al. Practice parameters for clinical use of the multiple sleep latency test and the maintenance of wakefulness test. Sleep. 2005; 28 (1): 113-121.
18. Berry R, Brooks R, Gamaldo C, et al. The AASM Manual for the Scoring of Sleep and Associated Events: Rules, Terminology and Technical Specifications, Version 2.0. Darien, Illinois: American Academy of Sleep Medicine; 2012.
19. Eggert T, Sauter C, Popp R, Zeitlhofer J, Danker-Hopfe H, et al. The Pupillographic SleepinessTest in adults: effect of age, gender, and time of day on pupillometric variables. Am J Hum Biol. 2012; 24 (6): 820-828.
20. Lüdtke H, Wilhelm B, Adler M, Schaeffel F, Wilhelm H. Mathematical procedures in data recording and processing of pupillary fatigue waves. Vision Res. 1998; 38 (19): 2889-2896.
21. Wilhelm B, Korner A, Heldmaier K, Moll K, Wilhelm H, Ludtke H. Normative Values of the Pupillographic Sleepiness Test in Male and Female Subjects Aged 20 to 60 Years. Somnologie. 2001; 5: 115-120.
22. Ludtke H, Korner A, Wilhelm B, Wilhelm H. Reproducibility of the pupillographic sleepiness test in healthy men. Somnologie. 2000; 4: 170-172.
23. Wilhelm B, Bittner E, Hofmann A, et al. Short-term reproducibility and variability of the pupillographic sleepiness test. Am J Hum Biol. 2015; 27 (6): 862-866.
24. Dinges DF, Powell JW. Microcomputer analysis of performance on a portable, simple visual RT task sustained operations. Behavioral Research Methods, Instrumentation, and Computers. 1985; 17: 652-655.
25. Aryee MJ, Jaffe AE, Corrada-Bravo H, et al. Minfi: a flexible and comprehensive Bioconductor package for the analysis of Infinium DNA methylation microarrays. Bioinformatics (Oxford, England). 2014; 30 (10): 1363-1369.
26. Maksimovic J, Gordon L, Oshlack A. SWAN: Subset-quantile within array normalization for illumina infinium HumanMethylation450 BeadChips. Genome biology. 2012; 13 (6): R44.
27. Wockner LF, Noble EP, Lawford BR, et al. Genome-wide DNA methylation analysis of human brain tissue from schizophrenia patients. Transl Psychiatry. 2014; 4: e339.
28. Johns MW. A new method for measuring daytime sleepiness: the Epworth sleepiness scale. Sleep. 1991; 14 (6): 540-545.
29. Leek JT, Johnson WE, Parker HS, Jaffe AE, Storey JD. The sva package for removing batch effects and other unwanted variation in high-throughput experiments. Bioinformatics (Oxford, England). 2012; 28 (6): 882-883.
30. Ritchie ME, Phipson B, Wu D, et al. limma powers differential expression analyses for RNA-sequencing and microarray studies. Nucleic acids research. 2015; 43 (7): e47.
31. Kuan PF, Chiang DY. Integrating prior knowledge in multiple testing under dependence with applications to detecting differential DNA methylation. Biometrics. 2012; 68 (3): 774-783.
32. Ohayon MM, Reynolds CF, Dauvilliers Y. Excessive sleep duration and quality of life. Ann Neurol. 2013; 73 (6): 785-794.
33. Lammers GJ, Bassetti CLA, Dolenc-Groselj L, et al. Diagnosis of central disorders of hypersomnolence: A reappraisal by European experts. Sleep Med Rev. 2020; 52: 101306.
34. Pelechano V, Steinmetz LM. Gene regulation by antisense transcription. Nat Rev Genet. 2013; 14 (12): 880-893.
35. Katayama S, Tomaru Y, Kasukawa T, et al. Antisense transcription in the mammalian transcriptome. Science. 2005; 309 (5740): 1564-1566.
36. Hongay CF, Grisafi PL, Galitski T, Fink GR. Antisense transcription controls cell fate in Saccharomyces cerevisiae. Cell. 2006; 127 (4): 735-745.
37. Wang Z, Wilson CM, Mendelev N, et al. Acute and Chronic Molecular Signatures and Associated Symptoms of Blast Exposure in Military Breachers. J Neurotrauma. 2020; 37 (10): 1221-1232.
38. Fernández LP, López-Márquez A, Santisteban P. Thyroid transcription factors in development, differentiation and disease. Nat Rev Endocrinol. 2015; 11 (1): 29-42.
39. Leenen FA, Muller CP, Turner JD. DNA methylation: conducting the orchestra from exposure to phenotype? Clin Epigenetics. 2016; 8: 92.
40. Shinno H, Ishikawa I, Yamanaka M, et al. Effect of levothyroxine on prolonged nocturnal sleep time and excessive daytime somnolence in patients with idiopathic hypersomnia. Sleep Med. 2011; 12 (6): 578-583.
41. Siegmund KD, Connor CM, Campan M, et al. DNA methylation in the human cerebral cortex is dynamically regulated throughout the life span and involves differentiated neurons. PLoS One. 2007; 2 (9): e895.
42. Trivedi MS, Holger D, Bui AT, Craddock TJA, Tartar JL. Short-term sleep deprivation leads to decreased systemic redox metabolites and altered epigenetic status. PLoS One. 2017; 12 (7): e0181978.
43. Nilsson EK, Boström AE, Mwinyi J, Schiöth HB. Epigenomics of Total Acute Sleep Deprivation in Relation to Genome-Wide DNA Methylation Profiles and RNA Expression. OMICS. 2016; 20 (6): 334-342.
44. Vernet C, Arnulf I. Idiopathic hypersomnia with and without long sleep time: a controlled series of 75 patients. Sleep. 2009; 32 (6): 753-759.
45. Evangelista E, Lopez R, Barateau L, et al. Alternative diagnostic criteria for idiopathic hypersomnia: A 32-hour protocol. Ann Neurol. 2018; 83 (2): 235-247.
46. Pizza F, Moghadam KK, Vandi S, et al. Daytime continuous polysomnography predicts MSLT results in hypersomnias of central origin. J Sleep Res. 2013; 22 (1): 32-40.
47. Liu K, Kim J, Kim DW, et al. Lhx6-positive GABA-releasing neurons of the zona incerta promote sleep. Nature. 2017; 548 (7669): 582-587.
48. Wang H, Lane JM, Jones SE, et al. Genome-wide association analysis of self-reported daytime sleepiness identifies 42 loci that suggest biological subtypes. Nat Commun. 2019; 10 (1): 3503.
49. Cortese R, Zhang C, Bao R, et al. DNA Methylation Profiling of Blood Monocytes in Patients With Obesity Hypoventilation Syndrome: Effect of Positive Airway Pressure Treatment. Chest. 2016; 150 (1): 91-101.
50. Ämmälä AJ, Urrila AS, Lahtinen A, et al. Epigenetic dysregulation of genes related to synaptic long-term depression among adolescents with depressive disorder and sleep symptoms. Sleep Med. 2019; 61: 95-103.
51. Alisch RS, Chopra P, Fox AS, et al. Differentially methylated plasticity genes in the amygdala of young primates are linked to anxious temperament, an at risk phenotype for anxiety and depressive disorders. J Neurosci. 2014; 34 (47): 15548-15556.
52. Papale LA, Seltzer LJ, Madrid A, Pollak SD, Alisch RS. Differentially Methylated Genes in Saliva are linked to Childhood Stress. Sci Rep. 2018; 8 (1): 10785.
53. Alisch RS, Van Hulle C, Chopra P, et al. A multi-dimensional characterization of anxiety in monozygotic twin pairs reveals susceptibility loci in humans. Transl Psychiatry. 2017; 7 (12): 1282.
54. Kim H, Dinges DF, Young T. Sleep-disordered breathing and psychomotor vigilance in a community-based sample. Sleep. 2007; 30 (10): 1309-1316.
55. Jansen EC, Dolinoy DC, O'Brien LM, et al. Sleep duration and fragmentation in relation to leukocyte DNA methylation in adolescents. Sleep. 2019; 42 (9).
56. Leu-Semenescu S, Louis P, Arnulf I. Benefits and risk of sodium oxybate in idiopathic hypersomnia versus narcolepsy type 1: a chart review. Sleep Med. 2016; 17: 38-44.
57. Gottlieb DJ, O'Connor GT, Wilk JB. Genome-wide association of sleep and circadian phenotypes. BMC Med Genet. 2007; 8 Suppl 1: S9.
58. Pak VM, Mazzotti DR, Keenan BT, et al. Candidate Gene Analysis in the São Paulo Epidemiologic Sleep Study (EPISONO) Shows an Association of Variant in PDE4D and Sleepiness. Sleep Med. 2018l 47: 106-12.
59. Chen YC, Chen TW, Su MC, et al. Whole Genome DNA Methylation Analysis of Obstructive Sleep Apnea: IL1R2, NPR2, AR, SP140 Methylation and Clinical Phenotype. Sleep. 2016; 39 (4): 743-755.
60. Fang J, Wang Y, Krueger JM. Effects of interleukin-1 beta on sleep are mediated by the type I receptor. Am J Physiol. 1998; 274 (3 Pt 2): R655-660.
61. Bodenmann S, Hohoff C, Freitag C, et al. Polymorphisms of ADORA2A modulate psychomotor vigilance and the effects of caffeine on neurobehavioural performance and sleep EEG after sleep deprivation. Br J Pharmacol. 2012; 165 (6): 1904-1913.
62. Rupp TL, Wesensten NJ, Newman R, Balkin TJ. PER3 and ADORA2A polymorphisms impact neurobehavioral performance during sleep restriction. J Sleep Res. 2013; 22 (2): 160-165.
63. Goel N, Banks S, Mignot E, Dinges DF. PER3 polymorphism predicts cumulative sleep homeostatic but not neurobehavioral changes to chronic partial sleep deprivation. PLoS One. 2009; 4 (6): e5874.
64. Shimada M, Miyagawa T, Kawashima M, et al. An approach based on a genome-wide association study reveals candidate loci for narcolepsy. Hum Genet. 2010; 128 (4): 433-441.
65. Byrne EM, Gehrman PR, Medland SE, et al. A genome-wide association study of sleep habits and insomnia. Am J Med Genet B Neuropsychiatr Genet. 2013; 162B (5): 439-451.
66. Parsons MJ, Lester KJ, Barclay NL, Nolan PM, Eley TC, Gregory AM. Replication of Genome-Wide Association Studies (GWAS) loci for sleep in the British G1219 cohort. Am J Med Genet B Neuropsychiatr Genet. 2013; 162B (5): 431-438.
67. Huang H, Zhu Y, Eliot MN, et al. Combining Human Epigenetics and Sleep Studies in Caenorhabditis elegans: A Cross-Species Approach for Finding Conserved Genes Regulating Sleep. Sleep. 2017; 40 (6).

## Claims

1. A method comprising:
isolating target DNA from a biofluid sample from a subject to obtain isolated target DNA;
treating the isolated target DNA with bisulfite to generate bisulfite modified DNA;
amplifying the bisulfite modified DNA with primers specific to one or more human genomic positions to generate amplified DNA, wherein the one or more human genomic positions comprise six or more of:
a genomic position having a C in a CpG site aligning to 113992930 of chromosome 2 of UCSC hg19 human reference genome,
a genomic position having a C in a CpG site aligning to 113993052 of chromosome 2 of UCSC hg19 human reference genome,
a genomic position having a C in a CpG site aligning to 113993142 of chromosome 2 of UCSC hg19 human reference genome,
a genomic position having a C in a CpG site aligning to 113993304 of chromosome 2 of UCSC hg19 human reference genome,
a genomic position having a C in a CpG site aligning to 113993313 of chromosome 2 of UCSC hg19 human reference genome,
a genomic position having a C in a CpG site aligning to 113994035 of chromosome 2 of UCSC hg19 human reference genome,
a genomic position having a C in a CpG site aligning to 113994578 of chromosome 2 of UCSC hg19 human reference genome,
a genomic position having a C in a CpG site aligning to 113995370 of chromosome 2 of UCSC hg19 human reference genome,
a genomic position having a C in a CpG site aligning to 113995456 of chromosome 2 of UCSC hg19 human reference genome,
a genomic position having a C in a CpG site aligning to 124985406 of chromosome 9 of UCSC hg19 human reference genome,
a genomic position having a C in a CpG site aligning to 124985756 of chromosome 9 of UCSC hg19 human reference genome,
a genomic position having a C in a CpG site aligning to 124987896 of chromosome 9 of UCSC hg19 human reference genome,
a genomic position having a C in a CpG site aligning to 124988720 of chromosome 9 of UCSC hg19 human reference genome,
a genomic position having a C in a CpG site aligning to 124989052 of chromosome 9 of UCSC hg19 human reference genome,
a genomic position having a C in a CpG site aligning to 124989241 of chromosome 9 of UCSC hg19 human reference genome,
a genomic position having a C in a CpG site aligning to 124989294 of chromosome 9 of UCSC hg19 human reference genome,
a genomic position having a C in a CpG site aligning to 124989337 of chromosome 9 of UCSC hg19 human reference genome,
a genomic position having a C in a CpG site aligning to 124989408 of chromosome 9 of UCSC hg19 human reference genome,
a genomic position having a C in a CpG site aligning to 124989550 of chromosome 9 of UCSC hg19 human reference genome,
a genomic position having a C in a CpG site aligning to 124989839 of chromosome 9 of UCSC hg19 human reference genome,
a genomic position having a C in a CpG site aligning to 46391392 of chromosome 4 of UCSC hg19 human reference genome,
a genomic position having a C in a CpG site aligning to 46391436 of chromosome 4 of UCSC hg19 human reference genome,
a genomic position having a C in a CpG site aligning to 46391448 of chromosome 4 of UCSC hg19 human reference genome,
a genomic position having a C in a CpG site aligning to 46391476 of chromosome 4 of UCSC hg19 human reference genome,
a genomic position having a C in a CpG site aligning to 46391524 of chromosome 4 of UCSC hg19 human reference genome,
a genomic position having a C in a CpG site aligning to 46391532 of chromosome 4 of UCSC hg19 human reference genome, and
a genomic position having a C in a CpG site aligning to 46391694 of chromosome 4 of UCSC hg19 human reference genome; and
quantifying methylation at the one or more human genomic positions in the amplified DNA.

2. A method as claimed in claim 1, wherein the one or more human genomic positions comprise seven or more of:
a genomic position having a C in a CpG site aligning to 113992930 of chromosome 2 of UCSC hg19 human reference genome,
a genomic position having a C in a CpG site aligning to 113993052 of chromosome 2 of UCSC hg19 human reference genome,
a genomic position having a C in a CpG site aligning to 113993142 of chromosome 2 of UCSC hg19 human reference genome,
a genomic position having a C in a CpG site aligning to 113993304 of chromosome 2 of UCSC hg19 human reference genome,
a genomic position having a C in a CpG site aligning to 113993313 of chromosome 2 of UCSC hg19 human reference genome,
a genomic position having a C in a CpG site aligning to 113994035 of chromosome 2 of UCSC hg19 human reference genome,
a genomic position having a C in a CpG site aligning to 113994578 of chromosome 2 of UCSC hg19 human reference genome,
a genomic position having a C in a CpG site aligning to 113995370 of chromosome 2 of UCSC hg19 human reference genome,
a genomic position having a C in a CpG site aligning to 113995456 of chromosome 2 of UCSC hg19 human reference genome,
a genomic position having a C in a CpG site aligning to 124985406 of chromosome 9 of UCSC hg19 human reference genome,
a genomic position having a C in a CpG site aligning to 124985756 of chromosome 9 of UCSC hg19 human reference genome,
a genomic position having a C in a CpG site aligning to 124987896 of chromosome 9 of UCSC hg19 human reference genome,
a genomic position having a C in a CpG site aligning to 124988720 of chromosome 9 of UCSC hg19 human reference genome,
a genomic position having a C in a CpG site aligning to 124989052 of chromosome 9 of UCSC hg19 human reference genome,
a genomic position having a C in a CpG site aligning to 124989241 of chromosome 9 of UCSC hg19 human reference genome,
a genomic position having a C in a CpG site aligning to 124989294 of chromosome 9 of UCSC hg19 human reference genome,
a genomic position having a C in a CpG site aligning to 124989337 of chromosome 9 of UCSC hg19 human reference genome,
a genomic position having a C in a CpG site aligning to 124989408 of chromosome 9 of UCSC hg19 human reference genome,
a genomic position having a C in a CpG site aligning to 124989550 of chromosome 9 of UCSC hg19 human reference genome,
a genomic position having a C in a CpG site aligning to 124989839 of chromosome 9 of UCSC hg19 human reference genome,
a genomic position having a C in a CpG site aligning to 46391392 of chromosome 4 of UCSC hg19 human reference genome,
a genomic position having a C in a CpG site aligning to 46391436 of chromosome 4 of UCSC hg19 human reference genome,
a genomic position having a C in a CpG site aligning to 46391448 of chromosome 4 of UCSC hg19 human reference genome,
a genomic position having a C in a CpG site aligning to 46391476 of chromosome 4 of UCSC hg19 human reference genome,
a genomic position having a C in a CpG site aligning to 46391524 of chromosome 4 of UCSC hg19 human reference genome,
a genomic position having a C in a CpG site aligning to 46391532 of chromosome 4 of UCSC hg19 human reference genome, and
a genomic position having a C in a CpG site aligning to 46391694 of chromosome 4 of UCSC hg19 human reference genome.

3. A method as claimed in claim 1 or 2, wherein the one or more human genomic positions comprise eight or more of:
a genomic position having a C in a CpG site aligning to 113992930 of chromosome 2 of UCSC hg19 human reference genome,
a genomic position having a C in a CpG site aligning to 113993052 of chromosome 2 of UCSC hg19 human reference genome,
a genomic position having a C in a CpG site aligning to 113993142 of chromosome 2 of UCSC hg19 human reference genome,
a genomic position having a C in a CpG site aligning to 113993304 of chromosome 2 of UCSC hg19 human reference genome,
a genomic position having a C in a CpG site aligning to 113993313 of chromosome 2 of UCSC hg19 human reference genome,
a genomic position having a C in a CpG site aligning to 113994035 of chromosome 2 of UCSC hg19 human reference genome,
a genomic position having a C in a CpG site aligning to 113994578 of chromosome 2 of UCSC hg19 human reference genome,
a genomic position having a C in a CpG site aligning to 113995370 of chromosome 2 of UCSC hg19 human reference genome, and
a genomic position having a C in a CpG site aligning to 113995456 of chromosome 2 of UCSC hg19 human reference genome.

4. A method as claimed in any preceding claim, wherein the one or more human genomic positions comprise each of:
a genomic position having a C in a CpG site aligning to 113992930 of chromosome 2 of UCSC hg19 human reference genome,
a genomic position having a C in a CpG site aligning to 113993052 of chromosome 2 of UCSC hg19 human reference genome,
a genomic position having a C in a CpG site aligning to 113993142 of chromosome 2 of UCSC hg19 human reference genome,
a genomic position having a C in a CpG site aligning to 113993304 of chromosome 2 of UCSC hg19 human reference genome,
a genomic position having a C in a CpG site aligning to 113993313 of chromosome 2 of UCSC hg19 human reference genome,
a genomic position having a C in a CpG site aligning to 113994035 of chromosome 2 of UCSC hg19 human reference genome,
a genomic position having a C in a CpG site aligning to 113994578 of chromosome 2 of UCSC hg19 human reference genome,
a genomic position having a C in a CpG site aligning to 113995370 of chromosome 2 of UCSC hg19 human reference genome, and
a genomic position having a C in a CpG site aligning to 113995456 of chromosome 2 of UCSC hg19 human reference genome.

5. A method as claimed in any preceding claim, wherein the one or more human genomic positions comprise each of:
a genomic position having a C in a CpG site aligning to 124985406 of chromosome 9 of UCSC hg19 human reference genome,
a genomic position having a C in a CpG site aligning to 124985756 of chromosome 9 of UCSC hg19 human reference genome,
a genomic position having a C in a CpG site aligning to 124987896 of chromosome 9 of UCSC hg19 human reference genome,
a genomic position having a C in a CpG site aligning to 124988720 of chromosome 9 of UCSC hg19 human reference genome,
a genomic position having a C in a CpG site aligning to 124989052 of chromosome 9 of UCSC hg19 human reference genome,
a genomic position having a C in a CpG site aligning to 124989241 of chromosome 9 of UCSC hg19 human reference genome,
a genomic position having a C in a CpG site aligning to 124989294 of chromosome 9 of UCSC hg19 human reference genome,
a genomic position having a C in a CpG site aligning to 124989337 of chromosome 9 of UCSC hg19 human reference genome,
a genomic position having a C in a CpG site aligning to 124989408 of chromosome 9 of UCSC hg19 human reference genome,
a genomic position having a C in a CpG site aligning to 124989550 of chromosome 9 of UCSC hg19 human reference genome, and
a genomic position having a C in a CpG site aligning to 124989839 of chromosome 9 of UCSC hg19 human reference genome.

6. A method as claimed in any preceding claim, wherein the one or more human genomic positions comprise each of:
a genomic position having a C in a CpG site aligning to 46391392 of chromosome 4 of UCSC hg19 human reference genome,
a genomic position having a C in a CpG site aligning to 46391436 of chromosome 4 of UCSC hg19 human reference genome,
a genomic position having a C in a CpG site aligning to 46391448 of chromosome 4 of UCSC hg19 human reference genome,
a genomic position having a C in a CpG site aligning to 46391476 of chromosome 4 of UCSC hg19 human reference genome,
a genomic position having a C in a CpG site aligning to 46391524 of chromosome 4 of UCSC hg19 human reference genome,
a genomic position having a C in a CpG site aligning to 46391532 of chromosome 4 of UCSC hg19 human reference genome, and
a genomic position having a C in a CpG site aligning to 46391694 of chromosome 4 of UCSC hg19 human reference genome.

7. A method as claimed in any preceding claim, wherein:
the primers comprise one or more pairs of primers, each pair of primers amplifying a DNA fragment of 150-350 bp in length, optionally, wherein the one or more pairs of primers consists of fewer than 10 pairs of primers.

8. A method as claimed in any preceding claim, wherein the biofluid is selected from the group consisting of saliva, blood, cerebrospinal fluid, sweat, tears, semen, and urine, optionally wherein the biofluid is blood or saliva.

9. A method as claimed in any preceding claim, wherein the subject is a human.

10. A method as claimed in any preceding claim, wherein the biofluid sample is from a healthy subject or the biofluid sample is from a subject previously diagnosed with diabetes, asthma, or hypertension.

11. A method as claimed in any preceding claim, wherein the biofluid sample is from a subject with a mean sleep latency as measured by a multiple sleep latency test of less than 10 minutes.

12. A method as claimed in any preceding claim, wherein the biofluid sample is from a subject with a pupillary unrest index as measured by infrared pupillometry of at least 9.8.

13. A method as claimed in any preceding claim, wherein the biofluid sample is from a subject demonstrating lapses in a psychomotor vigilance test of at least 4.8.

## Patentansprüche

1. Verfahren, aufweisend:
Isolieren von Ziel-DNA aus einer Körperflüssigkeitsprobe eines Individuums, um isolierte Ziel-DNA zu erhalten;
Behandeln der isolierten Ziel-DNA mit Bisulfit, um mit Bisulfit modifizierte DNA zu erzeugen;
Amplifizieren der mit Bisulfit modifizierten DNA mit Primern, die für eine oder mehrere humane genomische Positionen spezifisch sind, um amplifizierte DNA zu erzeugen, wobei die eine oder mehreren humanen genomischen Positionen sechs oder mehr aufweisen von:
eine genomische Position, die ein C an einer CpG-Stelle aufweist die mit 113992930 von Chromosom 2 des humanen UCSC-hg19-Referenzgenoms aligniert,
eine genomische Position, die ein C an einer CpG-Stelle aufweist die mit 113993052 von Chromosom 2 des humanen UCSC-hg19-Referenzgenoms aligniert,
eine genomische Position, die ein C an einer CpG-Stelle aufweist die mit 113993142 von Chromosom 2 des humanen UCSC-hg19-Referenzgenoms aligniert,
eine genomische Position, die ein C an einer CpG-Stelle aufweist die mit 113993304 von Chromosom 2 des humanen UCSC-hg19-Referenzgenoms aligniert,
eine genomische Position, die ein C an einer CpG-Stelle aufweist die mit 113993313 von Chromosom 2 des humanen UCSC-hg19-Referenzgenoms aligniert,
eine genomische Position, die ein C an einer CpG-Stelle aufweist die mit 113994035 von Chromosom 2 des humanen UCSC-hg19-Referenzgenoms aligniert,
eine genomische Position, die ein C an einer CpG-Stelle aufweist die mit 113994578 von Chromosom 2 des humanen UCSC-hg19-Referenzgenoms aligniert,
eine genomische Position, die ein C an einer CpG-Stelle aufweist die mit 113995370 von Chromosom 2 des humanen UCSC-hg19-Referenzgenoms aligniert,
eine genomische Position, die ein C an einer CpG-Stelle aufweist die mit 113995456 von Chromosom 2 des humanen UCSC-hg19-Referenzgenoms aligniert,
eine genomische Position, die ein C an einer CpG-Stelle aufweist die mit 124985406 von Chromosom 9 des humanen UCSC-hg19-Referenzgenoms aligniert,
eine genomische Position, die ein C an einer CpG-Stelle aufweist die mit 124985756 von Chromosom 9 des humanen UCSC-hg19-Referenzgenoms aligniert,
eine genomische Position, die ein C an einer CpG-Stelle aufweist die mit 124987896 von Chromosom 9 des humanen UCSC-hg19-Referenzgenoms aligniert,
eine genomische Position, die ein C an einer CpG-Stelle aufweist die mit 124988720 von Chromosom 9 des humanen UCSC-hg19-Referenzgenoms aligniert,
eine genomische Position, die ein C an einer CpG-Stelle aufweist die mit 124989052 von Chromosom 9 des humanen UCSC-hg19-Referenzgenoms aligniert,
eine genomische Position, die ein C an einer CpG-Stelle aufweist die mit 124989241 von Chromosom 9 des humanen UCSC-hg19-Referenzgenoms aligniert,
eine genomische Position, die ein C an einer CpG-Stelle aufweist die mit 124989294 von Chromosom 9 des humanen UCSC-hg19-Referenzgenoms aligniert,
eine genomische Position, die ein C an einer CpG-Stelle aufweist die mit 124989337 von Chromosom 9 des humanen UCSC-hg19-Referenzgenoms aligniert,
eine genomische Position, die ein C an einer CpG-Stelle aufweist die mit 124989408 von Chromosom 9 des humanen UCSC-hg19-Referenzgenoms aligniert,
eine genomische Position, die ein C an einer CpG-Stelle aufweist die mit 124989550 von Chromosom 9 des humanen UCSC-hg19-Referenzgenoms aligniert,
eine genomische Position, die ein C an einer CpG-Stelle aufweist die mit 124989839 von Chromosom 9 des humanen UCSC-hg19-Referenzgenoms aligniert,
eine genomische Position, die ein C an einer CpG-Stelle aufweist die mit 46391392 von Chromosom 4 des humanen UCSC-hg19-Referenzgenoms aligniert,
eine genomische Position, die ein C an einer CpG-Stelle aufweist die mit 46391436 von Chromosom 4 des humanen UCSC-hg19-Referenzgenoms aligniert,
eine genomische Position, die ein C an einer CpG-Stelle aufweist die mit 46391448 von Chromosom 4 des humanen UCSC-hg19-Referenzgenoms aligniert,
eine genomische Position, die ein C an einer CpG-Stelle aufweist die mit 46391476 von Chromosom 4 des humanen UCSC-hg19-Referenzgenoms aligniert,
eine genomische Position, die ein C an einer CpG-Stelle aufweist die mit 46391524 von Chromosom 4 des humanen UCSC-hg19-Referenzgenoms aligniert,
eine genomische Position, die ein C an einer CpG-Stelle aufweist die mit 46391532 von Chromosom 4 des humanen UCSC-hg19-Referenzgenoms aligniert, und
eine genomische Position, die ein C an einer CpG-Stelle aufweist die mit 46391694 von Chromosom 4 des humanen UCSC-hg19-Referenzgenoms aligniert; und
Quantifizieren der Methylierung an der einen oder den mehreren humanen genomischen Positionen in der amplifizierten DNA.

2. Verfahren nach Anspruch 1, wobei die eine oder mehreren humanen genomischen Positionen sieben oder mehr aufweisen von:
eine genomische Position, die ein C an einer CpG-Stelle aufweist die mit 113992930 von Chromosom 2 des humanen UCSC-hg19-Referenzgenoms aligniert,
eine genomische Position, die ein C an einer CpG-Stelle aufweist die mit 113993052 von Chromosom 2 des humanen UCSC-hg19-Referenzgenoms aligniert,
eine genomische Position, die ein C an einer CpG-Stelle aufweist die mit 113993142 von Chromosom 2 des humanen UCSC-hg19-Referenzgenoms aligniert,
eine genomische Position, die ein C an einer CpG-Stelle aufweist die mit 113993304 von Chromosom 2 des humanen UCSC-hg19-Referenzgenoms aligniert,
eine genomische Position, die ein C an einer CpG-Stelle aufweist die mit 113993313 von Chromosom 2 des humanen UCSC-hg19-Referenzgenoms aligniert,
eine genomische Position, die ein C an einer CpG-Stelle aufweist die mit 113994035 von Chromosom 2 des humanen UCSC-hg19-Referenzgenoms aligniert,
eine genomische Position, die ein C an einer CpG-Stelle aufweist die mit 113994578 von Chromosom 2 des humanen UCSC-hg19-Referenzgenoms aligniert,
eine genomische Position, die ein C an einer CpG-Stelle aufweist die mit 113995370 von Chromosom 2 des humanen UCSC-hg19-Referenzgenoms aligniert,
eine genomische Position, die ein C an einer CpG-Stelle aufweist die mit 113995456 von Chromosom 2 des humanen UCSC-hg19-Referenzgenoms aligniert,
eine genomische Position, die ein C an einer CpG-Stelle aufweist die mit 124985406 von Chromosom 9 des humanen UCSC-hg19-Referenzgenoms aligniert,
eine genomische Position, die ein C an einer CpG-Stelle aufweist die mit 124985756 von Chromosom 9 des humanen UCSC-hg19-Referenzgenoms aligniert,
eine genomische Position, die ein C an einer CpG-Stelle aufweist die mit 124987896 von Chromosom 9 des humanen UCSC-hg19-Referenzgenoms aligniert,
eine genomische Position, die ein C an einer CpG-Stelle aufweist die mit 124988720 von Chromosom 9 des humanen UCSC-hg19-Referenzgenoms aligniert,
eine genomische Position, die ein C an einer CpG-Stelle aufweist die mit 124989052 von Chromosom 9 des humanen UCSC-hg19-Referenzgenoms aligniert,
eine genomische Position, die ein C an einer CpG-Stelle aufweist die mit 124989241 von Chromosom 9 des humanen UCSC-hg19-Referenzgenoms aligniert,
eine genomische Position, die ein C an einer CpG-Stelle aufweist die mit 124989294 von Chromosom 9 des humanen UCSC-hg19-Referenzgenoms aligniert,
eine genomische Position, die ein C an einer CpG-Stelle aufweist die mit 124989337 von Chromosom 9 des humanen UCSC-hg19-Referenzgenoms aligniert,
eine genomische Position, die ein C an einer CpG-Stelle aufweist die mit 124989408 von Chromosom 9 des humanen UCSC-hg19-Referenzgenoms aligniert,
eine genomische Position, die ein C an einer CpG-Stelle aufweist die mit 124989550 von Chromosom 9 des humanen UCSC-hg19-Referenzgenoms aligniert,
eine genomische Position, die ein C an einer CpG-Stelle aufweist die mit 124989839 von Chromosom 9 des humanen UCSC-hg19-Referenzgenoms aligniert,
eine genomische Position, die ein C an einer CpG-Stelle aufweist die mit 46391392 von Chromosom 4 des humanen UCSC-hg19-Referenzgenoms aligniert,
eine genomische Position, die ein C an einer CpG-Stelle aufweist die mit 46391436 von Chromosom 4 des humanen UCSC-hg19-Referenzgenoms aligniert,
eine genomische Position, die ein C an einer CpG-Stelle aufweist die mit 46391448 von Chromosom 4 des humanen UCSC-hg19-Referenzgenoms aligniert,
eine genomische Position, die ein C an einer CpG-Stelle aufweist die mit 46391476 von Chromosom 4 des humanen UCSC-hg19-Referenzgenoms aligniert,
eine genomische Position, die ein C an einer CpG-Stelle aufweist die mit 46391524 von Chromosom 4 des humanen UCSC-hg19-Referenzgenoms aligniert,
eine genomische Position, die ein C an einer CpG-Stelle aufweist die mit 46391532 von Chromosom 4 des humanen UCSC-hg19-Referenzgenoms aligniert, und
eine genomische Position, die ein C an einer CpG-Stelle aufweist die mit 46391694 von Chromosom 4 des humanen UCSC-hg19-Referenzgenoms aligniert.

3. Verfahren nach Anspruch 1 oder 2, wobei die eine oder mehreren humanen genomischen Positionen acht oder mehr aufweisen von:
eine genomische Position, die ein C an einer CpG-Stelle aufweist die mit 113992930 von Chromosom 2 des humanen UCSC-hg19-Referenzgenoms aligniert,
eine genomische Position, die ein C an einer CpG-Stelle aufweist die mit 113993052 von Chromosom 2 des humanen UCSC-hg19-Referenzgenoms aligniert,
eine genomische Position, die ein C an einer CpG-Stelle aufweist die mit 113993142 von Chromosom 2 des humanen UCSC-hg19-Referenzgenoms aligniert,
eine genomische Position, die ein C an einer CpG-Stelle aufweist die mit 113993304 von Chromosom 2 des humanen UCSC-hg19-Referenzgenoms aligniert,
eine genomische Position, die ein C an einer CpG-Stelle aufweist die mit 113993313 von Chromosom 2 des humanen UCSC-hg19-Referenzgenoms aligniert,
eine genomische Position, die ein C an einer CpG-Stelle aufweist die mit 113994035 von Chromosom 2 des humanen UCSC-hg19-Referenzgenoms aligniert,
eine genomische Position, die ein C an einer CpG-Stelle aufweist die mit 113994578 von Chromosom 2 des humanen UCSC-hg19-Referenzgenoms aligniert,
eine genomische Position, die ein C an einer CpG-Stelle aufweist die mit 113995370 von Chromosom 2 des humanen UCSC-hg19-Referenzgenoms aligniert, und
eine genomische Position, die ein C an einer CpG-Stelle aufweist die mit 113995456 von Chromosom 2 des humanen UCSC-hg19-Referenzgenoms aligniert.

4. Verfahren nach einem der vorhergehenden Ansprüche, wobei die eine oder mehreren humanen genomischen Positionen jede aufweisen von:
eine genomische Position, die ein C an einer CpG-Stelle aufweist die mit 113992930 von Chromosom 2 des humanen UCSC-hg19-Referenzgenoms aligniert,
eine genomische Position, die ein C an einer CpG-Stelle aufweist die mit 113993052 von Chromosom 2 des humanen UCSC-hg19-Referenzgenoms aligniert,
eine genomische Position, die ein C an einer CpG-Stelle aufweist die mit 113993142 von Chromosom 2 des humanen UCSC-hg19-Referenzgenoms aligniert,
eine genomische Position, die ein C an einer CpG-Stelle aufweist die mit 113993304 von Chromosom 2 des humanen UCSC-hg19-Referenzgenoms aligniert,
eine genomische Position, die ein C an einer CpG-Stelle aufweist die mit 113993313 von Chromosom 2 des humanen UCSC-hg19-Referenzgenoms aligniert,
eine genomische Position, die ein C an einer CpG-Stelle aufweist die mit 113994035 von Chromosom 2 des humanen UCSC-hg19-Referenzgenoms aligniert,
eine genomische Position, die ein C an einer CpG-Stelle aufweist die mit 113994578 von Chromosom 2 des humanen UCSC-hg19-Referenzgenoms aligniert,
eine genomische Position, die ein C an einer CpG-Stelle aufweist die mit 113995370 von Chromosom 2 des humanen UCSC-hg19-Referenzgenoms aligniert, und
eine genomische Position, die ein C an einer CpG-Stelle aufweist die mit 113995456 von Chromosom 2 des humanen UCSC-hg19-Referenzgenoms aligniert.

5. Verfahren nach einem der vorhergehenden Ansprüche, wobei die eine oder mehreren humanen genomischen Positionen jede aufweisen von:
eine genomische Position, die ein C an einer CpG-Stelle aufweist die mit 124985406 von Chromosom 9 des humanen UCSC-hg19-Referenzgenoms aligniert,
eine genomische Position, die ein C an einer CpG-Stelle aufweist die mit 124985756 von Chromosom 9 des humanen UCSC-hg19-Referenzgenoms aligniert,
eine genomische Position, die ein C an einer CpG-Stelle aufweist die mit 124987896 von Chromosom 9 des humanen UCSC-hg19-Referenzgenoms aligniert,
eine genomische Position, die ein C an einer CpG-Stelle aufweist die mit 124988720 von Chromosom 9 des humanen UCSC-hg19-Referenzgenoms aligniert,
eine genomische Position, die ein C an einer CpG-Stelle aufweist die mit 124989052 von Chromosom 9 des humanen UCSC-hg19-Referenzgenoms aligniert,
eine genomische Position, die ein C an einer CpG-Stelle aufweist die mit 124989241 von Chromosom 9 des humanen UCSC-hg19-Referenzgenoms aligniert,
eine genomische Position, die ein C an einer CpG-Stelle aufweist die mit 124989294 von Chromosom 9 des humanen UCSC-hg19-Referenzgenoms aligniert,
eine genomische Position, die ein C an einer CpG-Stelle aufweist die mit 124989337 von Chromosom 9 des humanen UCSC-hg19-Referenzgenoms aligniert,
eine genomische Position, die ein C an einer CpG-Stelle aufweist die mit 124989408 von Chromosom 9 des humanen UCSC-hg19-Referenzgenoms aligniert,
eine genomische Position, die ein C an einer CpG-Stelle aufweist die mit 124989550 von Chromosom 9 des humanen UCSC-hg19-Referenzgenoms aligniert, und
eine genomische Position, die ein C an einer CpG-Stelle aufweist die mit 124989839 von Chromosom 9 des humanen UCSC-hg19-Referenzgenoms aligniert.

6. Verfahren nach einem der vorhergehenden Ansprüche, wobei die eine oder mehreren humanen genomischen Positionen jede aufweisen von:
eine genomische Position, die ein C an einer CpG-Stelle aufweist die mit 46391392 von Chromosom 4 des humanen UCSC-hg19-Referenzgenoms aligniert,
eine genomische Position, die ein C an einer CpG-Stelle aufweist die mit 46391436 von Chromosom 4 des humanen UCSC-hg19-Referenzgenoms aligniert,
eine genomische Position, die ein C an einer CpG-Stelle aufweist die mit 46391448 von Chromosom 4 des humanen UCSC-hg19-Referenzgenoms aligniert,
eine genomische Position, die ein C an einer CpG-Stelle aufweist die mit 46391476 von Chromosom 4 des humanen UCSC-hg19-Referenzgenoms aligniert,
eine genomische Position, die ein C an einer CpG-Stelle aufweist die mit 46391524 von Chromosom 4 des humanen UCSC-hg19-Referenzgenoms aligniert,
eine genomische Position, die ein C an einer CpG-Stelle aufweist die mit 46391532 von Chromosom 4 des humanen UCSC-hg19-Referenzgenoms aligniert, und
eine genomische Position, die ein C an einer CpG-Stelle aufweist die mit 46391694 von Chromosom 4 des humanen UCSC-hg19-Referenzgenoms aligniert.

7. Verfahren nach einem der vorhergehenden Ansprüche, wobei:
die Primer ein oder mehrere Primerpaare aufweisen, wobei jedes Primerpaar ein DNA-Fragment mit einer Länge von 150-350 bp amplifiziert, optional wobei das eine oder die mehreren Primerpaare aus weniger als 10 Primerpaaren bestehen.

8. Verfahren nach einem der vorhergehenden Ansprüche, wobei die Körperflüssigkeit ausgewählt ist aus der Gruppe bestehend aus Speichel, Blut, Cerebrospinalflüssigkeit, Schweiß, Tränen, Samenflüssigkeit und Urin, optional wobei die Körperflüssigkeit Blut oder Speichel ist.

9. Verfahren nach einem der vorhergehenden Ansprüche, wobei das Individuum ein Mensch ist.

10. Verfahren nach einem der vorhergehenden Ansprüche, wobei die Körperflüssigkeitsprobe von einem gesunden Individuum stammt oder die Körperflüssigkeitsprobe von einem Individuum stammt, bei dem zuvor Diabetes, Asthma oder Hypertonie diagnostiziert wurde.

11. Verfahren nach einem der vorhergehenden Ansprüche, wobei die Körperflüssigkeitsprobe von einem Individuum mit einer mittels eines multiplen Schlaflatenztests gemessenen mittleren Schlaflatenz von weniger als 10 Minuten stammt.

12. Verfahren nach einem der vorhergehenden Ansprüche, wobei die Körperflüssigkeitsprobe von einem Individuum mit einem mittels Infrarot-Pupillometrie gemessenen Pupillenunruheindex von mindestens 9,8 stammt.

13. Verfahren nach einem der vorhergehenden Ansprüche, wobei die Körperflüssigkeitsprobe von einem Individuum stammt, das in einem psychomotorischen Vigilanztest mindestens 4,8 Aussetzer aufweist.

## Revendications

1. - Procédé comprenant :
l'isolement de l'ADN cible à partir d'un échantillon de biofluide provenant d'un sujet afin d'obtenir de l'ADN cible isolé ;
le traitement de l'ADN cible isolé avec du bisulfite pour générer de l'ADN modifié par bisulfite ;
l'amplification de l'ADN modifié par bisulfite à l'aide d'amorces spécifiques d'une ou plusieurs positions génomiques humaines afin de générer de l'ADN amplifié, la ou les positions génomiques humaines comprenant au moins six des parmi :
une position génomique ayant un C dans un site CpG s'alignant sur la position 113992930 du chromosome 2 du génome de référence humain UCSC hg19,
une position génomique ayant un C dans un site CpG s'alignant sur la position 113993052 du chromosome 2 du génome de référence humain UCSC hg19,
une position génomique ayant un C dans un site CpG s'alignant sur la position 113993142 du chromosome 2 du génome de référence humain UCSC hg19,
une position génomique ayant un C dans un site CpG s'alignant sur la position 113993304 du chromosome 2 du génome de référence humain UCSC hg19,
une position génomique ayant un C dans un site CpG s'alignant sur la position 113993313 du chromosome 2 du génome de référence humain UCSC hg19,
une position génomique ayant un C dans un site CpG s'alignant sur la position 113994035 du chromosome 2 du génome de référence humain UCSC hg19,
une position génomique ayant un C dans un site CpG s'alignant sur la position 113994578 du chromosome 2 du génome de référence humain UCSC hg19,
une position génomique ayant un C dans un site CpG s'alignant sur la position 113995370 du chromosome 2 du génome de référence humain UCSC hg19,
une position génomique ayant un C dans un site CpG s'alignant sur la position 113995456 du chromosome 2 du génome de référence humain UCSC hg19,
une position génomique ayant un C dans un site CpG s'alignant sur la position 124985406 du chromosome 9 du génome de référence humain UCSC hg19,
une position génomique ayant un C dans un site CpG s'alignant sur la position 124985756 du chromosome 9 du génome de référence humain UCSC hg19,
une position génomique ayant un C dans un site CpG s'alignant sur la position 124987896 du chromosome 9 du génome de référence humain UCSC hg19,
une position génomique ayant un C dans un site CpG s'alignant sur la position 124988720 du chromosome 9 du génome de référence humain UCSC hg19,
une position génomique ayant un C dans un site CpG s'alignant sur la position 124989052 du chromosome 9 du génome de référence humain UCSC hg19,
une position génomique ayant un C dans un site CpG s'alignant sur la position 124989241 du chromosome 9 du génome de référence humain UCSC hg19,
une position génomique ayant un C dans un site CpG s'alignant sur la position 124989294 du chromosome 9 du génome de référence humain UCSC hg19,
une position génomique ayant un C dans un site CpG s'alignant sur la position 124989337 du chromosome 9 du génome de référence humain UCSC hg19,
une position génomique ayant un C dans un site CpG s'alignant sur la position 124989408 du chromosome 9 du génome de référence humain UCSC hg19,
une position génomique ayant un C dans un site CpG s'alignant sur la position 124989550 du chromosome 9 du génome de référence humain UCSC hg19,
une position génomique ayant un C dans un site CpG s'alignant sur la position 124989839 du chromosome 9 du génome de référence humain UCSC hg19,
une position génomique ayant un C dans un site CpG s'alignant sur la position 46391392 du chromosome 4 du génome de référence humain UCSC hg19,
une position génomique ayant un C dans un site CpG s'alignant sur la position 46391436 du chromosome 4 du génome de référence humain UCSC hg19,
une position génomique ayant un C dans un site CpG s'alignant sur la position 46391448 du chromosome 4 du génome de référence humain UCSC hg19,
une position génomique ayant un C dans un site CpG s'alignant sur la position 46391476 du chromosome 4 du génome de référence humain UCSC hg19,
une position génomique ayant un C dans un site CpG s'alignant sur la position 46391524 du chromosome 4 du génome de référence humain UCSC hg19,
une position génomique ayant un C dans un site CpG s'alignant sur la position 46391532 du chromosome 4 du génome de référence humain UCSC hg19, et
une position génomique ayant un C dans un site CpG s'alignant sur la position 46391694 du chromosome 4 du génome de référence humain UCSC hg19 ; et
la quantification de la méthylation à la ou aux positions génomiques humaines dans l'ADN amplifié.

2. - Procédé selon la revendication 1, dans lequel la ou les positions génomiques humaines comprennent au moins sept parmi :
une position génomique ayant un C dans un site CpG s'alignant sur la position 113992930 du chromosome 2 du génome de référence humain UCSC hg19,
une position génomique ayant un C dans un site CpG s'alignant sur la position 113993052 du chromosome 2 du génome de référence humain UCSC hg19,
une position génomique ayant un C dans un site CpG s'alignant sur la position 113993142 du chromosome 2 du génome de référence humain UCSC hg19,
une position génomique ayant un C dans un site CpG s'alignant sur la position 113993304 du chromosome 2 du génome de référence humain UCSC hg19,
une position génomique ayant un C dans un site CpG s'alignant sur la position 113993313 du chromosome 2 du génome de référence humain UCSC hg19,
une position génomique ayant un C dans un site CpG s'alignant sur la position 113994035 du chromosome 2 du génome de référence humain UCSC hg19,
une position génomique ayant un C dans un site CpG s'alignant sur la position 113994578 du chromosome 2 du génome de référence humain UCSC hg19,
une position génomique ayant un C dans un site CpG s'alignant sur la position 113995370 du chromosome 2 du génome de référence humain UCSC hg19,
une position génomique ayant un C dans un site CpG s'alignant sur la position 113995456 du chromosome 2 du génome de référence humain UCSC hg19,
une position génomique ayant un C dans un site CpG s'alignant sur la position 124985406 du chromosome 9 du génome de référence humain UCSC hg19,
une position génomique ayant un C dans un site CpG s'alignant sur la position 124985756 du chromosome 9 du génome de référence humain UCSC hg19,
une position génomique ayant un C dans un site CpG s'alignant sur la position 124987896 du chromosome 9 du génome de référence humain UCSC hg19,
une position génomique ayant un C dans un site CpG s'alignant sur la position 124988720 du chromosome 9 du génome de référence humain UCSC hg19,
une position génomique ayant un C dans un site CpG s'alignant sur la position 124989052 du chromosome 9 du génome de référence humain UCSC hg19,
une position génomique ayant un C dans un site CpG s'alignant sur la position 124989241 du chromosome 9 du génome de référence humain UCSC hg19,
une position génomique ayant un C dans un site CpG s'alignant sur la position 124989294 du chromosome 9 du génome de référence humain UCSC hg19,
une position génomique ayant un C dans un site CpG s'alignant sur la position 124989337 du chromosome 9 du génome de référence humain UCSC hg19,
une position génomique ayant un C dans un site CpG s'alignant sur la position 124989408 du chromosome 9 du génome de référence humain UCSC hg19,
une position génomique ayant un C dans un site CpG s'alignant sur la position 124989550 du chromosome 9 du génome de référence humain UCSC hg19,
une position génomique ayant un C dans un site CpG s'alignant sur la position 124989839 du chromosome 9 du génome humain de référence UCSC hg19,
une position génomique ayant un C dans un site CpG s'alignant sur la position 46391392 du chromosome 4 du génome humain de référence UCSC hg19,
une position génomique ayant un C dans un site CpG s'alignant sur la position 46391436 du chromosome 4 du génome de référence humain UCSC hg19,
une position génomique ayant un C dans un site CpG s'alignant sur la position 46391448 du chromosome 4 du génome de référence humain UCSC hg19,
une position génomique ayant un C dans un site CpG s'alignant sur la position 46391476 du chromosome 4 du génome de référence humain UCSC hg19,
une position génomique ayant un C dans un site CpG s'alignant sur la position 46391524 du chromosome 4 du génome de référence humain UCSC hg19,
une position génomique ayant un C dans un site CpG s'alignant sur la position 46391532 du chromosome 4 du génome de référence humain UCSC hg19, et
une position génomique ayant un C dans un site CpG s'alignant sur la position 46391694 du chromosome 4 du génome de référence humain UCSC hg19.

3. - Procédé selon la revendication 1 ou 2, dans lequel la ou les positions génomiques humaines comprennent au moins huit des éléments suivants :
une position génomique ayant un C dans un site CpG s'alignant sur la position 113992930 du chromosome 2 du génome de référence humain UCSC hg19,
une position génomique ayant un C dans un site CpG s'alignant sur la position 113993052 du chromosome 2 du génome de référence humain UCSC hg19,
une position génomique ayant un C dans un site CpG s'alignant sur la position 113993142 du chromosome 2 du génome de référence humain UCSC hg19,
une position génomique ayant un C dans un site CpG s'alignant sur la position 113993304 du chromosome 2 du génome de référence humain UCSC hg19,
une position génomique ayant un C dans un site CpG s'alignant sur la position 113993313 du chromosome 2 du génome de référence humain UCSC hg19,
une position génomique ayant un C dans un site CpG s'alignant sur la position 113994035 du chromosome 2 du génome de référence humain UCSC hg19,
une position génomique ayant un C dans un site CpG s'alignant sur la position 113994578 du chromosome 2 du génome de référence humain UCSC hg19,
une position génomique ayant un C dans un site CpG s'alignant sur la position 113995370 du chromosome 2 du génome de référence humain UCSC hg19,
une position génomique ayant un C dans un site CpG s'alignant sur la position 113995456 du chromosome 2 du génome de référence humain UCSC hg19.

4. - Procédé selon l'une quelconque des revendications précédentes, dans lequel la ou les positions génomiques humaines comprennent chacune :
une position génomique ayant un C dans un site CpG s'alignant sur la position 113992930 du chromosome 2 du génome de référence humain UCSC hg19,
une position génomique ayant un C dans un site CpG s'alignant sur la position 113993052 du chromosome 2 du génome de référence humain UCSC hg19,
une position génomique ayant un C dans un site CpG aligné sur la position 113993142 du chromosome 2 du génome de référence humain UCSC hg19,
une position génomique ayant un C dans un site CpG s'alignant sur la position 113993304 du chromosome 2 du génome de référence humain UCSC hg19,
une position génomique ayant un C dans un site CpG s'alignant sur la position 113993313 du chromosome 2 du génome de référence humain UCSC hg19,
une position génomique ayant un C dans un site CpG s'alignant sur la position 113994035 du chromosome 2 du génome de référence humain UCSC hg19,
une position génomique ayant un C dans un site CpG s'alignant sur la position 113994578 du chromosome 2 du génome de référence humain UCSC hg19,
une position génomique ayant un C dans un site CpG s'alignant sur la position 113995370 du chromosome 2 du génome de référence humain UCSC hg19,
une position génomique ayant un C dans un site CpG s'alignant sur la position 113995456 du chromosome 2 du génome de référence humain UCSC hg19.

5. - Procédé selon l'une quelconque des revendications précédentes, dans lequel la ou les positions génomiques humaines comprennent chacune :
une position génomique ayant un C dans un site CpG s'alignant sur la position 124985406 du chromosome 9 du génome de référence humain UCSC hg19,
une position génomique ayant un C dans un site CpG s'alignant sur la position 124985756 du chromosome 9 du génome de référence humain UCSC hg19,
une position génomique ayant un C dans un site CpG aligné sur la position 124987896 du chromosome 9 du génome de référence humain UCSC hg19,
une position génomique ayant un C dans un site CpG s'alignant sur la position 124988720 du chromosome 9 du génome de référence humain UCSC hg19,
une position génomique ayant un C dans un site CpG s'alignant sur la position 124989052 du chromosome 9 du génome de référence humain UCSC hg19,
une position génomique ayant un C dans un site CpG s'alignant sur la position 124989241 du chromosome 9 du génome de référence humain UCSC hg19,
une position génomique ayant un C dans un site CpG s'alignant sur la position 124989294 du chromosome 9 du génome de référence humain UCSC hg19,
une position génomique ayant un C dans un site CpG s'alignant sur la position 124989337 du chromosome 9 du génome de référence humain UCSC hg19,
une position génomique ayant un C dans un site CpG s'alignant sur la position 124989408 du chromosome 9 du génome de référence humain UCSC hg19,
une position génomique ayant un C dans un site CpG s'alignant sur la position 124989550 du chromosome 9 du génome de référence humain UCSC hg19,
une position génomique ayant un C dans un site CpG s'alignant sur la position 124989839 du chromosome 9 du génome de référence humain UCSC hg19.

6. - Procédé selon l'une quelconque des revendications précédentes, dans lequel la ou les positions génomiques humaines comprennent chacune :
une position génomique ayant un C dans un site CpG s'alignant sur la position 46391392 du chromosome 4 du génome de référence humain UCSC hg19,
une position génomique ayant un C dans un site CpG s'alignant sur la position 46391436 du chromosome 4 du génome de référence humain UCSC hg19,
une position génomique ayant un C dans un site CpG aligné sur la position 46391448 du chromosome 4 du génome de référence humain UCSC hg19,
une position génomique ayant un C dans un site CpG s'alignant sur la position 46391476 du chromosome 4 du génome de référence humain UCSC hg19,
une position génomique ayant un C dans un site CpG s'alignant sur la position 46391524 du chromosome 4 du génome de référence humain UCSC hg19,
une position génomique ayant un C dans un site CpG s'alignant sur la position 46391532 du chromosome 4 du génome de référence humain UCSC hg19, et
une position génomique ayant un C dans un site CpG aligné sur la position 46391694 du chromosome 4 du génome de référence humain UCSC hg19.

7. - Procédé selon l'une quelconque des revendications précédentes, dans lequel :
les amorces comprennent une ou plusieurs paires d'amorces, chaque paire d'amorces amplifiant un fragment d'ADN d'une longueur de 150 à 350 paires de bases, facultativement, dans lequel la ou les paires d'amorces sont en nombre inférieur à 10.

8. - Procédé selon l'une quelconque des revendications précédentes, dans lequel le fluide biologique est choisi dans le groupe constitué par la salive, le sang, le liquide céphalo-rachidien, la sueur, les larmes, le sperme et l'urine, facultativement dans lequel le fluide biologique est le sang ou la salive.

9. - Procédé selon l'une quelconque des revendications précédentes, dans lequel le sujet est un être humain.

10. - Procédé selon l'une quelconque des revendications précédentes, dans lequel l'échantillon de fluide biologique provient d'un sujet en bonne santé ou l'échantillon de fluide biologique provient d'un sujet pour lequel on a déjà diagnostiqué le diabète, l'asthme ou l'hypertension.

11. - Procédé selon l'une quelconque des revendications précédentes, dans lequel l'échantillon de fluide biologique provient d'un sujet présentant une latence moyenne d'endormissement, telle que mesurée par un test itératif de latence d'endormissement, inférieure à 10 minutes.

12. - Procédé selon l'une quelconque des revendications précédentes, dans lequel l'échantillon de fluide biologique provient d'un sujet présentant un indice d'agitation pupillaire, tel que mesuré par pupillométrie infrarouge, d'au moins 9,8.

13. - Procédé selon l'une quelconque des revendications précédentes, dans lequel l'échantillon de fluide biologique provient d'un sujet présentant des baisses de vigilance d'au moins 4,8 lors d'un test de vigilance psychomotrice.
